# EUROPEAN PATENT APPLICATION

(11) **EP 1 894 925 A1**
(43) Date of publication of application: **05.03.2008**
(21) Application number: 06017677.3
(22) Date of filing: 24.08.2006
(51) Int. Cl.: C07D 401/14, C07D 401/04, A61K 31/435, A61K 31/4164, A61P 29/00

(54) **Imidazole compounds having an antiinflammatory effect**

(71) Applicant: MERCKLE GMBH, 89079 Ulm (DE)
(72) Inventor: The designation of the inventor has not yet been filed
(74) Representative: Reitstötter - Kinzebach

(57) **Abstract**

The invention relates to 2-sulfinyl- or 2-sulfonyl-substituted imidazole derivatives of the formula I 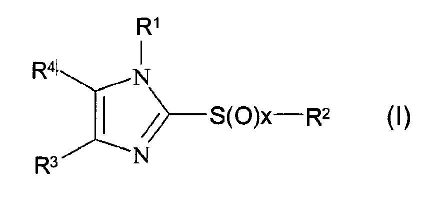 in which the radicals R¹, R², R³ and R⁴ have the meaning indicated in the description. The compounds of the invention have an immunomodulating and/or cytokine release-inhibiting effect and are therefore suitable for the treatment of disorders associated with an impairment of the immune system.

## Description

The present invention relates to imidazole compounds having an immunomodulating and cytokine release-inhibiting effect, to pharmaceutical compositions which comprise the compounds, and to their use in pharmacy.

Pharmacologically active imidazole compounds having antiinflammatory activity are known, see GB 1,155,580; US 4,585,771; EP 236 628 A; EP 372 445 A; US 4,355,039; US 5,364,875; US 4,190,666; WO 02/076 951, WO 9113876; GB 1,564,184; JP 64-40467; WO 88/01167; WO 96/03387; J. Med. Chem. 1995, 38, 1067-1083; Acta Chim. 1969, 61(1), 69-77; and J. Med. Chem. 1999, 2180-2190.

Very diverse pharmaceutical effects have been described for 2-thioimidazole compounds having 4,5-diaryl and 4(5)-(heteroaryl)arylimidazole elements. Similar is also true of compounds related thereto having a substitution on N1 and/or C2 on the imidazole ring.

EP 0 043 788 A (US 4,528,298 and US 4,402,960) describe 4,5-di(hetero)aryl-imidazole derivatives which are substituted at position 2 via a thio or sulfinyl or sulfonyl group by a phenyl, pyridyl, N-oxypyridyl, pyrimidyl, thiazolyl or thienyl radical and have an antiinflammatory and antiallergic activity.

WO 00/17192 (and Angew. Chem. Int. Ed. 2002, 41, 2290-2291) relates to 4-heteroaryl-5-phenylimidazole derivatives which are substituted at position 2 by a phenylalkylthio group. They have no N1 substituent and exist in 2 tautomers. These compounds act as an antiinflammatory agent and inhibitor of cytokine release. In the compounds described in DE 35 04 678 there are sulphur-linked alkanecarboxylic acid residues in position 2 of the 1,4,5-triaryl-substituted imidazole. The 4-heteroaryl-5-phenylimidazoles described in WO 99/03837 have functionalized and nonfunctionalized alkanes, which are also linked via sulfur atoms, at position C2, and carbonyl-linked radicals in position N1.

WO 93/14081 describes 2-substituted imidazoles which inhibit the synthesis of a number of inflammatory cytokines. The compounds described in WO 93/14081 have a phosphorus-containing substituent linked via a sulfur atom, or an aryl or heteroaryl substituent in position 2. US 5,656,644 describes similar compounds. WO 91/10662 describes imidazole derivatives which inhibit acyl-coenzyme A: cholesterol 0-acyltransferase and the binding of thromboxane TxA₂. WO 95/00501 describes imidazole derivatives which can be used as cyclooxygenase inhibitors. The imidazole derivatives described in EP 005 545 A (US 4,440,776 and US 4,269,847) have an antiinflammatory, antiallergic and immunostimulating effect.

J. Med. Chem. 1996, 39, 3927-37 describes compounds having a 5-lipoxygenase-and cyclooxygenase-inhibiting effect, with 2-(4-methylsulfinylphenyl)-4-(4-fluorophenyl)-5-(pyrid-4-yl)imidazole also having a cytokine-inhibiting effect. In addition, EP 004 648 and the corresponding US 4,461,770, US 4,584,310 and US 4,608,382 disclose 2-alkylthio-, 2-alkylsulfinyl and 2-alkylsulfonyl- and N1-alkylsubstituted imidazole derivatives which have in position 4 and 5 in each case a heteroaryl radical (preferably 3-pyridyl and 2-thienyl) combined with an aryl radical which is then located in the respective other ring position (preferably phenyl and 4-fluorophenyl). These compounds have an antiinflammatory effect and antinociceptive activity (rat paw edema and mouse phenylquinone writhing test) in the dose range 50-200 mg/kg orally and 100 mg/kg orally, respectively. The compounds inhibit prostaglandin synthesis from arachidonic acid (cyclooxygenase/5-lipoxygenase inhibition according to Prostaglandins 7, 123 (1974)) in the range 10 - 30 mg/L (10⁻⁴ to 10⁻⁵ M).

WO 04/018458 A1 describes 2-thio-, 2-sulfinyl- and 2-sulfonyl-substituted imidazole compounds having a cytokine-inhibiting effect which are unsubstituted on N1. The compounds substituted on N1 which are disclosed in WO 02/066458 A2 show an in vitro activity, which is improved compared with the prior art, on the main pharmacological target, the p38 MAP kinase alpha. Besides the P38 Map kinase alpha, prior art compounds influence further kinases of the cellular signal transduction cascade, e.g. the isoenzymes of p38 MAP kinase, extracellular receptor kinases, apoptotic kinases and cell cycle-regulating kinases. WO 02/066458 A2, WO 03/097633 and PCT/EP 2006/001801 describe 2-thio-substituted, N1-substituted imidazole compounds having a cytokine-inhibiting effect which inhibit P38 MAP kinase alpha with high selectivity and moreover exert a smaller influence on cytochrome P450 enzyme systems. In cell assays (isolated PMNLs), the compounds show an activity which is improved by comparison with the prior art in relation to the suppression of release of the proinflammatory cytokines TNFα and IL1β after stimulation with lipopolysaccharides. However, these compounds have proved to be relatively toxic.

Despite the numerous known compounds, therefore, there continues to be a need for compounds having an antiinflammatory effect which inhibit cytokine release and show low toxicity.

The object of the invention is to provide such compounds.

It has now surprisingly been found that certain thio-imidazole compoundss which have a sulfanyl, sulfinyl or sulfonyl substituent in position 2 show antiinflammatory properties.

The present invention therefore relates to the imidazole compounds of the formula I 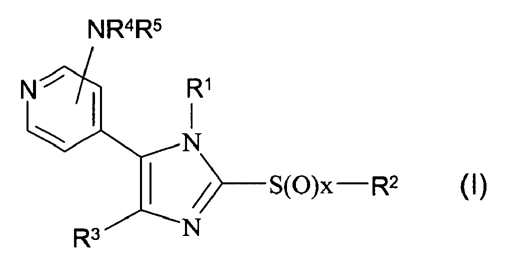 in which
R¹ is selected from:
a) C₁-C₆-alkyl which is optionally substituted by one or two groups independently of one another selected from
   hydroxy;
   C₁-C₄-alkoxy;
   C₂-C₆-alkenyloxy;
   C₂-C₆-alkynyloxy;
   CO₂H;
   CO₂-C₁-C₆-alkyl;
   CN;
   halogen;
   C₁-C₆-alkyl- SO₃;
   C₁-C₆-alkylthio;
   NR⁷R⁸, wherein R⁷ and R⁸ are independently of one another H, C₁-C₆-alkyl or hydroxy-C₁-C₆-alkyl;
   R⁹CONR¹⁰, R⁹ and R¹⁰ are independently of one another H or C₁-C₆-alkyl;
   a nonaromatic heterocyclic radical having 5 or 6 ring atoms and 1 or 2 heteroatoms, selected independently of one another from N, O and S, which heterocyclic radical may be substituted by 1, 2, 3 or 4 C₁-C₆-alkyl groups;
b) -A(̵OA)̵ₙ OB,
   in which
   A is -CH₂CH₂-, -CH₂CH₂CH₂-, 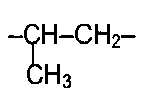 or 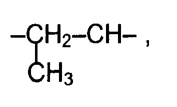 n is 1, 2, 3, 4 or 5, and B is H or C₁-C₄-alkyl;
c) C₁-C₆-oxoalkyl;
d) C₂-C₆-alkenyl;
e) C₃-C₇cycloalkyl;
f) (C₃-C₇cycloalkyl)-C₁-C₆-alkyl;
g) aryl which is optionally substituted by one or more halogen atoms or a C₁-C₄-alkylsulfanyl group;
h) aminoaryl, where the amino group is optionally substituted by one or two C₁-C₄-alkyl groups;
i) aryl-C₁-C₆-alkyl; and
j) an aromatic or nonaromatic heterocyclic radical having 5 or 6 ring atoms and 1 or 2 heteroatoms selected independently of one another from N, O and S, which heterocyclic radical is optionally substituted by 1, 2, 3 or 4 C₁-C₄-alkyl groups, an aryl or aryl-C₁-C₄-alkyl group; and
k) H;
R² is selected from:
a) C₁-C₆-alkyl,
b) phenyl-C₁-C₄-alkyl, where the phenyl group may have one or two substituents which are selected independently of one another from C₁-C₄-alkyl, halogen, C₁-C₄-alkylsulfanyl, C₁-C₄-alkylsulfinyl and C₁-C₄-alkylsulfonyl,
c) C₂-C₆-alkenyl,
d) C₂-C₆-alkenyl which is substituted by one or two halogen atoms and/or phenyl groups, where the phenyl group may be substituted independently by one or two C₁-C₄-alkyl or halogen atoms,
e) C₂-C₆-alkynyl,
f) C₂-C₆-alkynyl which is substituted by a phenyl group which may be optionally substituted by one or two C₁-C₄-alkyl or halogen atoms,
g) C₁-C₆-alkyl which is substituted by C₁-C₄-alkylsulfanyl, C₁-C₄-alkylsulfinyl or C₁-C₄-alkylsulfonyl;
h) C₁-C₆-alkyl which is substituted by -CO-Het wherein Het is a nonaromatic heterocyclic radical having 5 or 6 ring atoms and 1 or 2 heteroatoms which are selected independently of one another from N, O, and S, or C₁-C₄-alkylsulfanyl, C₁-C₄-alkylsulfinyl or C₁-C₄-alkylsulfonyl;
i) phenyl; and
j) phenyl which has one or two substituents which are selected independently of one another from C₁-C₄-alkyl, halogen, C₁-C₄-alkylsulfanyl, C₁-C₄-alkylsulfinyl or C₁-C₄-alkylsulfonyl; or
R¹ and R² together are -CH₂CH₂- or -CH₂CH₂CH₂-;
x is 0, 1 or 2,
R³ is phenyl which is unsubstituted or substituted by 1 or 2 halogen atoms, C₁-C₄-alkyl groups or trifluoromethyl groups;
R⁴ is selected from
a) (C₁-C₆-alkoxy)-C₁-C₆-alkyl;
b) (C₁-C₆-alkoxy)-C₃-C₇cycloalkyl;
c) hydroxy-C₁-C₆-alkyl;
d) hydroxy-C₃-C₇-cycloalkyl;
e) C₃-C₇-oxocycloalkyl;
f) a nonaromatic heterocyclic radical having 5 or 6 ring atoms and 1 or 2 heteroatoms selected independently of one another from N, O and S, which heterocyclic radical is attached via a carbon atom to the amino group and is optionally substituted by 1, 2, 3 or 4 C₁-C₄-alkyl groups, an aryl or aryl-C₁-C₄-alkyl group;
g) C₁-C₆ -alkyl which is substituted by a nonaromatic heterocyclic radical having 5 or 6 ring atoms and 1 or 2 heteroatoms selected independently of one another from N, O and S, which heterocyclic radical is optionally substituted by 1, 2, 3 or 4 C₁-C₄-alkyl groups, an aryl or aryl-C₁-C₄-alkyl group; and
h) C₁-C₆-oxoalkyl;
i) phenyl-C₁-C₄-alkyl, wherein the alkyl group is substituted by 1 or 2 substituents independently selected from OH or C₁-C₄-alkoxy;
j) phenyl-C₁-C₄-alkyl, wherein the phenyl group is substituted by 1 or 2 substituents independently selected from OH or C₁-C₄-alkoxy;
k) C₃-C₇cycloalkyl-C₁-C₄-alkyl, wherein the cycloalkyl group is substituted by 1 or 2 substituents independently selected from OH or C₁-C₄-alkoxy; and
l) phenyl, which is substituted by 1 or 2 substituents independently selected from Hal, OH, C₁-C₄-alkoxy, CF₃, amino, mono-C₁-C₄-alkylamino, di-C₁-C₄-alkylamino, C₁-C₄-alkyl, C₁-C₄-alkylcarbonyl and C₁-C₄-alkyl which is substituted by 1 or 2 substituents independently selected from OH or C₁-C₄-alkoxy;
R⁵ is H, C₁-C₄-alkyl, phenyl or benzyl, and
the optical isomers and physiologically tolerated salts thereof.

If the compounds of the invention have centres of asymmetry, then racemates and optical isomers (enantiomers, diastereomers and enriched forms thereof) are included. In particular, compounds in which R¹ or R² is 1-phenylethyl may exist as racemate (R,S) or enantiomers [(R) or (S)].

The compounds of the invention have an asymmetric center at the sulfur atom, if x is 1. Such compounds are in general obtained in the form of mixtures of the optical antipodes (racemates) which can be separated into the enantiomers by conventional methods. If further centers of asymmetry are present in the molecule, mixtures of diastereomers are formed in the oxidation and can be separated by conventional methods into the individual compounds. Conventional methods for separating said racemates and diastereomers are, e.g., fractional crystallization or chromatographic methods,. The enantiomers are separated preferably by methods of adsorption chromatography on chiral supports, e.g. on modified methyl starches or methylcelluloses (Chiralcel).

The invention includes the racemates, diastereomers and the specific enantiomers and any enriched forms thereof.

The term "alkyl" (also in other groups such as alkoxy, phenylalkyl, alkylsulfonyl etc.) includes straight-chain and branched alkyl groups having preferably 1 to 6 or 1 to 4 C atoms, such as methyl, ethyl, n- and i-propyl, n-, i- and t-butyl, sec-butyl, n-pentyl and n-hexyl.

The term "C₁-C₆-oxoalkyl" means an alkyl group which includes a carbonyl group either in the carbon chain (ketone) or at the end thereof (aldehyde). The carbonyl group is separated from the amino group by at least one carbon atom.

The term "aryl" includes aromatic ring systems such as phenyl or naphthyl.

The term "halogen" or "hal" stands for a fluorine, chlorine, bromine or iodine atom, in particular a fluorine or chlorine atom.

C₃-C₇-Cycloalkyl groups are cyclopropyl, cyclobutyl, cycloheptyl and in particular cyclopentyl and cyclohexyl.

The term "C₃-C₇-oxocycloalkyl" means a C₃-C₇-cycloalkyl group in which one of the carbon atoms is an oxo group (-co-).

The term "hydroxy-C₃-C₇-cycloalkyl" means a C₃-C₇-cycloalkyl group which is substituted with a hydroxy group, such as 2-hydroxy-cyclopentyl, 3-hydroxy-cyclopentyl, 2-hydroxy-cyclohexyl, 3-hydroxycyclohexyl or 4-hydroxycyclohexyl.

The term "alkenyl" (also in other groups such as "alkenyloxy" means a straight-chain or branched alkenyl group having 2 to 6 carbon atoms and a carbon-carbon double bond such as vinyl or allyl.

"Phenylalkenyl" is in particular styryl.

The term "alkynyl" (also in other groups such as "alkynyloxy" means a straight-chain or branched alkynyl group having 2 to 6 carbon atoms and a carbon-carbon triple bond such as acetylenyl or propargyl.

The aromatic or nonaromatic heterocyclic radicals in the compounds of the present invention have 5 or 6 ring atoms. 1 or 2 of said ring atoms are heteroatoms independently of one another selected from O, N and S.

Nonaromatic heterocyclic radicals may be saturated or unsaturated. Pyrrolidinyl, piperidinyl, piperazinyl, pyranyl, tetrahydrofuranyl or morpholinyl are preferred. The piperidinyl radical may be substituted by 1, 2, 3 or 4 C₁-C₄-alkyl groups, in particular methyl groups. A preferred piperidinyl radical is 2,2,6,6-tetramethylpiperidinyl.

Preferred aromatic heterocyclic radicals are pyridyl, especially 3- or 4-pyridyl, pyrimidinyl, pyrrolyl, imidazolyl, pyrazolyl, oxazolyl, isoxazolyl, furyl, thienyl or thiazolyl. The heterocyclic radical may be substituted as indicated above.

Phenyl-C₁-C₄-alkyl means in particular benzyl, 1-phenylethyl or 2-phenylethyl.

If R¹ is C₁-C₆-alkyl which is substituted by a nonaromatic heterocyclic radical, the latter preferably comprises at least one nitrogen atom, and the linkage to the alkyl group preferably takes place via a carbon atom. Preferred heterocyclic radicals are piperidinyl, 1,1,6,6-tetramethyl piperidinyl or morpholinyl.

If R¹ is an aromatic or nonaromatic heterocyclic radical, it is preferably linked via a carbon atom to the imidazole nitrogen. Preferred nonaromatic heterocyclic radicals are piperidinyl or piperidinyl which is substituted at the N-atom with C₁-C₄-alkyl or OCO-C₁-C₄-alkyl.

R¹ is preferably:

C₁-C₆-alkyl which is optionally substituted by one or two hydroxy or C₁-C₄-alkoxy groups or by a nonaromatic heterocyclic radical having 5 or 6 ring atoms and 1 or 2 heteroatoms which are selected independently of one another from N, O and S,

-A (̵ OA )̵ₙ OB,

in which
A is -CH₂CH₂-, -CH₂CH₂CH₂-, 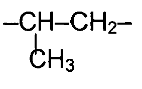 or 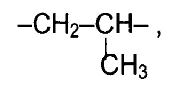 n is 1, 2, 3, 4 or 5, and B is H or C₁-C₄-alkyl,
C₂-C₆-alkenyl,
C₃-C₆-cycloalkyl,
amino-C₁-C₄-alkyl, where the amino group is optionally substituted by one or two C₁-C₄-alkyl groups,
an aromatic or nonaromatic heterocyclic radical having 5 or 6 ring atoms and 1 or 2 heteroatoms which are selected independently of one another from N, O and S, which is optionally substituted by 1, 2, 3 or 4 C₁-C₄-alkyl groups,

In particular R¹ is
C₁-C₆-alkyl which is optionally substituted by one or two hydroxy or C₁-C₄-alkoxy groups or a nonaromatic heterocyclic radical having 5 or 6 ring atoms and 1 or 2 heteroatoms which are selected independently of one another from N, O and S, or

-A (̵OA)̵ₙOB,

in which
A is -CH₂CH₂-, -CH₂CH₂CH₂-, 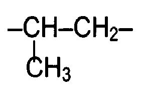 or 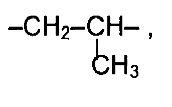 n is 1, 2, 3, 4 or 5 and B is H or C₁-C₄-alkyl.

R¹ is particularly preferably C₁-C₄-alkyl, especially methyl and ethyl, which is substituted by one C₁-C₄-alkoxy group, such as methoxypropyl, methoxyethyl or 2,3-dimethoxypropyl.

R² is preferably H, C₁-C₆-alkyl (especially methyl, ethyl, n-propyl or i-propyl), phenyl-C₁-C₄-alkyl, especially benzyl or phenylethyl (the phenyl group in benzyl or phenylethyl is optionally substituted as indicated above), phenyl or phenyl which has one or two substituents which are selected independently of one another from C₁-C₄-alkyl and halogen. R² is particularly preferably H or C₁-C₆-alkyl.

R³ is preferably phenyl which is substituted by 1 or 2 halogen atoms (in particular F or Cl) or trifluoromethyl groups, by a halogen atom (in particular F or Cl) and a C₁-C₄-alkyl group or by a trifluoromethyl group and a C₁-C₄-alkyl group. The substituents are preferably in 2 and/or 4-position.

R³ is most preferably 4-fluorophenyl, 2,4-difluorophenyl or 3-trifluoromethylphenyl.

R⁴ is preferably (C₁-C₆-alkoxy)-C₁-C₆-alkyl, hydroxy-C₁-C₆-alkyl, hydroxy-C₃-C₇-cycloalkyl, C₃-C₇-oxocycloalkyl, a nonaromatic heterocyclic radical having 5 or 6 ring atoms and 1 or 2 heteroatoms selected independently of one another from N, O and S, which heterocyclic radical is attached via a carbon atom to the amino group and is optionally substituted by 1, 2, 3 or 4 C₁-C₄-alkyl groups, or an aryl or aryl-C₁-C₄-alkyl group; C₁-C₆-alkyl which is substituted by a nonaromatic heterocyclic radical having 5 or 6 ring atoms and 1 or 2 heteroatoms selected independently of one another from N, O and S, which heterocyclic radical is optionally substituted by 1, 2, 3 or 4 C₁-C₄-alkyl groups, an aryl or aryl-C₁-C₄-alkyl group.

If R⁴ is hydroxy-C₃-C₇-cycloalkyl, the hydroxy group may be in cis-position and preferably trans-position to the amino group. A preferred group is hydroxycyclohexyl.

If R⁴ is a non-aromatic heterocyclic radical, it is preferably pyrrolidinyl, N-C₁-C₄-alkylpyrrolidinyl, piperidinyl, which may be substituted by 1, 2, 3 or 4 C₁-C₆-alkyl groups such as 1,1,6,6-piperidinyl, N-benzyl- or N-C₁-C₄-alkylpiperidinyl or tetrahydropyranyl.

If R⁴ is C₁-C₆-alkyl substituted by a non-aromatic heterocyclic radical, said radical is preferably pyrrolidinyl, N-C₁-C₄-alkylpyrrolidinyl, piperidinyl, which may be substituted by 1, 2, 3 or 4 C₁-C₆-alkylgroups such as 1,1,6,6-piperidinyl, N-benzyl- or N-C₁-C₄-alkylpiperidinyl or tetrahydropyranyl.

If R⁴ is defined as under i) above, hydroxybenzyl or 1-phenyl-2-hydroxyethyl are preferred.

If R⁴ is defined as under j) above, 2- and in paricular 4-substituted phenyl is preferred.

If R⁴ is defined as under k) above, 1-C₃-C₇-cycloalkyl (in particular cyclohexyl)-2-hydroxyethyl is preferred.

If R⁴ is defined as under I) above, 2- and in particular 4-substituted phenyl is preferred, suc has 4-hydroxyphenyl, 4-aminophenyl, 4-acetylphenyl and 4-(2-hydroxy-2-propyl)-phenyl.

R⁴ is in particular selected from 2-methoxyethyl, 2-hydroxyethyl, hydroxypropyl such as 2-hydroxy-1-propylyl or 1-hydroxy-2-propyl, hydroxycyclopentyl such as 2- or 3-hydroxycyclopentyl (cis or trans), hydroxycyclohexyl such as 2-, 3- or 4-hydroxy-cyclohexyl (cis or trans), oxocyclopentyl such as 2- or 3-oxocyclopentyl, oxocyclohexyl, such as 2-, 3- or 4-oxocyclohexyl, 2-oxo-1-propyl, tetrahydropyran-4-yl, 1,1,6,6-tetramethylpiperidin-4-yl, piperidin-4-yl, N-benzylpiperidin-4-yl, 2-pyrrolidinylmethyl and N-ethyl-2-pyrrolidinylmethyl.

R⁵ is preferably H.

The substituent NR⁴R⁵ is particularly preferably in position 2 of the 4-pyridyl group.

A particularly preferred embodiment are the compounds of the formula I in which R¹ is C₁-C₄-alkyl, C₁-C₄-alkoxy-C₁-C₄-alkyl or hydroxy-C₂-C₄-alkyl;
R² is H or C₁-C₆-alkyl;
R³ is 4-fluorophenyl;
R⁴ is 4-pyridyl which is substituted by (C₁-C₆-alkoxy)-C₁-C₆-alkyl, hydroxy-C₁-C₆-alkyl, hydroxy-C₃-C₇-cydoalkyl, C₃-C₇-oxocycloalkyl, a heterocyclic radical having 5 or 6 ring atoms and 1 or 2 heteroatoms selected independently of one another from N or S, which heterocyclic radical is attached via a carbon atom to the nitrogen atom and is optionally substituted by 1, 2, 3 or 4 C₁-C₄-alkyl groups or an aryl-C₁-C₄-alkyl group or C₁-C₆-alkyl which is substituted by a nonaromatic heterocyclic radical having 5 or 6 ring atoms and 1 or 2 heteroatoms selected independently of one another from N, O and S, which heterocyclic radical is optionally substituted by C₁-C₄-alkyl or aryl-C₁-C₄-alkyl; and
R⁵ is H.

The invention also relates to compounds of the formula 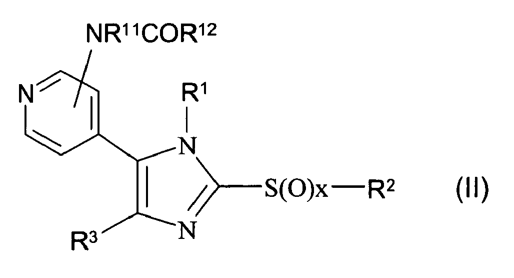 wherein R¹, R², R³ and x are as defined above, R¹¹ is H or C₁-C₄-alkyl and R¹² is C₁-C₄-alkyl which is substituted by 1 or 2 substituents independently selected from halogen, OH, C₁-C₄-alkoxy, C1-C₄-alkylcarbonyloxy, phenyl and substituted phenyl which carries 1 or 2 substituents independently selected from halogen, OH, C₁-C₄-alkoxy and C₁-C₄-alkyl, an aromatic or nonaromatic heterocyclic radical having 5 or 6 ring atoms and 1 or 2 heteroatoms selected independently of one another from N, O and S, which heterocyclic radical is optionally substituted by 1, 2, 3 or 4 C₁-C₄-alkyl groups, an aryl or aryl-C₁-C₄-alkyl group or C₁-C₆ -alkyl which is substituted by a aromatic heterocyclic radical having 5 or 6 ring atoms and 1 or 2 heteroatoms selected independently of one another from N, O and S, which heterocyclic radical is optionally substituted by 1 or 2 C₁-C₄-alkyl groups. R¹¹ is preferably H and R¹² is preferably furanyl, thienyl, tetrahydropyranyl, tetrahydrofuranyl, thiophen-C₁-C₄-alkyl or furanyl- C₁-C₄-alkyl. In a furher preferred embodiment R¹² is chloromethyl, hydroxymethyl, 1-hydroxyethyl, 2-hydroxyethyl, methoxymethyl, 1-methoxyethyl, 2-methoxyethyl, acetyloxymethyl, 1-acethyloxyethyl or 2-acethyloxyethyl. The substituent of the 4-pyridyl group is preferably in 2-position.

The physiologically tolerated salts may in the present case be acid addition salts or base addition salts. Employed for acid addition salts are inorganic acids such as hydrochloric acid, sulphuric acid or phosphoric acid, or organic acids such as tartaric acid, citric acid, maleic acid, fumaric acid, malic acid, mandelic acid, ascorbic acid, gluconic acid and the like.

The compounds of the invention can be prepared by the processes described in WO 02/066458 A2, which is incorporated in its entirety herein by reference. The reaction sequence is shown in scheme 1 to 5 and described in detail in WO 02/066458 and in the general preparation methods below.

The 2-thio compounds in which the pyridyl group is amino- or amido-substituted pyridyl are prepared as shown in scheme 1.

The amino group of the starting compound 2-amino-γ-picoline (1) is protected, e.g. by introducing an acetyl group with acetic anhydride. The methyl group of compound (2) is then oxidized to the carboxyl group, e.g. with potassium permanganate in aqueous medium at 20 to 90°C.

Reaction of the resulting pyridinecarboxylic acid (3) with 4-fluorophenylacetonitrile to give compound (4) and the subsequent elimination of the nitrile group are carried out by variant 1 described in WO 02/066458. In this case, the acetyl group on the amino group of the pyridine compound is also eliminated to form compound (5).

In the next step, the amino group is protected anew, e.g. by introducing an acetyl group with acetic anhydride. The resulting compound (6) is converted into thiono compound (9) as described in WO 02/066458, variant 1 or 2 (shown for variant 1 in scheme 1). The desired radical R² is introduced into (9) as described in WO 02/066458.

In order to introduce the desired substituent into the pyridyl group, firstly the acetyl group is eliminated by hydrolysis, e.g. with aqueous acid, resulting in the amino compound (12). An acyl radical is introduced by acylation, in particular with the appropriate acid chloride, in an inert solvent such as an ether, e.g. tetrahydrofuran, dioxane, or a chlorinated hydrocarbon, e.g. methylene chloride or 1,2-dichloroethane etc. The acylation generally takes place in the presence of a base, e.g. triethylamine, in at least equivalent amount.

The substituted amine compounds are prepared by reacting compound (12) with one or two mole equivalents of an appropriate alkyl bromide, cycloalkyl bromide, phenylalkyl bromide or of an optionally substituted iodobenzene in an inert solvent such as dimethylformamide in the presence of a base such as sodium hydride to give the compounds (14) or (15). Alternatively, the amide compound (13) can be reduced with lithium aluminum hydride in, for example, tetrahydrofuran to compound (16).

An alternative synthesis for compounds of the formula II in which the 4-pyridyl which is substituted by R¹¹CONR¹²- in position 2 is illustrated in scheme 6.

The acetamido compound (17) is converted by hydrolysis with aqueous acids, e.g. dilute HCl, into the compound (18). (18) is treated with tetrafluoroboric acid in the presence of sodium nitrite, resulting in compound (19). This is subjected to a nucleophilic aromatic substitution with the appropriate amine to give compound (20) which is then reacted with an acylating agent such as a carboxylic anhydride or carbonyl chloride, to give compound (21). Alternatively, compound (18) is reacted with a haloacyl chloride such as chloroacetyl chloride, to obtain a compound of formula II wherein R¹² is halogen substituted C₁-C₄-alkyl. The obtained compound can then be further converted by nucleophilic substitutions to obtain other compounds of formula II wherein R¹² is substituted alkyl.

In an alternative synthesis for the amine compounds of formula I R⁴ and R⁵ are introduced into the 2-F-4-pyridyl compound (19)(scheme 4) by reacting the fluorosubstituted 4-pyridyl compound with the desired amine HNR⁴R⁵, which is used in general in a 2- to 10-fold molar excess. The reaction is preferably carried out without solvent. The reaction temperature is in general in the range from 120 to 160°C, the reaction time in the range from 1 h to 72 h.

The compounds of formula I and II wherein R¹ and R² together are ethylene or propylene can be obtained from the thio compounds 22 (which can be prepared according to the methods disclosed in WO 02/066458) shown in scheme 3. Cyclisation occurs by activating the hydroxyl group, for example by converting it to the corresponding methane sulfonate by reaction with methane sulfonic acid chloride in the presence of a base such as pyridine, at a temperature from 50 to 90 °C. Under these reaction conditions the methane sulfonate which is formed as an intermediate cylices to the sulfanyl compound (23) which can be oxidized to the sulfinyl and sulfonyl compound as indicated below. The pyridyl substituent can be modified by subjecting compound (23), (24) or (25) to hydrolysis in aqueous acid to the amino pyridyl compound (26) or (30). The amino group is then substituted by a fluorine atom using Olah's reagent (HF 70% in pyridine) in the presence of sodium nitrite at-10 to -30 °C (Fukuhara et al.; Journal of Fluorine Chemistry, 38 (1988) 435-438, Reagent: 70% (HF)ₓ in pyridine). The obtained sulfanyl compound (27) can then be treated with amine reagents to introduce the desired substituent into the pyridine ring by nucleophilic substitution. The obtained amino substituted sulfanyl compounds can finally be converted to the sulfinyl and the sulfonyl compounds as described below.

The compounds having 2,3-dihydro-imidazo[2,1-b]thiazole and 6,7-dihydro-5H-imidazo[2,1-b] [1,3]thiazine structure can be prepared from N-{4-[5-(4-fluorophenyl)-3-(3-hydroxy-propyl)-2-thioxo-2,3-dihydro-1H-imidazol-4-yl]-pyridin-2-yl}-acetamide and N-{4-[5-(4-fluorophenyl)- 3-(2-hydroxyethyl)-2-thioxo-2,3-dihydro-1H-imidazol-4-yl]-pyridin-2-yl}-acetamide by activation of the hydroxyl group with methane sulfonic acid chloride in pyridine and intramolecular cyclisation. The obtained sulfanyl compounds can then be oxidized to the sulfinyl and sulfonyl compounds as described below. In order to obtain other acyl or alkyl substituted 4-[6-(4-fluorophenyl)- 2,3-dihydro-imidazo[2,1-b]thiazol-5-yl]-pyridin-2-yl-amine and 4-[2,-(4-Fluoro-phenyl)-6,7-dihydro-5H-imidazo[2,1-b][1,3]thiazin-3-yl]-pyridin-2-ylamine said acetamides are cleaved hydrolytically to obtain the free amines which are then alkylated or acylated with the corresponding alkylating or acylating agent.

The sulfanyl compounds of formula I (x = 0) can be converted into the corresponding sulfinyl derivatives and sulfonyl derivatives by oxidation reactions known in the art (scheme 2). The sulfinyl compounds can be obtained with a mild and selective oxidizing agent, in particular peroxocarboxylic acids such as m-chloroperbenzoic acid (mCPBA), hydrogen peroxide in solution in a carboxylic acid, such as acetic acid, or a hydro- peroxide, such as t-butyl hydroperoxide. The oxidizing agent is used in stoichiometric amounts in the cold, particularly at -20°C to room temperature (RT). In order to increase the selectivity of the oxidizing agent catalysts may be employed so that further oxidation to sulfonyl compounds and to imidazole N-oxides or pyridine N-oxides is suppressed. The catalysts can be employed together with oxidizing agents such as sodium metaperiodate, hydrogen peroxide, atmospheric oxygen and peroxy acids. One example of such a catalyst is methylrhenium trioxide which is preferably used together with H₂O₂. The oxidations can also be achieved with sodium hypochlorite in alcoholic solution or with sodium metaperiodate in a 2-phase system.

The sulfonyl compounds are obtained under more energetic conditions through use of excess oxidizing agent or through use of stronger oxidizing agents, such as potassium permanganate, or by applying elevated temperatures.

The compounds of the invention show *in vitro* and *in vivo* an immunomodulating and cytokine release-inhibiting effect. Cytokines are proteins such as TNF-α and IL-1β which play an important part in numerous inflammatory disorders. The compounds of the invention are suitable, owing to their cytokine release-inhibiting effect, for the treatment of disorders associated with an impairment of the immune system. They are suitable for example for the treatment of autoimmune diseases, cancer, rheumatoid arthritis, gout, septic shock, osteoporosis, neuropathic pain, HIV dissemination, HIV dementia, viral myocarditis, insulin-dependent diabetes, periodontal disorders, restenosis, alopecia, T-cell depletion in HIV infections or AIDS, psoriasis, acute pancreatitis, rejection reactions with allogenaic transplants, allergy-related inflammation of the lungs, arterosclerosis, multiple sclerosis, cachexia, Alzheimer's disease, stroke, jaundice, inflammatory bowel diseases such as ulcerative colitis and Crohn's disease, reperfusion damage, ischemia, congestive heart failure, pulmonary fibrosis, hepatitis, glioblastoma, Guillain-Barré syndrome, systemic lupus erythematosus, adult respiratory distress syndrome (ARDS) and respiratory distress syndrome.

The compounds of the invention can be administered either as single therapeutic active ingredients or as mixtures with other therapeutic active ingredients. The compounds can be administered alone, but they are generally dosed and administered in the form of pharmaceutical compositions, i.e. as mixtures of the active ingredients with suitable pharmaceutical carriers or diluents. The compounds or compositions can be administered orally or parenterally, and they are preferably given in oral dosage forms.

The nature of the pharmaceutical composition or carrier or of the diluent depends on the desired administration form. Oral compositions may be for example in the form of tablets or capsules and comprise conventional excipients such as binders (e.g. syrup, acacia, gelatin, sorbitol, tragacanth or polyvinylpyrrolidone), fillers (e.g. lactose, sugars, corn starch, calcium phosphate, sorbitol or glycine), lubricants (e.g. magnesium stearate, talc, polyethylene glycol or silicon dioxide), disintegrants (e.g. starch) or wetting agents (e.g. sodium lauryl sulfate). Liquid oral products may be in the form of aqueous or oily suspensions, solutions, emulsions, syrups, elixirs or sprays and the like. They may also be in the form of a dry powder which is prepared for reconstitution with water or another suitable carrier. Liquid products of this type may comprise conventional additives, for example suspending agents, flavorings, diluents or emulsifiers. Solutions or suspensions with conventional pharmaceutical carriers can be employed for parenteral administration.

The compounds or compositions of the invention can be administered to a mammal (human or animal) in a dose of about 0.5 mg to 100 mg per kg of body weight per day. They can be given in a single dose or in a plurality of doses. The range of effects of the compounds as inhibitors of cytokine release was investigated by means of the test systems as described by Donat C. and Laufer S. in Arch. Pharm. Pharm. Med. Chem. 333, Suppl. 1, 1-40, 2000.

### EXAMPLES

### General conditions and analyses:

| Melting points: | Mettler FP 5 |
|---|---|
| IR spectroscopy: | Thermo Nicolet Avatar 330 FT-IR, with Smart Endurance |
| NMR spectroscopy: | Varian Mercuryplus 400, 5 mm PFG IDP (1H: 400 MHz, 13C:100 MHz) |
| GC-MS: | Agilent GC 6890plus + MSD 5973 + ALS 7683 He-(5% phenyl methyl silicone, 15 m 250µm, 0.25 µm |
| LC-(MS): | HP1090 DAD: Thermo Hypersil Keystone, 150 mm x 4.6, Betasil C8, 5µm and HP1100 Phenomenex, Synergi 250 mm x 2.00 mm Polar-RP 80A, 4µ, DAD+MS (Bruker Esquire HCT-IonTrap) |
| Prep HPLC: | Varian PrepStar 2 SD1: 250 ml/min. column head Column C18, 21.4 mm x 250 mm, 8 µm CSP: Chiralpac AD |

TLC adsorbents and plates:
Polygram SIL G/UV, Macherey-Nagel, Düren.
Polygram ALOX N/UV, Macherey-Nagel, Düren.
Adsorbents for column chromatography:
Aluminium oxide ICN-Alumina TSC, No. 04511, ICN Biomedicals.
Silica gel SiO2 60 (0.063 mm), No. 7734, Merck, Darmstadt.
Silica gel Geduran Si 60 (0.063-0.200 mm), No. 110832, Merck, Darmstadt.
Deuterated solvents for NMR spectroscopy:
CDCl3, (99.96%), 0.03%TMS Euriso-top (C.E.Saclay, Gif-sur-Yvette, France) [D6]-DMSO (99.9%), 0.05% TMS Cambridge Isotop Laboratories (CIL), Andover MA, USA.
[D4]-Methanol: (99.8%)0.05% TMS Cambridge Isotop Laboratories (CIL), Andover MA, USA.

### Anhydrous (absolute or abs.) solvents

The solvents were purchased (from Fluka, Neu-Ulm), stored over molecular sieves and used without additional post-drying method. Anhydrous solvents and apparatuses employed with exclusion of water were blanketed with dry argon and kept under a gentle stream of dry argon.

### EI-MS

EI mass spectra were recorded from GC/MSD systems at 70 eV. The samples were dissolved in tetrahydrofuran (THF) or methanol, volume injected 1 µl, ALS split ratio 1:50, and measured using helium as carrier gas on a 5% phenyl-methyl silicone quartz capillary column. The temperature was in the range from 120 or 160°C to 280°C.

### NMR spectroscopy

The signals (chemical shifts) in the NMR spectra are reported relative to tetramethylsilane as internal standard (δ= 0 ppm). The chemical shift is reported in ppm (delta scale), and coupling constants are reported in Hz, ignoring the sign. Abbreviations used for first-order signals: s = singlet, d = doublet, dd= double doublet, t = triplet, q = quartet, qui = quintet, for 2nd-order signals: A or B, A,B or X
no assignment is made for higher order signals: m = multiplet,

### Infrared spectroscopy:

IR spectra are recorded in a diamond ATR system between 4000 cm ⁻¹ and 550 cm⁻¹ in absorption mode directly from solids or crystals.
Wave numbers (cm⁻¹) are recorded for the 10-20 most intense signals, together with the observed intensities in some examples.

Melting points are calibrated and corrected. The reference substances used are vanillin, phenacetin and caffeic acid standards.

The molecular weight and the molecular composition was calculated from the structure or the molecular formula.

The molecular composition is determined for carbon, hydrogen, nitrogen, sulfur and, if necessary, for halogen.

The compounds are named according to IUPAC rules.

Purity is determined as area percent (area % = proportion of the total of the integrated peak areas) measured via UV absorption at 230 nm for samples containing about 1.0 mg/ml, dissolved in dry MeOH (Uvasol, HPLC purity) and with a volume of 5-10 µl injected.

### Abbreviations:

- HPLC: high performance liquid chromatography
- m.p., mp: melting point
- RT: retention time
- THF: tetrahydrofuran
- MeOH: methanol
- EA, EtOAc: ethyl acetate
- DMF: dimethylformamide
- TLC: thin layer chromatography
- DCM: dichloromethane

### General preparation method 1- sulfoxides:

In a typical embodiment, 10-20 mmole (3.5 g-7 g) of the appropriate thio compound (e.g. compound a) to y)) is dissolved (30-100 ml, ~ 10 ml / g of precursor) or suspended in glacial acetic acid, and the suspension or solution is cooled in an ice bath to 0 -10°C and then stoichiometric amounts of a 35% strength aqueous hydrogen peroxide solution are added in slight excess (1.1 : 1.2 equivalents, 1-2 g) in 2-3 portions. The progress of the reaction is monitored by thin-layer chromatography, high pressure liquid chromatography or gas chromatography. If precursor is still detectable after the usual reaction time of 4-6 hours has elapsed, the reaction time can be extended to several hours (16-72 h), or the excess of hydrogen peroxide is raised to 2-3 equivalents.

If the sample shows no residual starting material, the reaction mixture is poured into ice-water (300-700 ml) and neutralized with 12.5 to 25% strength aqueous ammonia solution until pH 8 is reached, after which the product crystallizes out of the aqueous phase or separates as an oil, which crystallizes on standing in the cold.
The deposited solids are collected on a Buchner funnel and dried and, if necessary, purified by recrystallization from ethyl acetate or diethyl ether or by chromatography with ethyl acetate, ethyl acetate/methanol, ethyl acetate/THF or ethyl acetate/DMF (dimethylformamide) on silica gel or alumina. Substance fractions which elute early are discarded. There are obtained successively unreacted precursor in 5-10% yield and sulfone in 5-20% yield. The sulfoxide is present in the fractions which elute late. The yield of sulfoxide is typically 50-60% after column chromatography and 85-90% after recrystallization.

The acid addition salts are prepared by dissolving the imidazole bases in a suitable solvent such as ethyl acetate, THF, methanol, ethanol, isopropanol etc. This solution is then added to solutions of stoichiometric amounts of acids, e.g. gaseous HCl in ethanol, diethyl ether, isopropanol or aqueous HCl. The salts are then isolated in a conventional way.

### General preparation method 1a - sulfoxides:

1 equivalent of the corresponding [4-(3-alkyl or substituted alkyl-2-alkylsulfanyl-5-phenyl or 5-substituted phenyl-3H-imidazol-4-yl)-pyridin-2-yl]- alkyl-,cycloalkyl, aryl-or acyl-amine compound is taken up in a water-miscible solvent such as THF, dioxane, glyme, acetone and butanone or isopropyl-methylketone or mixtures thereof or mixtures of acetone and lower alcohols such as methanol, ethanol, isopropanol (~ 10 mL / g educt). An aqueous solution of the oxidation agent sodium metaperiodate is added to the water-miscible phase in one volume or in aliquots. To selectively obtain the sulfoxides stoichiometric amounts up to a small molar excess of periodate may be used in general. The educts may also be present in suspension. The suspension or solution is in general heated to the boiling temperature of the mixture (reflux) and the reflux is maintained for several hours to several days. The progress of the reaction is controlled by thin layer chromatography, HPLC or gas chromatography. If after the normal reaction time of 4 to 6 hours educt can be detected, the reaction time can be extended (16-72 h). An excess of sodium metaperiodate does in general not enhance the reaction but may result in increased formation of the corresponding sulfone. Advantageously, the reaction is terminated at 90-95% conversion. The selectivity for sulfoxide formation versus sulfone formation is then in general> 95%. Due to the lower polarity of the sulfanyl starting materials as compared to the sulfoxides traces of educts can be removed by extraction with lipophilic solvents (ethyl acetate, acetone, THF, diethylether) or by recrystallization from semi-polar organic solvents.

If the progress of the reaction is as desired (at most 0.5-1 % sulfone), the low-boiling organic components are evaporated. Unreacted starting materials and sulfones precipitate as solids. If required water may be added to dissolve undesired inorganic precipitates. The precipitated solids are then slurred with warm water, isolated by filtration and washed with cold water and dried. The solid material is purified by extraction with or recrystallization from ethyl acetate, acetone, THF or diethyl ether. Alternatively, the crude sulfoxides can be purified by chromatography on silica gel or aluminium oxide with ethyl acetate, ethyl acetate-methanol, ethyl acetate-THF or ethyl acetate-DMF as eluent.

The oxidation of the thio compounds results in racemates of the sulfoxides which can be resolved into the pure enantiomers by enantiomer separation. The pure enantiomers are isolated from the racemates preferably by preparative (high pressure) column chromatography with high enantiomeric purity (ee > 95%) by use of chemically modified celluloses and chemically modified starches, such as, for example, Chiralpak OD, Chiralpak AD, Chiralpak OJ, as stationary phase (chiral stationary phase = CSP). The eluent particularly preferably used comprises isopropanol-aliphatic hydrocarbon mixtures as eluent with an isopropanol content of 10-90%, particularly preferably under isocratic conditions with an isopropanol content of 60-80%.

A further possibility for separating into the enantiomers consists of salt formation and crystallization with enantiopure acids such as, for example, dextrorotatory L-(+)-lactic acid L-(+)-mandelic acid, (1 R)-(-)-camphor-10-sulfonic acid or (1 S)-(+)-camphor-10-sulfonic acid.

Oxidation of chiral precursor compounds to sulfoxides results in mixtures of diastereomers which can be separated in a conventional way, e.g. by crystallization.

### General preparation method 2 - sulfones:

1 mmole (~ 0.3-0.4 g) of the appropriate thio compound is suspended (30-100 ml, ~ 10 ml / g of precursor) or dissolved in glacial acetic acid, and this suspension or solution is heated to 40-50°C in a heating bath. An excess of 35% strength aqueous hydrogen peroxide solution (3 to 9 equivalents; 0.3 g-1 g) is added in one portion, and the process of the reaction is monitored by thin-layer chromatography, high pressure liquid chromatography or gas chromatography. If precursor is still detectable after the usual reaction time of 4-6 hours has elapsed, the reaction time can be extended to several hours (16-72 h), or the reaction temperature is raised further to 60-70°C. If the sample shows no starting material left, the reaction mixture is poured onto ice-water (300-700 ml) and neutralized with 12.5 to 25% aqueous ammonia solution until pH 8 is reached. The product crystallizes on standing or separates out as oil from the aqueous phase. The deposited solids are collected on a Buchner funnel, dried and, if necessary, purified by recrystallization from ethyl acetate or diethyl ether or by chromatography with ethyl acetate, ethyl acetate/methanol, ethyl acetate/THF or ethyl acetate/DMF on silica gel or alumina. There are obtained successively imidazole N-oxide sulfoxides in 10-30% yield and sulfones in 50-60% yield. Small amounts of sulfoxides are obtained from fractions which elute late, typically <10% yield.

### General preparation method 3 - salts:

### 3.1 Preparation of methane sulfonates

A solution of methane sulfonic acid in THF (1 M) is added to an approximately 2.5% by weight solution of the compound in THF (prepared by gentle warming) in stoichiometric amount. Upon cooling colorless crystals are formed after 5 to 10 minutes. Crystallization is completed by cooling to 3-5 °C for several hours. The precipitated salt is isolated by filtration and washed with a small amount of diisopropylether (2 x 1ml) and dried for several hours under vacuum at 40 to 50 °C.

### 3.2 Preparation of hydrochlorides

A 1.25 M solution of HCl in isopropanol is added to an approximately 2.5% by weight solution of the compound in THF (prepared by gentle warming) in stoichiometric amount. The salt is then isolated as given under 3.1.

### 3.3 Preparation of the hydrobromides

A 1 M solution of HBr in THF is added to an approximately 2.5% by weight solution of the compound in THF (prepared by gentle warming) in stoichiometric amount. The salt is then isolated as given under 3.1.

### 3.4 Preparation of the hydrogensulfates and sulfates

A 1 M solution of H₂S0₄ (96%) in THF is added to an approximately 2.5% by weight solution of the compound in THF (prepared by gentle warming) in stoichiometric amount. The solvent is evaporated under vacuum and the residue is suspended in diisopropylether, the crystalline solid is filtered off, washed with diisopropylether and dried.

For the preparation of the sulfates 0.5 equivalents of sulphuric acid are used.

### General preparation method 4 - fluoropyridyl-sulfanyl- and fluoropyridyl-sulfinyl-imidazole precursors for nucleophilic replacement by amines

General procedure for introduction of fluorine in place of amino function of 4-5-aryl-sulfanyl-imidazol-4-yl]-pyridin-2-ylamine precursors by nitrosation and diazonium replacement with HF or HBF₄.

The amino compounds (11) prepared according to the methodology of WO 02 /066458 were obtained by acidic hydrolysis from the acetamido-pyridyl precursors (10) following the sequence in reaction scheme 1.

As long as there is no other functional group present in the amino-precursor molecule, which is sensitive to the strong acidic conditions (acetals), to fluoride (silanes) or sensitive against nitrosation and diazotation (primary and secondary amines), the amino group may be transformed to the diazonium group by introducing the alkali nitrite under aqueous conditions to the HBF₄ acidic solution of these precursors. This solution is made by dissolving the aminopyridyl base in an aqueous or methanol solution of tetra fluoro boric acid (HBF₄) or by dissolving the base directly in Olah's reagent (70% HF in pyridine). Diazotizing with nitrous acid esters (i.e. isoamyl and isobutyl nitrite) under non-aqueous conditions is possible when Olah's reagent is used to dissolve the amino pyridyl base.

In cases where fluoride sensitive or acid sensitive functional groups are present or where primary or secondary (even tertiary) amino groups are present (R¹ and R²) a modified and optimized strategy is necessary to make these precursors available. The 1-(2-amino-pyridin-4-yl)-2-(4-fluoro-phenyl)-ethanone, which was prepared according to WO 02/066458, can be converted to the 2-(4-fluoro-phenyl)-1-(2-fluoropyridin-4-yl)-ethanone either with NaNO₂ under aqueous conditions with tetrafluoro boric acid solutions (methanol or water) or with isoamyl nitrite. More easily and with higher yields said conversion can be performed with Olah's reagent and sodium nitrite. Nitrosation of the CH-acidic α-carbon position of the diaryl-ethanone was never observed. This makes it necessary to perform the nitrosation/oximation of this intermediate 2-(4-fluoro-phenyl)-1-(2-fluoro-pyridin-4-yl)-ethanone as a separate step. According to WO 2004/ 018458 the nitrosation/ oximation was achieved analogously in glacial acetic acid with sodium nitrite in place of isoamyl nitrite in the presence of sodium methoxide.

In accordance with scheme 1 of WO 02 /066458 the 1-(4-fluoro-phenyl)-2-(2-fluoropyridin-4-yl)-ethane-1,2-dione 1-oxime (7') can be condensed with diverse (R¹ substituted) hexahydro-triazins, which are readily available from paraformaldehyde and corresponding amines, to obtain 3-oxo-imidazol intermediates (8'). For the preparation of the thion intermediate (9') *Mlostons* procedure (Mloston G.; Gendek, T.; Heimgartner, H.; Helv. Chim. Acta. 1998. 81; (9): 1585-1595) was applied to the fluoropyridin-imidazol-N-oxid derivatives. R² can then be introduced by alkylation with iodides or sulfonates in the presence of alkali hydrogen carbonate or alkali carbonate to obtain the 2-sulfanyl-substituted starting materials (10') which can be used for subsequent amination reaction.

Amino-fluoro-replacement reaction can also be performed on the sulfinyl level by first oxidizing the fluoropyridin-sulfanyl compounds according to the above general methods, either with H₂O₂/glacial acetic acid or with the NalO₄ method, and then proceeding as described above.

### General preparation method 5 - Acylation of the 2-aminopyridyl group with acid chlorides

The 2-aminopyridyl compound (1 equivalent) is dissolved in abs. pyridine and the corresponding acid chloride (1 equivalent) is added dropwise. The reaction is completed with stirring at 55 °C (control by TLC)). The pyridine is then removed under vacuum; the residue is taken up in ethyl acetate and washed several times with water. The organic phase is dried with anhydrous sodium sulfate and the ethyl acetate is removed under vacuum. The crude product was purified by column chromatography.

### General preparation method 6 - Acylation of the 2-aminopyridyl group with carboxylic acids

The carboxylic acid (1 equivalent) is dissolved in 50 ml abs. THF under argon atmosphere at room temperature. Carbonyldiimidazole (CDl, 1 equivalent) is then slowly added. After the gas evolution has ceased (1.5 h) pyridyl amine (1 equivalent) is added. The reaction mixture is then stirred at room temperature until completion of the reaction. THF is evaporated; ethyl acetate is added to the residue and washed several times with water. The organic phase is dried with anhydrous sodium sulfate and the ethyl acetate is removed under vacuum. The crude product was purified by column chromatography by means of MPLC (RP-18, acetonitrile : water = 6 : 4).

### Preparation of the starting compounds:

The compounds of the invention described in the examples were obtained using the compounds of WO 02/066458A2 which were prepared by the processes described therein:

(The numbers of the compounds refer to scheme 1)

### a) 2-Acetamido-4-methylpyridine (2)

200.0 g of 2-aminopicoline (1) are mixed with 400 ml of acetic anhydride and with 100 mg of 4-dimethylaminopyridine and refluxed for 5 h. After cooling, the excess acetic anhydride is substantially distilled off, and the residue is poured onto ice and neutralized with aqueous ammonia solution. The precipitate of (2) which separates out during this is filtered off and dried in vacuum over P₂O₅. Yield: 209.0 g (75%)

### b) 2-Acetamidopyridine-4-carboxylic acid (3)

214.0 g of (2) are introduced in portions with stirring into an aqueous solution of 160 g of potassium permanganate at 50°C. A further 360 g of potassium permanganate are added in portions over the course of one hour. The temperature of the reaction mixture should not exceed 90°C during this. The mixture is then stirred for 1.5 h and filtered hot, and the filtrate is adjusted to pH 3-4 with conc. HCl. The white precipitate of (3) which separates out is filtered off and dried in vacuum over P₂O₅.
Yield: 108.0 g (42%)

### c) 2-Cyano-2-(4-fluorophenyl)-1-(2-acetamido-4-pyridyl)ethanone (4)

18.0 g of (3) are taken up in 50 ml of abs. dimethylformamide (DMF) and, after addition of 17.0 g of carbonyldiimidazole (CDI), stirred at room temperature for 45 min. Then 14.9 g of 4-fluoroacetonitrile and 14.6 g of potassium tert-butanolate are added, and the reaction mixture is heated at 120°C for 2 h. After cooling, the mixture is stirred at room temperature overnight. Ice is then added to the solution, and it is neutralized with conc. HCl. The precipitate of (4) which separates out is filtered off and dried in vacuum over P₂O₅.
Yield: 18.1 g (65%)

### d) 2-(4-Fluorophenyl)-1-(2-amino-4-pyridyl)ethanone (5)

27.9 g of (4) are mixed with 150 ml of 48% strength hydrobromic acid, and the reaction mixture is kept at a gentle boil for 30 h. After cooling, the mixture is poured onto ice and neutralized with concentrated ammonia. The precipitate of (5) which separates out is filtered with vigorous suction, washed several times with petroleum ether and cold diethyl ether and dried.
Yield: 11.7 g (55%)

### e) 2-(4-Fluorophenyl)-1-(2-acetamido-4-pyridyl)ethanone (6)

12.0 g of compound (5) are suspended in 100 ml of acetic anhydride and, after addition of a spatula tip of 4-dimethylaminopyridine, the reaction mixture is refluxed for 5 h. The excess acetic anhydride is substantially distilled off, and the residue is hydrolyzed and adjusted to pH 7 with conc. ammonia. The pale precipitate of (6) which separates out is filtered off and dried in vacuum over P₂O₅.
Yield: 13.5 g (94%)

### f) 2-(4-Fluorophenyl)-1-(2-acetamido-4-pyridyl)-α-hydroxyiminoethanone (7)

2.1 g of sodium methoxide solution (30% in methanol) are mixed with 30 ml of methanol and added to a solution of 1.2 g of isoamyl nitrite in 20 ml of methanol. While stirring, 3.0 g of (6) are added in portions, and then stirring is continued at room temperature for 2 h. The solvent is distilled off, and the solid residue is taken up in water and adjusted to pH 7 with 10% strength HCl. The pale precipitate of (7) which separates out is filtered off and dried in vacuum over P₂O₅.
Yield: 1.8 g (54%)

### g) Preparation of compound (8):

(7) is dissolved together with twice the amount of the appropriate triazine in absolute ethanol and refluxed until the precursor has completely reacted. After cooling, ethanol is removed in a rotary evaporator. The partly oily residue solidifies on addition of diethyl ether. The precipitate of compounds (8) is filtered off and dried in vacuum.
Yields: R¹= -CH₃ : 74%
R¹= -C₃H₇: 62%
R¹= 2,2,6,6-tetramethylpiperidin-4-yl: 81%
R¹= N-morpholinopropyl-: 72%
R¹= 3-hydroxypropyl-: 56%

### h) Preparation of compound (9)

Compound (8) is dissolved in CHCl₃, and the reaction mixture is cooled in an ice bath. An equimolar solution of 2,2,4,4-tetramethyl-cyclobutan-1,3-dithione in CHCl₃ is slowly added dropwise, and the mixture is then stirred in the ice bath for 30 min. The ice bath is removed and stirring is continued at room temperature for 1 h. The solvent is then removed in a rotary evaporator, and the solid residue is stirred in diethyl ether. The precipitate of (9) is filtered off and dried in vacuum.
Yields: R¹= -CH₃ : 96%
R¹= -C₃H₇: 74%
R¹=2,2,6,6-tetramethylpiperidin-4-yl: 61 %
R¹= N-morpholinopropyl-: 82%
R¹= 3-hydroxypropyl-: 71 %

### i) Preparation of compound (10)

Compound (9) is suspended in abs. ethanol under protective gas, and the equimolar amount of methyl iodide is added. After addition of a spatula tip of Na₂C0₃, the reaction mixture is refluxed until the precursor has completely reacted. After cooling, the inorganic salts are filtered off, and the solvent is removed in a rotary evaporator. The crude product (10) is purified by column chromatography.

### j) 4-(4-Fluorophenyl)-1-methyl-5-(2-acetamido-4-pyridyl)-2-methylthioimidazole

R¹= -CH₃: yield 63%
NMR (CDCl₃, ppm): 8.75 (bs, 1H), 8.26-8.24 (m, 2H), 7.46- 7.39 (m, 2H), 6.97- 6.88 (m, 3H), 3.53 (s, 3H), 2.71 (s, 3H), 2.23 (s, 3H)
IR (1/cm): 1669, 1607, 1543, 1505, 1416, 1268, 1218, 843

### k) 4-(4-Fluorophenyl)-1-n-propyl-5-(2-acetamido-4-pyridyl)-2-methylthioimidazole

R¹= -C₃H₇: yield 28%
NMR (CDCl₃, ppm): 8.28- 8.25 (m, 2H), 7.44-7.37 (m, 2H), 6.96-6.88 (m, 2H), 3.85 (t, 2H, J = 7.7 Hz), 2.73 (s, 3H), 2.24 (s, 3H), 1.65-1.57 (m, 2H), 0.83 (t, 3H, J = 7.4 Hz)
IR (1/cm): 3303, 1674, 1544, 1501, 1416, 1264, 1213, 845

### l) 4-(4-Fluorophenyl)-1-(2,2,6,6-tetramethylpiperidin-4-yl)-5-(2-acetamido-4-pyridyl)-2-methylthioimidazole

R¹ = 2,2,6,6-tetramethylpiperidin-4-yl: yield 23%
NMR (CDCl₃, ppm): 10.62 (s, 1 H), 8.38-8.35 (m, 2H), 8.01 (s, 1 H), 7.33-7.26 (m, 2H), 7.04-6.95 (m, 3H), 4.19- 4.03 (m, 1H), 2.61 (s, 3H), 2.00 (s, 3H), 1.87 -1.81 (m, 2H), 1.52-1.47 (m, 2H), 0.93 (s, 6H), 0.78 (s, 6H)
IR (1/cm): 2976, 1699, 1533, 1407, 1255, 838

### m) 4-(4-Fluorophenyl)-1-[3-(N-morpholino)propyl]-5-(2-acetamido-4-pyridyl)-2-methylthioimidazole

R¹= N-morpholinopropyl-: yield 52%
NMR (CDCl₃, ppm):8.29 (m, 1 H), 8.12 (s, 1 H), 7.42-7.35 (m, 2H), 6.96-6.87 (m, 3H), 4.08-3.92 (m, 6H), 3.17-3.00 (m, 6H), 2.74 (s, 3H), 2.41-2.34 (m, 2H), 2.24 (s, 3H)

### n) 4-(4-Fluorophenyl)-1-(3-hydroxypropyl)-5-(2-acetamido-4-pyridyl)-2-methylthioimidazole

R¹= 3-hydroxypropyl: yield 32%
NMR(CDCl₃, ppm):8.69 (bs, 1 H), 8.23-8.19 (m, 2H), 7.44-7.37 (m, 2H), 6.98-6.86 (m, 3H), 4.04 (t, 2H, J= 7.9 Hz), 3.70 (t, 2H, J= 7.2 Hz), 2.74 (s, 3H), 2.25 (s, 3H), 2.13-2.05 (m, 2H)

### o) 4-(4-Fluorophenyl)-1-methyl-5-(2-amino-4-pyridyl)-2-methylthioimidazole

Compound j) is dissolved in 10% strength HCl and refluxed for 14 h. After cooling, 20% strength NaOH is used to neutralize. The pale precipitate which separates out is filtered off and dried in vacuum over P₂O₅.
Yield: 82%
NMR (CDCl₃, ppm): 8.16-8.13 (m, 1 H), 7.50-7.43 (m, 2H), 6.98-6.89 (m, 2H), 6.60-6.57 (m, 1 H), 6.41 (s 1 H), 4.60 (bs, 2H,) 3.46 (s, 3H), 2.70 (s, 3H)
IR(1/cm): 1629, 1542, 1509, 1215, 837, 814.

### p) 2-Fluoro-4-[5-(4-fluorophenyl)-3-(2-methoxyethyl)-2-methylsulfanyl-imidazol-4-yl]-pyridine

### A) 2-(4-fluorophenyl)-1-(2-fluoropyridin-4-yl)-ethanone

To Olah's reagent (58.0 g; 70% HF in pyridine) in a 100 mL FEP bottle (Perfluoroethylene propylene) cooled to -10 °C 1-(2-aminopyridin-4-yl)-2-(4-fluorophenyl)-ethanone (16.11 g) was added and the mixture was stirred. Over a period of 50 min. NaNO₂ (7.87 g) was added in small portions. After each aliquot the reaction bottle was closed loosely. As the inner temperature was kept below 0 °C, only little nitrous gases (with foaming) evolved. The reaction mixture turned yellow. After the last addition the stirring was continued for 1 h at 0°C and for 1 additional hour at room temperature. Water (200 mL) was poured into the mixture while stirring. CH₂Cl₂ (125 mL) was added and the layers were separated in a separatory funnel. The aqueous layer was extracted with CH₂Cl₂ (3 x 75 mL). The CH₂Cl₂ fractions were combined and washed with CaCO₃-solution (100 mL, 5 %) and water (100mL), dried over Na₂SO₄ , and the solvent was stripped off *in vacuum.* The residue obtained was treated several times with hot n-hexane. The title compound crystallized from the n-hexane extracts in the cold (refrigerator at 3-5°C) with 95% purity.

As an alternative the raw material obtained can be purified by column chromatography (cc): SiO₂/EtOAc-n-hexane=3:7. Yield: 10.8 g (66.3%)
Purity: 99% HPLC (after cc).
¹H NMR: (DMSO-d6) δ (ppm) = 4.503 (s, 2H, CH₂); 7.144-7.196 (m, 2H, C3/5, 4-F-Ph); 7.299-7.335 (m, 2H, C2/6, 4-F-Ph); 7.729 (s, 1 H, C3-H, Pyr); 7.851-7.872 (m, 1 H, C5-H, Pyr); 8.481-8.494 (m, 1 H, C6-H, Pyr);

### B) 1-(4-fluorophenyl)-2-(2-fluoropyridin-4-yl)-ethane-1,2-dione-1-oxime

2-(4-Fluorophenyl)-1-(2-fluoropyridin-4-yl)-ethanone (2.56 g; 11.0 mmole) was dissolved in glacial acetic acid (26 mL). An aqueous saturated solution of sodium nitrite (2.25 g, 32.0 mmole) was added dropwise at room temperature in such a rate that formation of nitrous gases was avoided. The slightly yellow solution was stirred overnight. Water (80 mL) was added and the suspension formed was stirred for at least 1 hour. The crystals were collected on a funnel by suction filtration and washed on the funnel with some aliquots of dematerialized water and finally with hexane. Yield 2.8 g (98%),
mp: 166 °C.
1H NMR: δ (ppm) (DMSO-d6) 7.291-7.344 (m, 2H, C3/5, 4-F-Ph); 7.537-7.588 (m, 3H, C2/6, 4-F-Ph;C3-H, Pyr);7.677 -7.697 (m, 1 H, C5-H, Pyr); 8.402-8.417 (m, 1 H, C6-H, Pyr); 13.105 (s, 1H, OH);

### C) (2-fluoro-4-[5-(4-fluorophenyl)-3-(2-methoxyethyl)-1-oxy-3H-imidazol-4-yl]-pyridine

To a suspension of 1-(4-fluorophenyl)-2-(2-fluoropyridin-4-yl)-ethan-1,2-dion-1-oxime (10.48 g; 0.04 mole) in ethanol (180 mL) 1,3,5-tris-(2-methoxy-ethyl)-[1,3,5]triazinane (5.12 g, 0.0196 mole) dissolved in ethanol (20 mL) was added at once. The mixture was brought to reflux temperature (90 °C) and refluxing conditions were held for 20 h. Work up started by stripping off the ethanol on a rotavapor and the residual solid was taken up in diethyl ether (100 mL). After 12 h storage of the ethereal suspension, the crystals were filtered from the mother liquor on a Buchner filter and dried at 45°C at 10 mbar.
C₁₇H₁₅F₂N₃O₂ (Mr 331,32)
Yield 9,44 g (91 %)
Purity (HPLC area method) > 99%

### (D) 4-(4-fluorophenyl)-5-(2-fluoropyridin-4-yl)-1-(2-methoxyethyl)-1,3-dihydroimidazole-2-thione

2-Fluoro-4-[5-(4-fluorophenyl)-3-(2-methoxyethyl)-1-oxy-imidazol-4-yl]-pyridine (9.44 g, 0.0285 mole), prepared according to step B, was suspended in CH₂Cl₂ (120 mL). While keeping the suspension at 0°C in an ice cooling bath a solution of 2,2,4,4-tetramethyl-cyclobutane-1,3-dithion (3.1 g, 0.018 mole) in CH₂Cl₂ (30 mL) was added dropwise. After about 15 min the clear solution was allowed to warm to room temperature and stirring was continued for 2 hours. After this time the product, which crystallized from the solution, was filtered from the mother liquor. A second crop was obtained when the volume of the mother liquor was reduced to half of the initial volume and the reduced volume substituted by the same volume of diisopropyl ether. The first and second crop were combined and dried.
C₁₇H₁₅F₂N₃OS (Mr 347,39):
Yield 8,49 g (88%)
Purity (HPLC area method) 95%;
mp: 209 °C,
GC-MS: 9,39 min m/z (%) 347 (22), 289 (100), 230 (5);
IR (λ[cm-1]): 3069, 2972, 2900, 1608, 1493, 1407, 1395, 122 (4-FPh), 1119 (=S), 881,844,815

### E) 2-fluoro-4-[5-(4-fluorophenyl)-3-(2-methoxy-ethyl)-2-methylsulfanyl-imidazol-4-yl]-pyridine

A suspension of 4-(4-fluorophenyl)-5-(2-fluoropyridin-4-yl)-1-(2-methoxyethyl)-1,3-dihydro-imidazol-2-thion (8.42 g, 23.5 mmole) in methanol (150 ml) was prepared. After adding potassium carbonate (2.68 g, 19 mmole) a solution of methyl iodide (4.47 g; 32 mmole) in MeOH (30 mL) was added dropwise. The mixture was stirred for 20 h at room temperature. The volume of the suspension was reduced under vacuum to dryness. The residual solids were partitioned between a mixture of ethyl acetate and water (250 mL, 3:2). The aqueous layer was reextracted with ethyl acetate and removed. The combined organic layers were washed with water, dried over Na₂SO₄ sicc. and evaporated. The raw material was recrystallized from diisopropyl ether. This material is suitable to be used for fluorine-amine-replacement reaction.
C₁₈H₁₇F₂N₃OS (MG 361.42)
Yield 7.9 g (89%)
Purity (HPLC area % ; RT=7.6 min.): >99%.
GC-MS: 7.81 min m/z (%) 361 (100),330 (19), 303 (21), 270 (81), 121 (14)
IR (λ[cm⁻¹]): 3061, 2925, 2890, 1609, 1542, 1506, 1390, 1222 (4-FPh), 1121, 880, 851,828.

### 2-Fluoro-4-[5-(4-fluorophenyl)-3-methyl-2-methanesulfinyl-3H-imidazol-4-yl]-pyridine

The compound was prepared from 2-fluoro-4-[5-(4-fluorophenyl)-3-methyl-2-methylsulfanyl-3H-imidazol-4-yl]-pyridine (0.952 g, 0.003 mole) in glacial acetic acid (10 mL) and a solution of hydrogen peroxide 30% (0.36 g; 0.0032 mol) in glacial acetic acid (1 mL), reaction time 168 h (7 d). After completion the mixture was poured onto ice water (15 mL). The solution was made alkaline (pH 8-9) with ammonia (32%). The precipitated product was taken into ethyl acetate (40 mL), while the alkaline aqueous layer was extracted five times with ethyl acetate (20 mL). The combined organic extracts were washed with water (20 mL), dried over Na₂SO₄ and evaporated. The white crystalline material is suitable for preparation of the 2-amin-substituted pyridines without further purification.
C₁₆H₁₃F₂N₃OS (Mr 333,36):
Yield: 870 mg (90%);
Purity: from HPLC area: 5,66 min 84,35%.
GC-MS: 7.99 min m/z (%) 333 (27), 317 (100), 284 (82), 244 (47);
IR (λ[cm-1]):1617, 1541, 1509, 1407, 1221 (4-FPh), 1194, 1160, 1053, 950,881, 847, 657.

### 2-Fluoro-4-[5-(4-fluorophenyl)-2-methanesulfinyl-3-(2-methoxyethyl)-3H-imidazol-4-yl]-pyridine

A solution of 2-fluoro-4-[5-(4-fluorophenyl)-3-(2-methoxyethyl)-2-methylsulfanyl-3H-imidazol-4-yl]-pyridine (0.903 g; 0.0025 mole) in glacial acetic acid (10 mL) and solution of hydrogen peroxide 30% (0.3 g; 0.0026 mol) in glacial acetic acid (1 mL) were mixed and stirred at room temperature (according to general procedure 1); reaction time: 84 h (3,5 d).). The mixture was poured onto ice water (10 mL). The solution was made alkaline (pH 8-9) with ammonia (32%) and extracted five times with 15 mL ethyl acetate. The combined ethyl acetate extracts were washed with water (10 mL), dried over Na₂SO₄, filtrated and the solvent was removed. Purification was achieved by cc: Al₂O₃/Eluent: n-hexane= 2:1
After removal of the solvent the product crystallized from n-hexane.
C₁₈H₁₇F₂N₃O₂S *(Mr 377,42):* Yield: 704 mg (79%);
Purity: from HPLC area: (RT=6,2 min.) 99%.
GC-MS: (RT=8.46 min.); m/z (rel. int. [%]) 377 (1), 361 (100), 330 (16), 303 (15), 270 (65), 121 (10).
IR (λ[cm⁻¹]): 2972, 2931, 2895, 1610, 1508, 1397, 1220 (4-FPh), 1051 (SO), 880, 840
¹H-NMR (DMSO-d6): δ (ppm) 3.1 (s, 1-H, SOCH₃); 3.13 (s, 3-H, OCH₃); 3.23-3.50 ( m, 2-H, N-CH₂-CH₂-OCH₃ near water resonance 3.3 ppm); 4.23-4.39 (m, 2-H, N-CH₂-CH₂-OCH₃); 7.13-7.17 (m, 2-H, C3-H pyr., C5-H pyr.); 7.37-7.41 (m, 4-H, C3/5-H 4-FPh +C2/6-H 4-FPh); 8.40 (d, 1-H, J= 5.2 Hz, C6-HPyr

### Preparation of 2-fluorpyridine-substituted compounds with the basic structure of 2,3-dihydroimidazo[2,1-b]thiazoles and 6,7-dihydro-5H-imidazo[2,1-b]thiazines

### - Alternative preparation method of 4-[6-(phenyl)-2,3-dihydro-imidazo[2,1-b]thiazol-5-yl]-pyridin-2-ylamines and 4-[2-(Phenyl)-6,7-dihydro-5H-imidazo[2,1-b][1,3]thiazin-3-yl]-pyridin-2-ylamines

The conversion of 1-(2-amino-pyridin-4-yl)-2-(4-fluoro-phenyl)-ethanone to 2-(4-fluoro-phenyl)-1-(2-fluoro-pyridin-4-yl)-ethanone is described above. In accordance to the reaction scheme of the acetamido-analogue N-(4-{2-(4-fluoro-phenyl)-2-[(E/Z)-hydroxyimino]-acetyl}-pyridin-2-yl)-acetamide (WO 02/066458) 1-(4-fluoro-phenyl)-2-(2-fluoro-pyridin-4-yl)-ethane-1,2-dione 1-oxime can be condensed with 1,3,5-tris-2-hydroxyethyl-1,3,5-hexahydrotriazine under heating in ethanol. The triazine can be made from paraformaldehyde (1 part) and 2-amino-ethanol (3 parts). 2-[4-(4-Fluorophenyl)-5-(2-fluoro-pyridin-4-yl)-3-oxy-imidazol-1-yl]-ethanol is obtained. Reaction with 1,3,5-tris-3-hydroxypropyl-1,3,5-hexahydrotriazine results in an analogous manner in a 3-[4-(4-fluoro-phenyl)-5-(2-fluoro-pyridin-4-yl)-3-oxy-imidazol-1-yl]-propan-1-ol.
Both fluoropyridine derivates are converted in analogy to the acetamido-pyridine-compounds to the 2-thiooxo-imidazoles by means of 1,3-dipolar cycloaddition to 2,2,4,4-tetramethyl-cyclobutan-1,3-dithione and subsequent CSO (thioxo-methanone) extrusion. The resulting products are 4-(4-fluoro-phenyl)-5-(2-fluoropyridin-4-yl)-1-(2-hydroxy-ethyl)-1,3-dihydro-imidazol-2-thione and 4-(4-fluorophenyl)-5-(2-fluoro-pyridin-4-yl)-1-(3-hydroxy-propyl)-1,3-dihydro-imidazol-2-thione.

### 2-[4-(4-Fluoro-phenyl)-5-(2-fluoro-pyridin-4-yl)-3-oxy-imidazol-1-yl]-ethanol

1-(4-Fluoro-phenyl)-2-(2-fluoro-pyridin-4-yl)-ethan-1,2-dion-1-oxime (7.87 g) was mixed with 1,3,5- tris-2-hydroxyethyl-1,3,5-hexahydrotriazine (2.85 g) in EtOH (150 mL) and refluxed for 16 h (overnight). Ethanol was removed in a rotary evaporator. The oily, sticky residue could by used directly for the following process.
The product crystallizes after adding ether. In case the crystals are too sticky the ether can be combined with ethanol (95:5). The filtered crystals can be washed with Ether/EtOH (95:5).
Yield 7.6 g (80%);
Purity (HPLC) : > 99,9%
GC-MS: 70 eV EI-MS: 301 (1 00); 270(21); 256(26); 243(5); 215(5), 202(6), 121 (10)

### 4-(4-Fluoro-phenyl)-5-(2-fluoro-pyridin-4-yl)-1-(2-hydroxy-ethyl)-1,3-dihydro-imidazol-2-thione

To a solution of crude 2-[4-(4-fluoro-phenyl)-5-(2-fluoro-pyridin-4-yl)-3-oxy-imidazol-1-yl]-ethanol (13,5 g containing 47%, equivalent to 6.34 g pure material) dissolved in 60 mL CH₂Cl₂ a solution of 2,2,4,4-tetramethyl-cyclobutan-1,3-dithione (3,38 g) in 20 mL CH₂Cl₂ was added dropwise within 30 min. A precipitate was separating from the organic layer. The solution was stirred at RT overnight, then the precipitate was evaporated on a Buchner funnel and washed with CH₂Cl₂ (5mL) and ether (3 mL). Yield:6.35 g (95%),
Purity (HPLC): 93%
GC-MS: 70 eV EI-MS: 315(100); 285(5); 164(9), 121(16).

### 6-(4-Fluoro-phenyl)-5-(2-fluoro-pyridin-4-yl)-2,3-dihydro-imidazo[2,1-b]thiazole

4-(4-Fluoro-phenyl)-5-(2-fluoro-pyridin-4-yl)-1-(2-hydroxy-ethyl)-1,3-dihydro-imidazol-2-thione (6.35g) was dissolved in 21 mL pyridine at 50° C, then methanesulfonic acid chloride (1.98 mL) was added dropwise. After stirring at RT overnight (22h) the content of product was 71 %. No educt could be detected. The precipitate was filtered and washed with cold isopropanol: 3.67 g (yield 68.5 %) of pure product. Purity >99% (HPLC). The mother liquor was combined with cold isopropanol. A second fraction of precipitate was obtained which was filtered and washed with cold isopropanol.
Yield: 2,27 g (29 %).
GC-MS: 70 eV EI-MS: 315(100); 286(10); 165(10); 121(18)

### Example 1

### (1-Ethyl-pyrrolidin-2-ylmethyl)-{4-[5-(4-fluoro-phenyl)-3-methyl-2-methylsulfanyl-3H-imidazol-4-yl]-pyridin-2-yl}-amine

The title compound was obtained from 1.1 g (0.0035 mole) 2-fluoro-4-[5-(4-fluorophenyl)-3-methyl-2-methylsulfanyl-3H-imidazol-4-yl]-pyridine and 2.29 g (0.017 mole) C-(1-ethyl-pyrrolidin-2-yl)-methylamine after a reaction time of 4 h at 130 °C. The mixture was dissolved in ethyl acetate (20 mL) and the organic layer was washed eight times with 10 mL water. The aqueous phases were combined and extracted with 10 mL ethyl acetate. The organic phases were dried over Na₂SO₄ and the solvent was removed. The product crystallized.
C₂₃H₂₈FN₅S (Mr 425,58)
Yield: 1.29 g (89%) Purity (HPLC area % RT=3.8 min.) 97%
LC-MS: m/z (MH)+ 426
IR (λ[cm⁻¹]): 3227 (NH), 2962, 2931, 2870, 2784, 1605, 1559, 1505,1432, 1302, 1218 (4-FPh), 1153, 835, 813
¹H-NMR (DMSO-d6) δ (ppm) 1.01 (t, J= 6.8 Hz, 3H, CH₃); 1.50-1.56 (m, 1-H, C3-H Pyrrolidine); 1.59-1.66 (m, 2-H, C4-H Pyrrolidine);1.76-1.83 (m, 1-H, C3-H, Pyrrolidine); 2.04-2.19 (m, 2-H,C5-H, Pyrrolidine, N-**CH₂**-CH₃); 2.5-2.51 (m, 1-H, C2-H,
Pyrrolidine under DMSO Peak); 2.63 (s, 3-H, SCH₃); 2.76-2.81 (m, 1-H, N-**CH₂**-CH₃); 2.973.05 (m, 2-H, C5-H, Pyrrolidine, NH-**CH₂**-Pyrrolidine (1H von **CH₂**); 3.39 (s 3-H,NCH₃); 3.38-3.46 (m, 1-H NH-**CH₂**-Pyrrolidine) 6.44-6.48 (m, 3-H, C5-H +C3-H Pyr,+ N-H ); 7.07-7.12 (m, 2-H, C3/5-H 4-FPh); 7.43-7.46 (m, 2-H, C2/6-H 4-FPh); 8.06 (d, J= 6.0 Hz, 1-H, C6-H, Pyr)
¹³C-NMR (DMSO-d6) δ (ppm) 13.81(1-C, CH₃); 15.25 (1-C, S-CH₃); 22.25 (1-C, 4-C, Pyrrolidine); 28.65 (1-C, C3, Pyrrolidine); 31.37 (1-C, NCH₃); 44.32 (1C, NH-**CH₂**-CH-Pyrrolidine); 48.00 (1-,C, N-**CH₂**-CH₃); 53.11 (C5, Pyrrolidine); 62.35 (1-C, C2, Pyrrolidine); 108.67 (1-C, C3, Pyr); 112.22 (1-C, C5, Pyr); 114.94 (d, 2-C, ²J(C,F)=21.43 Hz, 4-FPh); 128.03 (d, 2-C ³J(C,F)=8.55 Hz, 4-FPh), 128.68; 130.61 (d, 1-C, ⁴J=3.019 Hz, C1 4-FPh); 136.33; 138.29; 143.26 ; 148.48 (1-C, C6 Pyr); 159.48; 160.85 (d, 1-C,¹J(C,F)=243.12 Hz)

### Example 2

### 1-Ethyl-pyrrolidin-2-ylmethyl)-{4-5-(4-fluoro-phenyl)-3-(2-methoxy-ethyl)-2-methylsulfanyl-3H-imidazol-4-yl]-pyridin-2-yl}-amine

0,903 g (0.0025 mole) 2-fluoro-4-[5-(4-fluoro-phenyl)-3-(2-methoxy-ethyl)-2-methylsulfanyl-3H-imidazol-4-yl]-pyridine and 3.21 g (0.0238 mole) C-(1-ethyl-pyrrolidin-2-yl)-methylamine were reacted in a closed tube under slight pressure for 4 h at 120 °C. The mixture was dissolved in ethyl acetate (30 mL) and the organic layer was extracted with water. The ethyl acetate phase was dried over Na₂SO₄ and the solvent was removed. The product crystallized.
C₂₅H₃₂FN₅0S
Yield: 1.0 g (99%)
Purity: (HPLC 4.78 min.) 98%
Melting point 93° C
LC-MS: m/z 470 (M+1)⁺;
IR (λ[cm⁻¹]): 3383 (NH), 2968 und 2875 und 2793, 1604, 1541, 1490, 1471, 1452, 1108 (4-FPh), 1117, 963, 845
¹H-NMR (DMSO-d6) δ (ppm) 1.00 (t, J= 6.8 Hz, 3H, CH₃); 1.48-1.56 (m, 1-H, C3-H Pyrrolidine); 1.58-1.66 (m, 2-H, C4-H Pyrrolidine);1.75-1.84 (m, 1-H, C3-H, Pyrrolidine); 2.05-2.11 (m, 1-H, C5-H, Pyrrolidine); 2.13-2.22 (m, 1-H, N-**CH₂-**CH₃);2.49-2.55 (m, 1-H, C2-H, Pyrrolidine-under DMSO Peak); 2.63(s, 3-H, SCH₃); 2.75-2.83 (m, 1-H, N-**CH₂**-CH₃); 3.00-3.06 (m, 2-H, C5-H,Pyrrolidine, NH-**CH₂-**Pyrrolidine) 3.073.12 (m, 3-H, OCH₃); 3.39-3.47 (m, 3-H, N-CH₂-**CH₂**-OCH₃, NH-**CH₂**-Pyrrolidine); 3.96 (t,J= 6.0 Hz, 2-H, N-**CH₂**-CH₂-OCH₃); 6.46-6.51 (m, 3-H,C5-H +C3-H; Pyr,+ N-H); 7.06-7.12 (m, 2-H, C3/5-H, 4F-Ph); 7.40-7.45 (m, 2-H, C2/6-H, 4F-Ph); 8.06-8.07 (d, 1-H, J=5.61 Hz, C6-H, Pyr)
¹³C-NMR (DMSO-d6): δ (ppm) 14.53 (1-C, CH₃); 16.47 (1-C S-CH₃); 23.07 (1C, 4C, Pyrrolidine); 29.41 (1-C, C3, Pyrrolidine); 44.40 (1-C, N-**CH₂** CH₂-OCH₃); 45.13 (1C, NH-**CH₂**-Pyrrolidine);48.83 (1-,C, N-**CH₂**-CH₃); 53.92 (C5, Pyrrolidine); 58.79 (1-C, OCH₃); 63.13 (1-C, C2,Pyrrolidine); 70.60 (1-C N-CH₂-**CH₂**-OCH₃, 109.98 (1-C, C3, Pyr); 113.35 (1-C, C5, Pyr); 115.72 (d, 2-C,²J(C,F)=21.23 Hz, 4-FPh); 128.65 (d, 2-C ³J(C,F)=7.75 Hz, 4-FPh), 129.20; 131.27 (d, ⁴J=3.2 Hz, C1 4-4-FPh); 137.03; 139.35 ;143.99 ; 149.32 (1-C, C6 Pyr)160.28; 161.6 (d, 1-C, ¹J(C,F)=242.93 Hz)

### Example 3

### 4-[5-(4-Fluoro-phenyl)-3-methyl-2-methylsulfanyl-3H-imidazol-4-yl]-pyridin-2-yl)-(2,2,6,6-tetramethyl-piperidin-4-yl)-amine

The title substance was prepared from 0.952 g (0.003 mole) 2-fluoro-4-[5-(4-fluorophenyl)-3-methyl-2-methylsulfanyl-3H-imidazol-4-yl]-pyridine und 2.34 g (0.0143 mole) 2,2,6,6-tetramethyl-piperidin-4-ylamine under argon atmosphere at 170 °C. Reaction time: 5 h. The mixture was dissolved in ethyl acetate (20 mL) and extracted with water (9 x 10 mL each). The aqueous phases were extracted with ethyl acetate (10 mL). The ethyl acetate layers were combined and dried over Na₂SO₄. After removal of the solvent *in vacuum* the product foams and solidifies.
C₂₅H₃₂FN₅S
Yield: 1,16 g (90%)
Purity (HPLC RT=3.4 min.) 97%
Melting point: 75 °C (glass temp).
GC-MS: m/z (rel. Int. [%]) 453 (22), 315 (45), 124 (100).
IR (λ[cm⁻¹]): 3292, 2957, 2927, 1603, 1500, 1364, 1219 (4-FPh), 838, 811,
¹H-NMR (DMSO-d6): δ (ppm) 0.91-1.04 (m, 2-H, C3/5-H Piperidine); 1.00 (s, 6-H, 2CH₃ Piperidine); 1.09 (s, 6-H, 2CH₃ Piperidine); 1.69-1.73 (m, 2-H, C3/5-H Piperidine); 2.63 (s, 1-H, SCH₃); 3.4 (s, 3-H, NCH₃); 4.00-4.04 (m, 1-H, C4-H Piperidine); 6.31 (s, 1-H, C3-Pyr); 6.4 (d, 1-H, J= 7.6 Hz, NH); 6.47 (d, 1-H, J= 6.4 Hz, C5-H Pyr); 7.09-7.13 (m, C3/5-H 4F-Ph); 7.42-7.45 (m, 1-H, C2/6-H 4F-Ph); 8.08 (d, 1-H, J= 5.2 Hz, C6-HPyr)
¹³C-NMR (DMSO-d6): δ (ppm) 15.22 (1-C, SCH₃); 28.53 (2-C, 2CH₃ Piperidine); 31.38 (1-C, NCH₃); 34.5 (2-C, 2CH₃ Piperidine); 42.91 (1-C, C4-H Pip.); 50.36 (1-C, C3/C5-H Piperidine); C3 Pyr not visible; 111.98 (1-C, C5-H, Pyr); 114.97 (d, 2-C, ²J(C,F)=21.53 Hz, 4-FPh); 128.11 (d, 2-C ³J(C,F)=7.80 Hz, 4-FPh); 128.74; 130.59 (1-C, ⁴J= 3.02 Hz, C1-H 4-FPh).; 136.39; 138.3; 143.09; 148.72 (1-C, C6, Pyr); 158.63; 160.85 (d, 1-C, ¹J(C,F)=243.12 Hz)

### Example 4

### {4-[5-(4-Fluoro-phenyl)-3-(2-methoxy-ethyl)-2-methylsulfanyl-3H-imidazol-4-yl]-pyridin-2-yl}-(2,2,6,6-tetramethyl-piperidin-4-yl)-amine

0.903 g (0.0025 mole) 2-fluoro-4-[5-(4-fluoro-phenyl)-3-(2-methoxy-ethyl)-2-methylsulfanyl-3H-imidazol-4-yl]-pyridine and 1.95 g (0.0119 mole) 3,3,5,5-tetramethyl-piperidin-4-ylamine were reacted under argon atmosphere for 10.5 h at 140-190° C. The reaction mixture was dissolved in ethyl acetate (30 mL). The organic phase was extracted with water 10 times (15 mL each). The aqueous phases were combined and extracted with ethyl acetate (20 mL). The ethyl acetate layers were combined, dried over Na₂SO₄ and the solvent was removed. The residue crystallized when triturated with n-hexane.
C₂₇H₃₆FN₅OS;
Yield: 1.0 g (85%);
Purity (HPLC, RT= 4.07 min.) 96%;
Melting point: 153 °C.
GC-MS: 18.21 min; m/z (%) 497 (24), 439 (12), 359 (48), 281 (9), 124 (100)
IR (λ[cm⁻¹]): 3347 (-NH-), 2928, 2963, 1601, 1547, 1501, 1451, 1366, 1217 (4-FPh), 1103, 847, 811
¹H-NMR (DMSO-d6): δ (ppm) 0.92- 0.98 (t, 2-H, J= 12 Hz, C3/5-H Piperidine); 1.01 (s, 6-H, 2CH₃ Piperidine); 1.11 (s, 6-H, 2CH₃ Piperidine); 1.72-1.75 (m, 2-H, C3/5-H Piperidine); 2.63 (s, 3-H, SCH₃); 3.13 (s, 3-H, OCH₃); 3.41 (t, 2-H, ³J= 5,6 Hz, N-CH₂-**CH₂**-OCH₃); 3.97 (t, 2-H, ³J=5,6 Hz, N-**CH₂**-CH₂-OCH₃); .02-4.06 (1-H, C4-H-Piperidine); 6.33 (s, 1-H, C3-H Pyr); 6.41 (d, 1-H, J= 7,6 Hz, NH); 6,49 (d, 1-H, ³J= 4,8 Hz, C5-H Pyr); 7.08-7.71 (m, 1-H, C3/5-H 4F-Ph); 7.41-7.44 (m, 1-H, C2/6-H 4F-Ph); 8.08 (d, 1-H, ³J= 4,8 Hz, C6-H Pyr).
¹³C-NMR (DMSO-d6): δ (ppm) 16.21 (1-C S-CH₃) 29.08 (2-C, 2CH₃ Piperidine); 35.00 (2-C, 2CH₃ Piperidine); 43.38 (1-C, N-**CH₂**-CH₂-OCH₃); 44.16 (1-C, C3 Piperidine); 45.06 (1-C, C5 Piperidine); 50.99 (1-C, C4 Piperidine); 58.58 (1-C, OCH₃); 70.35 (1-C, N-CH₂-**CH₂**-OCH₃); C3 von Pyr not visible; 112.89 (1-C, C5, Pyr); 115.54 (d, 2-C, ²J(C,F)=20.73 Hz, 4-FPh); 128.50 (d, 2-C ³J(C,F)=7.75 Hz, 4-FPh); 128.99.; 131.03; 136.88; 139.11; 143.83; 149.34 (1-C, C6, Pyr); 159.22; 161.37 (d, 1-C, ¹J(C,F)=242 Hz)

### Example 5

### (1-Benzyl-piperidin-4-yl)-{4-[5-(4-fluoro-phenyl)-3-methyl-2-methylsulfanyl-3H-imidazol-4-yl]-pyridin-2-yl}-amine

0.5078 g (0.0016 mole) 2-fluoro-4-[5-(4-fluoro-phenyl)-3-methyl-2-methylsulfanyl-3H-imidazol-4-yl]-pyridine and 0.63 g (0.0032 mole) 1-benzyl-piperidin-4-ylamine were reacted under argon atmosphere for 11.5 h at 130 -160° C. The reaction mixture was dissolved in ethyl acetate (150 mL). The organic layer was washed with a saturated aqueous solution of sodium chloride for 10 times (10 mL each). The water phases were combined and extracted with ethyl acetate (10 mL). The ethyl acetate phases were dried over Na₂SO₄ and the solvent was removed. The product crystallized.
C₂₈H₃₀FN₅S
Yield: 670 mg (87%);
Purity (HPLC 3.4 min) 96 %;
Melting point: 167° C
HPLC-MS: m/z (M+) 488
IR (λ[cm⁻¹]): :
3290 (NH), 2928, 2801, 2795, 1603, 1500, 1450, 1366, 1218 (4-FPh), 1091, 838, 735,697
¹H-NMR (DMSO-d6)
δ (ppm) 1.37-1.46 (m, 2-H, C3/5-H Piperidine,); 1.85-1.92 (m, 2H, C3/5-H Piperidine); 2.01-2.06 (m, 2-H, C2/6-H Piperidine,); 2.63 (s, 3-H, SCH₃); 2.69-2.78 ( m, 2-H, C2/6-H Piperidine); 3.38 (s, 3H, NCH₃); 3.45 (s, 2-H, CH₂ Benzyl); 3.65-3.70 (s, 1-H, C4- H Piperidine); 6.39 (s,1-H, C3-H Pyr); 6.43 (d 1-H, J= 5.6 Hz, C5-H Pyr) ; 6.56 (d, 1-H, J= 7.6 Hz, NH) ; 7.08-7.15 (m, 2H, 4-FPh C3/5-H, 4-FPh); 7.22-7.34 (m, 5H, C1-C5 Benzyl); 7.42-7.46 (m, 3H, F-Ph C2/6, 4-FPh); 8.05 (d ,1H,J=5.2 Hz, C6-H Pyr)
¹³C-NMR (DMSO-d6)
δ (ppm) 15.79 (1-C, SCH₃); 31.92 (1-C, NCH₃); 32.73 (2-C, C3/5 Piperidine); 47.95 (1-C, C4 Piperidine); 52.46 (2-C, C2/6 Piperidine); 62.69 (1-C, CH₂-Benzyl); 09.55 (1-C, C3, Pyr); 112.73 (1-C, C5 Pyr); 115.52 (d, 2-C, ²J(C, F=21.43 Hz); 127.26 (1-C, Benzyl) 128.28; 128.54-128.61 (m, 3-C, ⁴J= 3.0 Hz, Benzyl+ ³J(C,F)=7.35 Hz, 4-FPh ); 129.16 (2-C, J=3.1 Hz, Benzyl); 131.16 (1-C, ⁴J (C,F) = 3.2 Hz, C1 4-FPh); 136.85; 138.87; 139.14; 143.57; 149.03 (1-C, C6 Pyr) 159.17; 161.4. (¹J(C,F)=243.73 Hz)

### Example 6

### (1-Benzyl-piperidin-4-yl)-{4-[5-(4-fluoro-phenyl)-3-(2-methoxy-ethyl)-2-methylsulfanyl-3H-imidazol-4-yl]-pyridin-2-yl}-amine

0.9 g (0.0025 mole) 2-fluoro-4-[5-(4-fluoro-phenyl)-3-(2-methoxy-ethyl)-2-methylsulfanyl-3H-imidazol-4-yl]-pyridine und 2.4 g (0.0124 mole) 1-benzyl-piperidin-4-ylamine were reacted for 7.5 h at 130 -145° C under an argon atmosphere. The resulting mixture was dissolved with at least 70 mL of ethyl acetate. The ethyl acetate layer was extracted with water for 9 times (25 mL each). The water phases were combined and extracted with ethyl acetate (10 mL). The ethyl acetate phases were combined, dried over Na₂SO₄ and the solvent was evaporated. The oily product was purified by column chromatography and crystallized from ethanol/ether.
C₃₀H₃₄FN₅OS
Yield: 810 mg (62%);
Purity (HPLC, 4.17 min): 99%;
Melting point: 59° C
HPLC-MS:
m/z (M+1) 532
IR (λ[cm⁻¹]): 3311 (NH), 2928, 2807, 2760, 1603, 1515, 1450, 1219 (4-FPh), 1118, 839,735,698
¹H-NMR (DMSO-d6)
δ (ppm) 1.37-1.45 (m, 2-H, C3/5-H Piperidine); 1.86-1.89 (m, 2H, C3/5-H Piperidine); 2.03 (m, 2-H, C2/6-H Piperidine); 2.63 (s, 3-H, SCH₃); 2.75-2.78 (m, 2-H, C2/6-H Piperidine); 3.12 (s, 3H, OCH₃); 3.40 (t, 2H, J=5.6 Hz, N-CH₂-**CH₂**-OCH₃); 3.45 (s, 2-H, CH₂ Benzyl); 3.95 (s, 1-H, C4-H); 3.95 (t, 2H, J= 5.2 Hz, N-**CH₂**-CH₂OCH₃); 6.4 (s, 1-H, C3-H Pyr); 6.45 (d 1-H, J= 4.4 Hz, C5-H Pyr) ; 6.58 (d, 1-H, J= 7.2 Hz, NH) ; 7.07-7.12 (m, 2-H, C3/5-H 4-FPh); 7.24-7.34 (m, 5-H, C1-C5 4-FPh); 7.40-7.44 (m, 3H, 4-FPh C2/6); 8.06 (d , 1-H, J=4.8 Hz, C6-H Pyr)
¹³C-NMR (DMSO-d6)
δ (ppm) 15.67 (1-C, SCH₃); 31.07 (2-C, C3/5 Piperidine); 43.60 (1-C, N-**CH₂**-CH₂-OCH₃); 47.45 (1-C, C-4 H Piperidine); 52.00 (2-C, C2/6 Piperidine); 58.02 (1-C, OCH₃); 62.13 (1-C, CH₂ Benzyl); 69.81 (1C, N-CH₂-**CH₂**-OCH₃); 109.39 (1-C, C3-H Pyr); 112.59 (C5, C5 Pyr); 114.84 (d, 2-C, ²J(C,F)= 21.53 Hz, 4-FPh); 126.72 (1-C, C4-Benzyl); 127.85 (2-C ³J(C,F)=7.949, 4-FPh); 128.03 (2-C, Benzyl); 128.4; 128.62 (2C, Benzyl); 130.49 (1-C, ⁴J= 3.02 Hz, C1 4-FPh); 136.23; 138.60; 143.18 ; 148.55 (1-C, C6-H Pyr); 158.65; 160.82. (¹J(C,F)=243.73 Hz)

### Example 7

### (1-Ethyl-pyrrolidin-2-ylmethyl)-{4-[5-(4-fluoro-phenyl)-2-methanesulfinyl-3-methyl-3H-imidazol-4-yl]-pyridin-2-yl}-amine

0.698 g (0.0016 mole) (1-ethyl-pyrrolidin-2-ylmethyl)-{4-[5-(4-fluoro-phenyl)-3-methyl-2-methylsulfanyl-3H-imidazol-4-yl]-pyridin-2-yl}-amine were dissolved in 9 mL acetic acid (100%) and treated with a solution of 0.25 g (0.0022 mole) hydrogen peroxide 30% in 1 mL acetic acid. Reaction time was 132 h (5.5 d).
The mixture was poured into ice water (10 mL) and ammonia (32%) was added until pH 8-9 was reached. The resulting precipitate was dissolved in ethyl acetate (20 mL) and the phases were separated. The aqueous phase was extracted 5 times with ethyl acetate (10 mL each). The ethyl acetate phases were combined, dried over Na₂SO₄ and the solvent was removed. The product was purified by column chromatography : Al₂O₃ / ethyl acetate: dichloromethane: triethylamine = 11:8:1
*C₂₃H₂₈FN₅OS* (Mr 441,57):
Yield: 0.5 g (70%)
Purity (HPLC RT=3.16 min.): 98%
The product is glass-like.
IR (λ[cm⁻¹]): 3344 (NH), 2967, 2873, 2795, 1735, 1606, 1502, 1372, 1236(4-FPh), 1044 (SO), 840

### Example 8

### 1-Ethyl-pyrrolidin-2-ylmethyl)-{4-[5-(4-fluoro-phenyl)-2-methanesulfinyl-3-(2-methoxy-ethyl)-3H-imidazol-4-yl]-pyridin-2-yl}-amine

0.0516 g (0.0011 mole) (1-ethyl-pyrrolidin-2-ylmethyl)-{4-[5-(4-fluoro-phenyl)-3-(2-methoxy-ethyl)-2-methylsulfanyl-3H-imidazol-4-yl]-pyridin-2-yl}-amine were dissolved in 10 mL acetic acid (100%) and treated with a solution of 0.13 g (0.0012 mole) hydrogen peroxide 30% in 2 mL acetic acid. Reaction time: 72 h.
The mixture was poured into ice water (7 mL) and ammonia (32%) was added until pH 8-9 was reached. The aqueous phase was extracted 5 times with ethyl acetate (17 mL each). The ethyl acetate phases were combined, dried over Na₂SO₄ and the solvent was removed. The product was purified by column chromatography :SiO2 /toluene:THF = 1:1. The product crystallized.
C₂₅H₃₂FN₅O₂S *(Mr 485,63):*
Yield after purification: 83 mg (16%)
Purity (HPLC RT=3,38 min). 97%;
mp: 75° C.
IR (λ[cm⁻¹]): 3375 (-NH-), 2964, 2872, 2796, 1605, 1517, 1498, 1221 (4-FPh F), 1118, 1054 (SO), 841, 813, 605
¹H-NMR (DMSO-d6): δ (ppm) 0.99 (t, J= 7.6 Hz, 3-H, CH₃); 1.49-1.53 (m, 1-H, C3-H, Pyrrolidine); 1.61 (m, 2-H, C4-H, Pyrrolidine); 1.77-1.79 (m, 1-H, C3-H, Pyrrolidine); 2.07-2.09 (m, 1-H, C5-H, Pyrrolidine); 2.16 (m, 1-H, N-**CH₂**-CH₃); 2.48-2.53 (m, 1-H, C2-H, Pyrrolidine) 2.77-2.81 (m, 1-H, N-CH₂-CH₃.); 3.06-3.09 (m, 2-H, NH-CH₂-Pyrrolidine, C5-H Pyrrolidine) 3.02-3.12 (m, 3-H, SOCH₃); 3.11 ( s, 3-H, OCH₃); 3.42-3.492 (m, 3-H, 2-H, N-CH₂-**CH₂**-OCH₃, 1-H NH-**CH₂-**Pyrrolidine) 4.20-4.30 (m, 2-H, N-**CH₂**-CH₂-OCH₃); 6.50-6.14 (m, 3-H, C3/C5-H Pyr,+ NH); 7.10-7.14 (m, 2-H, C3/5-H, 4-FPh); 7.46-7.48 (m, 2-H, C2/6-H 4-FPh); 8.093 (m, J= 4.4 Hz, 1-H, C6-H Pyr)
¹³C-NMR (DMSO-*d6*): δ (ppm) 14.70 (1-C, CH₃); 23.3 (1-C, C4 Pyrrolidine); 29.61 (1-C, C3-Pyrrolidine); 39.60 (1-C, SOCH₃); 44.92 (1-C, N-**CH₂**-CH₂-OCH₃); 45.28 (1-C, NH-CH₂-Pyrrolidine); 49.09 (1-C, N-**CH₂**-CH₃); 54.14 (1-C, C5-Pyrrolidine); 59.04 (1-C, OCH₃); 63.41 (1-C, C2- Pyrrolidine); 71.45 (1-C, N-CH₂-**CH₂**-OCH₃); 110.31 (1-C, Pyr-C3); 116.18 (d, ²J= 21.93 Hz, 4-FPh); 129.247 (d, 2-C, ³J(C,F)=8,35 Hz, 4-FPh); 130.72 (d, 1-C, ⁴J= 2.52 Hz, C1 4-FPh); 130.91; 137.63; 138.51; 140.09; 149.06; 149.81 (1-C, C6, Pyr); 160.51; 162.23 (d, 1-C, ¹J= 244.43 Hz)

### Example 9

### {4-[5-(4-Fluoro-phenyl)-2-methanesulfinyl-3-methyl-3H-imidazol-4-yl]-pyridin-2-yl}-(2,2,6,6-tetramethyl-piperidin-4-yl)-amine

0.4536 g (0.001 mole) {4-[5-(4-fluoro-phenyl)-3-methyl-2-methylsulfanyl-3H-imidazol-4-yl]-pyridin-2-yl}-(2,2,6,6-tetramethyl-piperidin-4-yl)-amine were dissolved in 10 mL acetic acid (100%) and treated with a solution of 0.4 g (0.0029 mole) hydrogen peroxide 30% in 2 mL acetic acid. Reaction time: 72 h. The mixture was poured into ice water (12 mL) and ammonia (32%) was added until pH 8-9 was reached. The aqueous phase was extracted 3 times with ethyl acetate (10 mL each). The ethyl acetate phases were combined, washed with water (10 mL), dried over Na₂SO₄ and the solvent was removed. The product crystallized from n-hexane.
C₂₅H₃₂FN₅OS *(Mr 469, 63*)
Yield: 428 mg (93%); Purity (HPLC: 2.6 min): 94%;
Mp.: 178° C
HPLC-MS: m/z (M+) 470
IR (λ[cm⁻¹]): 3312 (NH), 2958, 2926, 1732, 1605, 1501, 1370, 1221 (4-FPh), 1042 (SO), 840, 655, 588
¹H-NMR (CDCl₃): δ (ppm) 0.91-0.97 (t, 2-H, J=10.0 Hz, C3/5-H Piperidine); 1.10 (d, J= 2.4 Hz, 6-H, 2CH₃ Piperidine); 1.14 (d, J= 4.0 Hz 6-H, 2CH₃ Piperidine); 1.84-1.89 (m, 2-H, C3/5-H Piperidine); 3.25 (s, 3-H, SOCH₃); 3.79-3.81(m, 1-H, C4-H Piperidine); 3.85 (s, 3-H, NCH₃); 4.51(d, 1-H, J= 8.0 Hz, NH.); 6.20 (s, 1-H, C3-H Pyr); 6.54 (t, 1-H, J= 5.2 Hz, C5-H Pyr); 6.95-6.99 (m, 1-H, C3/5-H 4-FPh); 7.45-7.49 (m, 1-H, C2/6-H 4-FPh); 8.21 (d, 1-H, J= 5.2 Hz, C6-H Pyr)
¹³C-NMR (CDCl₃): δ (ppm) 28.40 (2-C, 2CH₃Piperidine); 32.18 (1-C, NCH₃); 34.77 (2-C, 2CH₃Piperidine); 38.11 (1-C, SOCH₃); 44.40 (1-C, C-H Pip.); 45.42 (1-C, C3-H/C5-H Piperidine); 107.52 (1-C, C3 Pyr); 113.38 (1-C, C5, Pyr); 115.38 (d, 2-C, ²J(C,F)=22.04 Hz); 129.15 (d, 2-C ³J(C,F)=8.15 Hz, 4-FPh); 129.33 (1-C, ⁴J= 2.9 Hz, C1 4-FPh). 131.25; 138.48; 138.56; 146.48; 149.76 (1-C, C6, Pyr); 158.48; 162.22 (d, 1-C, ¹J(C,F)=246.84 Hz)

### Example 10

### ({4-[5-(4-Fluoro-phenyl)-2-methanesulfinyl-3-(2-methoxy-ethyl)-3H-imidazol-4-yl]-pyridin-2-yl}-(2,2,6,6-tetramethyl-piperidin-4-yl)-amine

0.0957 g (0.00018 mole) {4-[5-(4-fluoro-phenyl)-3-(2-methoxy-ethyl)-2-methylsulfanyl-3H-imidazol-4-yl]-pyridin-2-yl}-(3,3,5,5-tetramethyl-piperidin-4-yl)-amine were dissolved in 10 mL acetic acid (100%) and treated with a solution of 0.08 g (0.0002 mole) hydrogen peroxide 30% in 2 mL acetic acid at room temperature. Reaction time: 26 h. The mixture was poured into ice water (20 mL) and ammonia (32%) was added until pH 8-9 was reached. The aqueous phase was extracted 6 times with ethyl acetate (10 mL each). The ethyl acetate phases were combined, dried over Na₂SO₄ and the solvent was removed. The product was glass-like.
C₂₇H₃₆FN₅O₂S (Mr 513,68)
Yield: 60 mg (67%):
Purity (HPLC: 3,24 min); 96%;
Mp.: 65 °C
GC-MS: 22,79 min; m/z (%) 513 (2), 497 (14), 439 (7), 359 (35), 124 (100)
IR (λ[cm⁻¹]): 3308 (NH), 2958, 2927, 1605, 1545, 1501, 1365, 1220 (4-FPh), 1044 (SO), 839, 812
¹ H-NMR (CDCl₃): δ (ppm) 1.00 (m, 2-H, C3/5-H Piperidine); 1.15 (s, 6-H, CH₃ Piperidine); 1.19 (s, 6-H, CH₃ Piperidine); 1.89-1.89 (m, 2-H, C3/5-H Piperidine); 3.23 (s, 3-H, SOCH₃); 3.28 (s, 3-H, OCH₃); 3.50-3.61 (m, 2-H, N-CH₂-**CH₂**-OCH₃); 4.24-4.31 (m, 1-H, N-**CH₂**-CH₂-OCH₃); 4.49-4.55 (m, 1-H, N-**CH₂**-CH₂-OCH₃+ NH); 6.24 (s, 1-H, C3-H Pyr); 6.6 (d, 1-H, J= 4.8 Hz, C5-H Pyr); 6.94-6.98 (m, 1-H, C3/5-H 4-FPh); 75-7.50 (m, 1-H, C2/6-H 4-FPh); 8.21 (d, 1-H, J= 5.2 Hz, C6-H Pyr) C4-H not visible

### Example 11

### (1-Benzyl-piperidin-4-yl)-{4-[5-(4-fluoro-phenyl)-2-methanesulfinyl-3-methyl-3H-imidazol-4-yl]-pyridin-2-yl}-amine

0.75 g (0.00225 mol) 2-fluoro-4-[5-(4-fluoro-phenyl)-2-methanesulfinyl-3-methyl-3H-imidazol-4-yl]-pyridine were dissolved in 25 mL glacial acetic acid and treated with a solution of 0.89 g (0.0045 mole) hydrogen peroxide 30% in 2 mL acetic acid. Reaction time: 4.5 h. The mixture was dissolved in ethyl acetate (25 mL). The organic phase was washed 8 times with a saturized solution of sodium chloride in water (10 mL each). The aqueous phases were combined and extracted with ethyl acetate (20 mL). The ethyl acetate phases were combined, dried over Na₂SO₄ and the solvent was removed. The residue was purified by column chromatography: Al₂O₃/ ethyl acetate: n-hexane = 2:1 and recrystallized from ethanol.
C₂₈H₃₀FN₅OS *(Mr 503,65)*
Yield: 450 mg (47%)
Purity (HPLC 3,04 min) : 98%
Mp.: carbonizes at 180-220° C
LC-MS m/z (M+1) 504
IR (λ[cm⁻¹]): 3307, 2942, 2804, 1603, 1546, 1496, 1442, 1217 (FPh), 1040 (SO), 832,736,694
¹H-NMR (DMSO-d6)
δ (ppm) 1.38-1.46 (m, 2-H, C3/5-H Piperidine,); 1.86-1.89 (m 2H, C3/5-H Piperidine); 2.04 (m, 2-H, C2/6-H Piperidine); 2.75-2.78 (m, 2-H, C2/6-H Piperidine,); 3.14 (s, 3-H, SOCH₃); 3.46 (s, 2-H, CH₂ Benzyl); 3.46 (s, 4-H, 3-H SOCH₃+ 1-H, C4-H Piperidine); 6.43 (s, 1-H, C3-H Pyr); 6.48 (d, 1-H, J= 5.2 Hz, C5-H Pyr); 6.46 (d, 1-H, J= 7.6 Hz, NH); 7.12-7.18 (m, 2-H, C3/5-H 4-FPh); 7.24-7.34 (m, 5-H, C2-C5 4-FPh); 7.45-7.48 (m, 2-H, C2/6 4-FPh); 8.1 (d , 1-H, J=4.8 Hz, C6-H Pyr)

### Example 12

### (1-Benzyl-piperidin-4-yl)-{4-[5-(4-fluoro-phenyl)-2-methanesulfinyl-3-(2-methoxy-ethyl)-3H-imidazol-4-yl]-pyridin-2-yl}-amine

0.2 g (0.00135 mole) 2-fluoro-4-[5-(4-fluoro-phenyl)-2-methanesulfinyl-3-(2-methoxyethyl)-3H-imidazol-4-yl]-pyridine and 0.94 g (0.0047 mole) 1-benzyl-piperidin-4-ylamin were reacted under an argon atmosphere for 6 h at 145° C. The mixture was dissolved in ethyl acetate (30 mL). The remaining amine was removed by washing 8 times with a saturated aqueous sodium chloride solution (15 mL each). The aqueous phases were combined and washed with ethyl acetate (15 mL), the ethyl acetate phase was dried over Na₂SO₄. After filtering and removing the solvent the oily product was purified by column chromatography: Al₂O₃ / ethyl acetate: n-hexane = 2:1
C₃₀H₃₄FN₅0₂S *(Mr 547, 7):*
Yield: 340 mg (46%);
Purity (HPLC: RT=3,66 min.) 99%,
mp.: 54°C,
HPLC-MS: m/z (M+) 548
IR (λ[cm⁻¹]): 3312 (NH), 2925, 2809,1604, 1520, 1500, 1366, 1220 (4-FPh), 1047,839,740,698
¹H-NMR (DMSO-d6): δ (ppm) 1.38-1.46 (m, 2-H, C3/5-H Piperidine,); 1.86-1.89 (m, 2-H, C3/5-H Piperidine); 2.4 (m, 2-H, C2/6-H Piperidine); 2.76-2.79 (m, 2-H, C2/6-H Piperidine,); 3.11 (s, 3H, SOCH₃); 3.13 (s, 3-H, OCH₃); 3.41-3.51 (m, 2H, N-CH₂-**CH₂**-OCH₃); 3.46 (s, 2-H, CH₂ Benzyl); 3.68 (s, 1 H, C4-H Piperidine); 4.16-4.31 (m, 2-H, N-**CH₂**-CH₂-OCH₃), 6.44 (s, 1-H, C3-H Pyr); 6.49 (d 1 H, J= 5.2 Hz, C5-H Pyr); 6.65 (d, 1 H, J= 7.2 Hz, NH) ; 7.13-7.17 (m, 2-H, C3/5-H 4-FPh); 7.24-7.34 (m, 5H, C2-C5 Benzyl); 7.45-7.48 (m, 3H, C2/6 4-FPh); 8.13 (d ,1-H, J=4.8 Hz, C6-H Pyr)
¹³C-NMR (DMSO-d6): δ (ppm) 31.67 (2-C, C3/5 Piperidine); 38.57 (1C, SOCH₃); 43.89 (1-C, N-**CH₂**-CH₂-OCH₃); 47.48 (1-C, C4 Piperidine); 51.99 (2-C, C2/6 Piperidine) 58.03 (1-C, OCH₃); 62.12 (1-C, CH₂ Benzyl); 70.44 (1C, N-CH₂-**CH₂-**OCH₃); 109.43 (1-C, C3, Pyr); 112.38 (1C, C5 Pyr); 115.21 (d, 2-C, ²J(C,F)= 21.53 Hz, 4-FPh); 126.71 (1-C, C4 Benzyl); 128.03 (2-C, Benzyl); 128.54 (2-C ³J(C,F)=7.95 Hz, 4-FPh), 128.61 (2-C, Benzyl); 129.71 (1-C, ⁴J = 3.02 Hz, C1 4-FPh); 129.85; 136.58, 137.52, 138.57; 148.06; 148.82 (1-C, C6, Pyr) 158.63; 161.2. (d, ¹J(C,F)=244.33 Hz)

### Example 13

### 1-{4-[5-(4-Fluoro-phenyl)-3-methyl-2-methylsulfanyl-3H-imidazol-4-yl]-pyridin-2-ylamino}-propan-2-ol

1.43 g (0.0045 mole) 2-fluoro-4-[5-(4-fluoro-phenyl)-3-methyl-2-methylsulfanyl-3H-imidazol-4-yl]-pyridine and 3.41 g (0.0446 mole) 1-amino-2-propanole were reacted for 3.5 h at 160° C. The residue was dissolved in ethyl acetate (30 mL) and washed with water (6x10 mL).
The aqueous phases were combined and extracted with ethyl acetate (10 mL), the ethyl acetate phase was dried over Na₂SO₄. After filtering and removing the solvent the product began to foam. The obtained crystals were hygroscopic and rapidly changed into a glass-like state.
C₁₉H₂₁FN₄OS *(Mr 372,47)* :
Yield: 1,26 g (76%);
Purity (HPLC RT=3,6 min.) 98%;
GC- MS: 11,61 min; m/z (%) 372 (40), 327 (100), 313 (22), 295 (20), 279 (19)
IR (λ[cm⁻¹]): 3318 (-OH), 2967, 2926, 1732, 1607, 1502, 1218(4-FPh), 838, 811 ¹H-NMR (DMSO-d6): δ (ppm) 1.07 (d, 3-H, J= 6.4 Hz, CH₃); 2.63 (s, 3-H, SCH₃); 3.12-3.26 (m, 2-H, NH-**CH₂**-CH-(OH)CH₃); 3.32; 3.38 (s, 3-H, NCH₃); 3.74-3.80 (m, 1-H, NH-CH₂-**CH**-(OH)CH₃) 4.74 (d, 1-H, J= 4.4 Hz, OH); 6.43 (d, 1-H, J= 5.2 C5-H Pyr); 6.48 (1-H, C3-H Pyr); 6.18 (t, 1-H, J=5.6 Hz, NH) 7.08-7.13 (m, 1-H, C3/5-H 4-FPh); 7.43-7.47 (m, 1-H, C2/6-H 4-FPh); 8.04 (d, 1-H, J= 5.2 Hz, C6-H Pyr).
¹³C-NMR (DMSO-d6): δ (ppm) 15.80 (1-C, S-CH₃) 21.81 (1-C, C(OH)-**CH₃**); 31.90 (1-C, NCH₃); 49.10 (1-C, NH-**CH₂**-CH-(OH)CH₃); 65.62 (1-C, NH-CH₂-**CH**-(OH)CH₃) ; 109.52 (1-C, C3-Pyr); 112.87 (1-C, C5, Pyr); 115.54 (d, 2-C, ²J(C,F)=21.53 Hz 4-Ph); 128.50 (d, 2-C ³J(C,F)=7.75 Hz 4-FPh); 129.16.; 131.11; 136.77; 138.90; 143.53; 148.53 (1-C, C6, Pyr); 159.93; 161.38 (d, 1-C, ¹J(C,F)=242.92 Hz)

### Example 14

### 1-{4-[5-(4-Fluoro-phenyl)-3-(2-methoxy-ethyl)-2-methylsulfanyl-3H-imidazol-4-yl]-pyridin-2-ylamino}-propan-2-ol

1.01 g (0.0028 mol) 2-fluoro-4-[5-(4-fluoro-phenyl)-3-(2-methoxy-ethyl)-2-methylsulfanyl-3H-imidazol-4-yl]-pyridine were reacted with 2.12 g (0.0277 mole) 1-amino-2-propanol at 160° C for 3 h. The residue was dissolved in ethyl acetate (40 mL) and washed with water (6 x 10 mL). The aqueous phases were combined and extracted with ethyl acetate (10 mL). The ethyl acetate phases were combined, dried over Na₂SO₄ and filtered. The product crystallized from n-hexane. The obtained crystals were hygroscopic and rapidly changed into a glass-like state.
C₂₁ H₂₅FN₄0₂S *(Mr 416,52):*
Yield: 1.04 g (90%);
Purity (HPLC RT=4.3 min.) 99%:
GC-MS: (RT=12.92 min); m/z (%) 416 (55), 371 (100), 357 (26), 313 (23), 298 (12), 279 (24)
IR (λ[cm⁻¹]): 3318 , 2967, 2928, 2892, 1607, 1503, 1219 (4-FPh), 118, 838, 812, ¹H-NMR (DMSO-d6): δ (ppm) 1.073 (d, 3-H, J= 6.0 Hz, CH₃); 2.63 (s, 1-H, SCH₃); 3.13 (s, 3-H, OCH₃); 3.10-3.27 (m, 2-H, NH-**CH₂**-CH (OH)-CH₃); 3.4 (t, 2-H, J= 5.6 Hz, N-CH₂-**CH₂**-OCH₃); 3.78 (s, 1-H, NH-CH₂-**CH**(OH)-CH₃) 3.97 (t, 2-H, J= 5.6 Hz, N-**CH₂**-CH₂-OCH₃); 4.75 (d, 1-H, J= 3.6 Hz, OH); 6.45 (d, 1-H, J= 5.6 Hz, C5-H Pyr); 6.51 (s, 1-H, C3-H Pyr); 6.34 (t, 1-H, J= 5.6 Hz, NH); 7.07-7.12 (m, 1-H, C3/5-H 4-FPh); 7.42-7.46 (m, 1-H, C2/6-H 4-FPh); 8.05 (d, 1-H, J= 5.2 Hz, C6-H Pyr)
¹³C-NMR (DMSO-d6): δ (ppm) 16.24 (1-C, SCH₃); 21.80 (1-C, -CH(OH)**CH₃**); 44.16 (1-C, N-**CH₂**-CH₂-OCH₃); 49.11 (1-C, NH-**CH₂**-CH (OH)-CH₃); 58.57 (1-C, OCH₃); 65.63 (1-C, NH-CH₂-**CH** (OH)-CH₃); 70.36 (1-C, N-CH₂-**CH₂**-OCH₃) ; 109.93 (1-C, C3-Pyr); 113.25 (1-C, C5, Pyr); 115.52 (d, 2-C, ²J(C,F)=20.73 Hz); 128.35 (d, 2-C ³J(C,F)=7.75 Hz 4-FPh); 128.90; 130.51 (d, 1-C,⁴J(C,F)=3.12 Hz, 4-FPh); 136.73, 139.18; 143.74; 148.92 (1-C, C6 Pyr); 159.95; 161.36 (d, 1-C, ¹J(C,F)= 242.92 Hz)

### Example 15

### 4-{4-[5-(4-Fluoro-phenyl)-3-methyl-2-methylsulfanyl-3H-imidazol-4-yl]-pyridin-2-ylamino}-cyclohexanol

1.1 g (0.0035 mole) 2-fluoro-4-[5-(4-fluoro-phenyl)-3-methyl-2-methylsulfanyl-3H-imidazol-4-yl]-pyridine and 1.22 g (0.0104 mole) trans-4-aminocyclohexanol were reacted at 110-130° C for 5.5 h. The residue was dissolved in ethyl acetate (40 mL) and washed with water (7 x 10 mL). The aqueous phases containing a precipitate were combined and extracted with ethyl acetate (10 mL), the ethyl acetate phase was dried over Na₂SO₄. After removal of the solvent the product began to foam to give voluminous crystals
C₂₂H₂₅FN₄0S *(Mr 412,53)*
Yield: 1.5 g
Purity: (HPLC 3.88 min) 98%
Mp: 108°C
GC-MS:
19.028 min; m/z (%) 412 (58), 365 (11), 353 (35), 339 (23), 313 (100), 299 (14), 281 (37)
IR (λ[cm⁻¹]): 3311 8-OH), 2930, 2856, 1731, 1605, 1501, 1449, 1371, 1218 (4-FPh),
1046,838,812,590
¹H-NMR (DMSO-d6)
δ (ppm) 1.12-1.28 (m, 4-H trans- 4-aminocyclohexanol); 1.8-1.92 (m, 4-H, trans- 4-aminocyclohexanol); 2.63 (s, 3H, SCH₃); 3.32 (s, 3-H, NCH₃); 3.38-3.45 (m, 1-H, C1-H trans-4-aminocyclohexanol); 3.55-3.57 (s, 1-H, C4-H trans-4-aminocyclohexanol); 4.51 (d, 1-H, J=4.4 Hz, OH); 6.35 (d, 1-H, C3-H Pyr); 6.42 (d, 1-H, J=5.2 Hz, C5-H Pyr) ; 6.45 (d, 1 H, J= 7.6 Hz, NH) ; 7.07-7.13 (m, 2-H, C3/5-H 4-FPh); 7.41-7.46 (m, 2-H, C2/6-H 4-FPh); 8.05 (d, 1-H, 5.6 Hz, C6-H Pyr)
¹³C-NMR (DMSO-d6)
δ (ppm) 15.24 (1C, S-CH₃); 30.35 (2-C, trans-4-aminocyclohexanol); 31.37 (1-C, NCH₃); 33.96 (2-C, trans-4-aminocyclohexanol); 48.60 (-1C, C4 trans-4-aminocyclohexanol); 68.35 (1-C, C1 trans-4-aminocyclohexanol); 108.89 (1-C, C3-Pyr); 112.02 (1-C, C5-Pyr); 114.97 (d, 2-C, ²J(C,F)=20.93 Hz, 4-FPh); 128.01 (d, -2C, ³J(C,F)=8.05 Hz, 4-FPh); 128.68; 130.62 (d, ⁴J (C,F)= 3.12 Hz, C1 4-FPh); 136.27; 138.28; 142.97; 148.49 (1-C, C6 Pyr); 158.72; 160.84 (1-C, ¹J(C,F)=243.12 Hz)

### Example 16

### 4-{4-[5-(4-Fluoro-phenyl)-3-(2-methoxy-ethyl)-2-methylsulfanyl-3H-imidazol-4-yl]-pyridin-2-ylamino}-cyclohexanol

0.18 g (0.0005 mole) 2-fluoro-4-[5-(4-fluoro-phenyl)-3-(2-methoxy-ethyl)-2-methylsulfanyl-3H-imidazol-4-yl]-pyridine and 0,29 g (0.0025 mole) trans-4-aminocyclohexanol reacted under pressure for 3.5 h at 205° C (outside temperature). The mixture then was dissolved in ethyl acetate (20 mL) and washed with water (5 x 10 mL). The aqueous phases were combined and extracted with ethyl acetate (15 mL), the ethyl acetate phase was dried over Na₂SO₄ and the solvent was removed. The product crystallized.
C₂₄H₂₉FN₄O₂S *(Mr 456,49)*
Yield: 190 mg (84%)
Mp: 54 °C
GC-MS)
22.3 min m/z (%) 456 (100), 397 (32), 383 (41), 357 (97), 300 (25), 267 (24), 207 (24)
IR (λ[cm⁻¹]): 3319 (OH), 2928, 2856, 1604, 1546, 1501, 1448, 1219 (4-FPh), 1117, 839,812
¹H-NMR (DMSO-d6)
δ (ppm) 1.13-1.28 (m, 4-H trans-4-aminocyclohexanol); 1.80-1.93 (m, 4-H, trans-4-aminocyclohexanol); 2.63 (s, 3H, SCH₃); 3.12 (s, 3-H, OCH₃); 3.47-3.42 (t, J= 5.62 Hz, 3H, NCH₂-**CH₂**-OCH₃+ C1-H trans-4-aminocyclohexanol); 3.57 (s, 1-H, C4-H trans-4-aminocyclohexanol); 3.95 (t, J= 5.6 Hz, 1 H, NH-**CH₂**-CH₂-OCH₃); 4.52 (d, 1-H, J=4.4 Hz, OH); 6.37 (s, 1-H, C3-H Pyr); 6.45 (m, 2H, C5-H Pyr+ NH); 7.07-7.12 (m, 2-H, C3/5-H 4-FPh); 7.40-7.44 (m, 2-H, C2/6-H 4-FPh); 8.06 (d, 1-H, J=5.2 Hz, C6-H Pyr)
¹³C-NMR (DMSO-d6)
δ (ppm) 15.68 (1C, S-CH₃); 30.33 (2C,trans-4-aminocyclohexanol); 33.95 (2C, trans-4-aminocyclohexanol); 43.60 (2C, NH-**CH₂**-CH₂-OCH₃); 48.63 (1C, C4 trans-4-aminocyclohexanol); 58.01 (1C, OCH₃)); 68.35 (1-C, C1 trans-4-aminocyclohexanol); 69.81 (1-C, NH-CH₂-**CH₂**-OCH₃) ; 109.32 (1C, C3-Pyr); 112.43 (1C, C5-Pyr); 114.95 (d, 2C, ²J(C,F)=21.43 Hz, 4-FPh); 127.85 (d, 2C, ³J(C,F)=7.95 Hz, 4-FPh); 128.43; 130.50; 136.21; 138.55; 143.15; 148.56 (1C, C6, Pyr); 158.74; 160.81 (1C, ¹J(C,F)=243.12 Hz)

### Example 17

### 2-{4-[5-(4-Fluoro-phenyl)-3-methyl-2-methylsulfanyl-3H-imidazol-4-yl]-pyridin-2-ylamino}-cyclohexanol

0.63 g (0.002 mole) 2-fluoro-4-[5-(4-fluoro-phenyl)-3-methyl-2-methylsulfanyl-3H-imidazol-4-yl]-pyridine and 0.45 g (0.0038 mole) trans-2-aminocyclohexanol were reacted under pressure for 3.5 h at 205° C (outside temperature). After cooling to RT the mixture was dissolved in ethyl acetate (55 mL) and washed with water (4 x 10 mL) and finally with a saturated aqueous sodium chloride solution (1 x 10 mL). The aqueous phases containing a precipitate were combined and extracted with ethyl acetate (20 mL), the ethyl acetate phase was dried over Na₂SO₄ and the solvent was removed. The product crystallized from n-hexane.
C₂₂H₂₅FN₄OS *(Mr 412,53)*
Yield: 350 mg (44 %)
Purity (HPLC 4.9 min) 92%
Mp:114°C
GC-MS:
15,14 min (silylized) M 484
GC- MS:
17,75 min; m/z (%) 412 (59), 353 (12), 341 (98), 313 (1009, 280 (36), 267 (14), 206.9 (28)
IR (λ[cm⁻¹]): 3635, 3303, 2931, 2857, 1613, 1524, 1504, 1480, 1220 (4-FPh), 1071,841
¹H-NMR (DMSO-d6)
δ (ppm) 1.09-1.28 (m, 4H 2-aminocyclohexanol); 1.58-1.64 (m, 2H, 2-aminocyclohexanol); 1.86-1.99 (m, 2H, 2-aminocyclohexanol); 2.63 (s, 3H, SCH₃); 3,31 (water +C1-H 2-aminocyclohexanol); 3.38 (s, 3H, NCH₃); 3.45-3.48 (m, 1-H,C2-H 2-aminocyclohexanol); 4.78 (d, 1 H, J=4.4 Hz, OH); 6.41 (d, 1 H, J= 4.8 Hz, C5-H Pyr); 6.44-6.46 (m, 2-H, C3-H Pyr+ NH) 7.08-7.13 (m, 2H, C3/5-H 4-FPh); 7.44-7.48 (m, 2H, C2/6-H 4-FPh); 8.02 (d, 1 H, J=4.8 Hz, C6-H Pyr)
¹³C-NMR (DMSO-d6)
δ (ppm) 15.24 (1C, S-CH₃); 23.67 (1-C, 2-amino-cyclohexanol); 23.92 (1-C, 2-aminocyclohexanol); 30.79 (1C, 1-C, 2-aminocyclohexanol); 31.37 (1C, NCH₃); 34.10 (1C, 2-aminocyclohexanol); 55.95 (1C, C2 2-aminocyclohexanol); 72.13 (1C, C1 2-amino-cyclohexanol); 109.12 (1C, C3-Pyr); 112.14 (1C, C5-Pyr); 114.97 (d, 2C, ²J(C,F)=21.53 Hz 4-FPh); 128.01 (d, 2C, ³J(C,F)=8.65 Hz 4-FPh); 128.67; 130.60; 136.27; 138.31; 142.98; 148.14 (1C, C6, Pyr); 159.45; 1C,
¹J(C,F) not visible

### Example 18

### 2-{4-[5-(4-Fluoro-phenyl)-3-methyl-2-methylsulfanyl-3H-imidazol-4-yl]-pyridin-2-ylamino}-cyclohexanol

1,51 g (0.0047 mole) 2-fluoro-4-[5-(4-fluoro-phenyl)-3-methyl-2-methylsulfanyl-3H-imidazol-4-yl]-pyridine und 2,68 g (0.023 mole) 2-amino-cyclohexanol were reacted for 2.5 h at 140° C. The mixture was dissolved in ethyl acetate (50 mL) and washed with water until the washing water had a neutral pH. The aqueous phases were combined and extracted with ethyl acetate (15 mL), the ethyl acetate phase was dried over Na₂SO₄ and the solvent was removed. The product crystallized.
C₂₂H₂₅FN₄OS *(Mr 412.53)*
Yield: 2.1 g
Purity: (HPLC 5.0 min) 96,3%
GC- MS
15,27 min m/z (%) 412 (80), 384 (18), 383 (11), 365 (18), 341 (100), 327 (32), 313 (99), 291 (12), 281 (27), 266 8 11)
GC-MS
15,27 min M 484 (silylated)
IR (λ[cm⁻¹]): 3358 (OH), 2929, 2856, 1734 (ethyl acetate-CO), 1605, 1498, 1218 (4-FPh), 1131, 979, 838, 811, 590
¹H-NMR (DMSO-d6)
δ (ppm) 1.25-1.71 (m, 8-H, 2-aminocyclohexanol); 2.63 (s, 3-H, SCH₃); 3.38 (s, 3-H, NCH₃); 3.79 (s, 1-H, C2-H, 2-aminocyclohexanol); 3.84 (m, 1-H, C1-H 2-aminocyclohexanol); 4.59 (s, 1-H, J= 4.00 Hz, OH); 6.21 (d, 1-H, J= 7.6 Hz, NH); 6.4 (d, 1-H, J= 4.4 Hz, 5-H Pyr); 7.08-7.13 (m, 2-H, C3/5-H 4-FPh); 7.43-7.47 (m, 2-H, C2/6-H 4-FPh); 8.03 (d, 1-H, J= 4.0 Hz; C6-H Pyr)
¹³C-NMR (DMSO-d6)
δ (ppm) 15.79 (1-C, SCH₃); 20.16 (1-C, 2-aminocyclohexanol); 24.40 (1-C, 2-aminocyclohexanol); 27.08 (1-C, 2-aminocyclohexanol), 31.91 (1-C, NCH₃); 32.35 (1-C, 2-aminocyclohexanol); 52.46 (1-C, C2 2-aminocyclohexanol); 67.29 (1-C, C1 2-aminocyclohexanol); 109.82 (1-C, C3 Pyr); 112.73 (1-C, C5 Pyr); 115.5 (1-C, ²J( C,F)= 21.43 Hz, C3/5 4-FPh); 128.57 (1-C, ³J(C,F)=7.95 Hz, C2/6 4-FPh); 131.17 (1-C, ⁴J= 2.42 Hz, C1 4-FPh); 136.83; 138.83, 143.51; 148.82 (1-C, C6 Pyr); 159.23; 161.39 (1-C, ⁴J (C,F)=243.12 Hz)

### Example 19

### 2-{4-[5-(4-Fluoro-phenyl)-3-(2-methoxy-ethyl)-2-methylsulfanyl-3H-imidazol-4-yl]-pyridin-2-ylamino}-cyclohexanol

3,07 g (0.0085 mole) 2-methoxy-ethyl)-2-methylsulfanyl-3H-imidazol-4-yl]-pyridine und 5,7 g (0.049 mole) 2-amino-cyclohexanol were reacted for 2.5 h at 140° C. The warm mixture was dissolved in ethyl acetate (50 mL) and washed with water (15 mL each) until the washing water had a pH-value of 6-7. The aqueous phases were combined and extracted with ethyl acetate (15 mL), the ethyl acetate phase was dried over Na₂SO₄ and the solvent was removed. The product was foam-like after removal of the solvent.
C₂₄H₂₉FN₄0₂S *(Mr* 456, 59)
Yield:3.25 g (96%)
GC-MS:
20,95 min; m/z (%) 456 (78), 439 (14), 428 (14), 427 (14), 409 (18), 385 (100), 371 (29), 357 (899, 267 (14)
GC- MS:
17,47 min; M 528 (silylated)
IR (λ[cm⁻¹]): 3378 (OH), 2857, 1735, 1605, 1499, 1219 (4-FPh), 1118, 978, 839, 606
¹H-NMR (DMSO-d6)
δ (ppm) 1.26-1.30 (m, 2-H, 2-aminocyclohexanol); 1.43-1.67 (m, 5-H, 2-aminocyclohexanol); 2.63 (s, 3-H, SCH₃) 3.11 (s, 3-H, OCH₃); 3.38 (t, 2-H, J= 5.6 Hz, NH-CH₂-**CH₂**-OCH₃); 3.76 (s, 1-H, C2-H 2-amino-cyclohexanol); 3.83 (m, 1-H, C1-H 2-amino-cyclohexanol); 3.93 (t, 2-H, J= 5.6 Hz, NH-**CH₂**-CH₂-OCH₃); 4.56 (s, 1-H, OH); 6.19 (d, 1-H, J= 8.0 Hz, NH); 6.41 (d, 1-H, J=5.2 Hz, C5-H Pyr); 6.52 (s, 1-H, C3-H Pyr); 7.05-7.09 (m, 2-H, C3/5-H 4-FPh); 7.40-7.44 (m, 2-H, C2/6-H 4-FPh); 8.01 (d, 1-H, J= 5.2 Hz; C6-H Pyr)
¹³C-NMR (DMSO-d6)
δ (ppm) 16.23 (1-C, SCH₃); 20.13 (1-C, 2- aminocyclohexanol); 24.38 (1-C, 2-aminocyclohexanol) 27.04 (1-C, 2- aminocyclohexanol), 32.67 (1-C, 2-aminocyclohexanol); 44.12 (1-C, NH-**CH₂**-CH₂-OCH₃); 52.51 (1-C, C2 2-aminocyclohexanol); 58.57 (1-C, SCH₃); 67.28 (1-C, C1 2- aminocyclohexanol; 70.37 (1-C, NH-CH₂-**CH₂**-OCH₃); 110.19 (1-C, C3 Pyr); 113.15 (1-C, C5 Pyr); 115.49 (1-C, ²J (C,F)= 21.53 Hz, C3/5 4-FPh); 128.4 (1-C, ³J (C,F)= 8.1 Hz, C2/6 4-FPh) ; 128.4; 131.05 (1-C, ⁴J= 3.02 Hz, C1 4-FPh); 136.77; 139.12; 143.71; 148.90 (1-C, C6 Pyr); 159.25; 161.37 (1-C, ¹J(C,F)= 243.12 Hz)

### Example 20

### 1-{4-[5-(4-Fluoro-phenyl)-2-methanesulfinyl-3-methyl-3H-imidazol-4-yl]-pyridin-2-ylamino}-propan-2-ol

0.484 g (0.0013 mole) 1-{4-[5-(4-fluoro-phenyl)-3-methyl-2-methylsulfanyl-3H-imidazol-4-yl]-pyridin-2-ylamino}-propan-2-ol dissolved in 10 mL acetic acid (100%) and 0.16 g (0.0014 mole) hydrogen peroxide 30% dissolved in 1 ml glacial acetic acid were reacted for 24 h. The mixture was poured into ice water (15 mL), adjusted to pH 8-9 with ammonia and extracted with ethyl acetate (6x 5 mL). The organic phases were combined and washed with water (10 mL). The ethyl acetate phase was dried over Na₂SO₄ and solvent was removed. The product was glass-like.
C₁₉H₂₁FN₄O₂S *(Mr388,47)*
Yield :470 mg (96%)
Purity: (HPLC 2,87 min) 99%
GC-MS:
14,15 min; m/z (%) 388 (8), 344 (52), 328 (100), 279 (16), 207 (15)
IR (λ[cm⁻¹]): 3330, 1608, 1503, 1376, 1298, 1219 (4-FPh), 1026, 839, 811, 657, 589
¹H-NMR (CDCl₃)
δ (ppm) 1.24 (d, 3-H, J= 6.0 Hz, CH₃); 3.24; (s, 3-H, SOCH₃); 2.27-3.52 (m, 2-H, NH-**CH₂**-CH(OH)CH₃); 3.79 (s, 3-H, NCH₃); 4.00-4.04 (m, 1-H, NH-CH₂-**CH**-(OH)CH₃) 5.06 (s, 1-H, OH); 6.35 (d, 1-H, C3-H Pyr); 6.52 (d, 1-H, J= 4.8 Hz, C5-H Pyr); 6.94-6.98 (m, 1-H, C3/5-H 4-FPh); 7.45-7.48 (m, 1-H, C2/6-H 4-FPh); 8.16 (d, 1-H, J= 4.8 Hz, C6-H Pyr)
¹³C-NMR (CDCl₃)
δ (ppm) 21.02 (1-C, CH(OH)-**CH₃**); 32.11 (1-C, NCH₃); 38.11 (1-C, SOCH₃); 49.99 (1-C, NH-CH₂-CH-(OH)CH₃); 67.97 (1-C, NH-CH₂-**CH**-(OH)CH₃) ; 109.48 (1-C, C3-Pyr); 114.08 (1-C, C5, Pyr); 115.37 (d, 2-C, ²J(C,F)=21.53 Hz 4-FPh); 128.83 (d, 2-C ³J(C,F)=7.92 Hz 4-FPh); 129.18; 130.72; 138.40; 138.84; 146.39; 148.73 (1-C, C6, Pyr); 159.44; 162.23 (d, 1-C, ¹J(C,F)=247.45 Hz)

### Example 21

### 1-{4-[5-(4-Fluoro-phenyl)-2-methanesulfinyl-3-(2-methoxy-ethyl)-3H-imidazol-4-yl]-pyridin-2-ylamino}-propan-2-ol

0.114 g (0.00024 mole) 1-f4-[5-(4-fluoro-phenyl)-3-(2-methoxy-ethyl)-2-methylsulfanyl-3H-imidazol-4-yl]-pyridin-2-ylamino}-propan-2-ol dissolved in 2 mL acetic acid (100%) and 0.03 g (0.0026 mole) hydrogen peroxide 30% dissolved in 0.5 ml glacial acetic acid were reacted for 24 h. The mixture was poured into ice water (15 mL), adjusted to pH 8-9 with ammonia (32%) and extracted with ethyl acetate (6x 5 mL). The organic phases were combined and washed with water (10 mL). The ethyl acetate phase was dried over Na₂SO₄ and solvent was removed. The product is glass-like.
C₂₁ H₂₅FN₄O₃S *(Mr 432,52)*
Yield: 80 mg (78%)
Purity: (HPLC 2.87 min) 99%
GC-MS
15,47 min; m/z (%) 432 (25), 388 (89), 373 (1009, 355 (84), 313 (42), 281 (59), 207(70)
IR (λ[cm⁻¹]): 3326, 2966, 2926, 1732, 1608, 1503, 1220 (4-FPh)), 1116, 1039/1014 ( SO), 839, 812
¹H-NMR (CDCl₃)
δ (ppm) 1.25 (d, 3-H, J= 6.8 Hz, CH₃); 3.23 (s, 3-H, SOCH₃); 3.26 (s, 3-H, OCH₃); 3.20-3.61 (m, 4-H, NH-**CH₂**-CH(OH)CH)+ N-CH₂-**CH₂**-OCH₃); 4.01-4.04 (m, 1-H, NH-CH₂-**CH**-(OH)CH₃) 4.2-4.25 (m, 1-H, N-**CH₂**-CH₂-OCH₃); 4.45-4.51 (m, 1-H, N-**CH₂**-CH₂-OCH₃); 5.18 (s, 1-H, OH); 6.45 (d, 1-H, C3-H Pyr); 6.55 (s, 1-H, J=5.6 Hz, C5-H Pyr); 6.93-6.97 (m, 1-H, C3/5-H 4-FPh); 7.45-7.49 (m, 1-H, C2/6-H 4-FPh); 8.13(d, 1-H, J= 5.6 Hz, C6-H Pyr)
¹³C-NMR (CDCl₃)
δ (ppm) 21.01 (1-C, CH(OH)**CH₃**); 38.96 (1-C, SOCH₃); 44.48 (1-C, N-**CH₂**-CH₂-OCH₃); 50.03 (1-C, NH-**CH₂**-CH-(OH)CH₃); 58.94 (1-C, OCH₃); 67.97 (1-C, NH-CH₂-**CH**-(OH)CH)); 71.30 (1-C, N-CH₂-**CH₂**-OCH₃) ; 109.89 (1-C, C3-Pyr); 114,54 (1-C, C5, Pyr); 115.29 (d, 2-C, ²J(C,F)=22.04 Hz 4-FPh); 128.82 (d, 2-C ³J(C,F)=8.15 Hz 4-FPh); 129.73.; 138.74; 138.69 139.46, 147.64; 148.25 (1-C, C6, Pyr); 159.24; 162.21 (d, 1-C, ¹J(C,F)=246.85 Hz)

### Example 22

### 4-{4-[5-(4-Fluoro-phenyl)-2-methanesulfinyl-3-methyl-3H-imidazol-4-yl]-pyridin-2-ylamino}-cyclohexanol

0,13 g (0.0009 mole) 4-{4-[5-(4-fluoro-phenyl)-3-methyl-2-methylsulfanyl-3H-imidazol-4-yl]-pyridin-2-ylamino}-cyclohexanol dissolved in 5 mL acetic acid (100%) and 0.03 g (0.0003 mole) hydrogen peroxide 30% dissolved in 1 ml glacial acetic acid were reacted for 72 h. The mixture was poured into ice water (15 mL) and adjusted to pH 8-9 with ammonia (32%) to give a precipitate. The precipitate was dissolved in ethyl acetate (20 mL) and separated from the aqueous phase. The aqueous phase was extracted with ethyl acetate (2x 10 mL). The organic phases were combined and extracted with water (10 mL). The ethyl acetate phase was dried over Na₂SO₄ and the solvent was removed. The product began to foam and crystallized.
C₂₂H₂₅FN₄O₂S *(Mr 428,53)*
Yield: 260 mg (68%)
Purity: (HPLC 2.68 min) 95%
Mp: 126 °C
GC-MS
23,69 min; m/z (%) 429 (5), 329 (14), 281 (33), 206.9 (100), 43.9 (17)
IR (λ[cm⁻¹]): 3319 (OH), 2930, 2856, 1731 (?), 1606, 1520, 1501, 1371, 1220 (4-FPh), 1041 (SO), 840, 588
¹H-NMR (CDCl₃)
δ (ppm) 1.20-1.44 (m, 4-H trans-4-aminocyclohexanol); 1.98-2.08 (m, 4-H, trans-4-aminocyclohexanol); 3.25 (s, 3-H, SOCH₃); 3.46-3.51 (m, 1-H, C1-H trans-4-aminocyclohexanol); 3.64-3.69 (m, 1-H, C4-H trans-4-aminocyclohexanol); 3.81 (s, 3-H, NCH₃); 4.55 (d, 1-H, J=7.2 Hz, OH); 6.21 (s, 1-H, C3-H Pyr); 6.50 (d, 1-H, J=5.2 Hz, C5-H Pyr) ; 6.94-6.99 (m, 2-H, C3/5-H 4-FPh); 7.45-7.49 (m, 2-H, C2/6-H 4-FPh); 8.19 (d, 1-H, J=5.2 Hz, C6-H Pyr)
¹³C-NMR (CDCl₃)
δ (ppm) 30.82 (2-C, 2 trans-4-aminocyclohexanol); 32.15 (1-C, NCH₃); 33.84 (2-C, trans-4-aminocyclohexanol); 38.10 (1-C, SOCH₃); 49.68 (1-C, C4 trans-4-aminocyclohexanol); 69.84 (1-C, C1 trans-4- aminocyclohexanol); 108.04 (1-C, C3-Pyr); 113.53 (1-C, C5-Pyr); 115.35 (d, 2-C, ²J(C,F)=21.43 Hz, 4-FPh); 128.93 (d, 2C, ³J(C,F)=8.05 Hz, 4-FPh); 129.3 (d, ⁴J( C,F)= 2.9 Hz, C1, 4-FPh); 131.1; 138.40; 138.70; 146.37; 149.47 (1C, C6, Pyr); 158.51; 162.24 (1-C, ¹J(C,F)=246.95 Hz)

### Example 23

### 4-{4-[5-(4-Fluoro-phenyl)-2-methanesulfinyl-3-(2-methoxy-ethyl)-3H-imidazol-4-yl]-pyridin-2-ylamino}-cyclohexanol

0.13 g (0.00028 mole) 4-{4-[5-(4-fluoro-phenyl)-3-(2-methoxy-ethyl)-2-methylsulfanyl-3H-imidazol-4-yl]-pyridin-2-ylamino}-cyclohexanol dissolved in 5 mL acetic acid (100%) and 0.11 g (0.0003 mole) hydrogen peroxide 30% dissolved in 0.5 ml glacial acetic acid were reacted for 48 h. The mixture was poured into ice water (15 mL) and adjusted to pH 8-9 with ammonia (32%) to give a precipitate. The precipitate was dissolved in ethyl acetate (10 mL) and separated from the aqueous phase. The aqueous phase was extracted with ethyl acetate (5x 5 mL). The organic phases were combined and washed with water (10 mL). The ethyl acetate phase was dried over Na₂SO₄ and solvent was removed. The product crystallized. C₂₄H₂₉FN₄O₃S *(Mr 472,59)*
Yield: 124 mg (95%)
Purity (HPLC 3.03 min): 95%
Mp: 115 °C
GC-MS:
28,61 min; m/z (%) 472 (45), 456 (58), 373 (61), 357 (58), 311 (76), 281 (64), 207 (100), 175 (27)
IR (λ[cm⁻¹]): 3312(OH) 2930, 2857, 1731, 1606, 1521, 1502, 1220(4-FPh) 1041 (SO), 957, 840
¹H-NMR (DMSO-d6)
δ (ppm) 1.17-1.28 (m, 4-H, trans-4-aminocyclohexanol); 1.81-1.93 (m, 4-H, trans-4-aminocyclohexanol); 3.11 (s, 3-H, SOCH₃); 3.13 (1-H, OCH₃); 3.42 (m, 3-H, NCH₂-**CH₂**-OCH₃); 3.49-3.52 (m, 1-H, C1-H trans-4-aminocyclohexanol); 3.59 (m/s, 1-H, C4-H trans-4-aminocyclohexanol); 4.16-4.32 (m, 1-H, NH-**CH₂**-CH₂-OCH₃); 4.51 (d, 1-H, J=4.0 Hz, OH); 6.42 (s, 1-H, C3-H Pyr); 6.48 (d, 2-H, J=5.2 Hz , C5-H Pyr); 6.53 (d, 1-H, J=7.6 Hz, NH); 7.13-7.17 (m, 2-H, C3/5-H 4-FPh); 7.45-7.48 (m, 2-H, C2/6-H 4-FPh); 8.11 (d,1-H, J=4.8 Hz, C6-H Pyr)
¹³C-NMR (DMSO-d6)
δ (ppm) 30.29 (2C, trans-4-aminocyclohexanol); 33.94 (2C, aminocyclohexanol)); 38.57 (1C, SCH₃); 43.89 (2C, NH-**CH₂**-CH₂-OCH₃); 48.64 (1C, C1 trans-4-aminocyclohexanol); 58.03 (1C, OCH₃); 68.33 (1-C, C4 trans-4-aminocyclohexanol); 70.44 (1-C, NH-CH₂-**CH₂**-OCH₃) C3-Pyr not visible; 112.21 (1C, C5-Pyr); 115.23 (d, 2C, ²J(C,F)=22.14 Hz, 4-FPh); 128.22 (d, 2C, ³J(C,F)=7.95 Hz, 4-FPh); 129.73; 129.90; 148.04; 148.83 (1C, C6, Pyr); 158.74; 159.99; ¹J (C,F) not visible

### Example 24

### 2-{4-[5-(4-Fluoro-phenyl)-2-methanesulfinyl-3-methyl-3H-imidazol-4-yl]-pyridin-2-ylamino}-cyclohexanol

0.116 g (0.00028 mole) 4-{4-[5-(4-Fluoro-phenyl)-3-methyl-2-methylsulfanyl-3H-imidazol-4-yl]-pyridin-2-ylamino}-cyclohexanol dissolved in 5 mL acetic acid (100%) and 0.03 g (0.0026 mole) hydrogen peroxide 30% dissolved in 0.5 ml glacial acetic acid were reacted for 25 h at RT. The mixture was poured into ice water (15 mL) and adjusted to pH 8-9 with ammonia (32%). The aqueous phase was extracted with ethyl acetate (4 x 10 mL). The organic phases were combined and washed with water (10 mL). The ethyl acetate phase was dried over Na₂S0₄ and solvent was removed. The product crystallized. C₂₂H₂₅FN₄O₂S *(Mr 428,53)*
Yield: 120 mg (100%)
Mp: 112°C
GC-MS:
23,273 min; M 501 (silylation)
IR (λ[cm⁻¹]): (ATR)
3375 (-NH-), 2964, 2872, 2796, 1605, 1517, 1498, 1221 (4-FPh), 1118, 1054 (SO), 841,813,605
¹H-NMR (DMSO-d6)
δ (ppm) 1.09-1.28 (m, 4-H trans-2-aminocyclohexanol); 1.58-1.64 (m, 2-H, trans-2-aminocyclohexanol); 1.78 -2.00 (m, 2-H, trans-2-aminocyclohexanol); 3.13 (s, 3-H, SOCH₃); 3.27-3.34 (m, 1-H, C1 trans-2-aminocyclohexanol), 3.50-3.52 (m, 1-H, C 2 trans-2-aminocyclohexanol); 3.70 (s, 3-H, NCH₃) 4.75 (s, 1-H, OH); 6.46 (d, 1-H, J= 5.2 Hz, C5-H Pyr), 6.51-6.54 (m, 2-H, C3-H Pyr+ NH); 7.13-7.18 (m, 1-H, C3/5-H 4-FPh); 7.47-7.50 (m, 2-H, C2/6-H 4-FPh); 8.07 (d, 1-H, J=5.2 Hz, C6-H Pyr)
¹³C-NMR (DMSO-d6)
δ (ppm) 23.69 (1-C, trans-2-aminocyclohexanol); 23.94 (1-C, trans-2aminocyclohexanol); 30.76 (1C, trans-2aminocyclohexanol); 31.66 (1C, NCH₃); 34.10 (1C, trans-2-aminocyclohexanol); 37.87 (1C, SOCH₃); 55.92 (1C, C2 trans-2-aminocyclohexanol); 72.06 (1-C, C1 trans-2-aminocyclohexanol); 109.27 (1-C, C3 Pyr); 111.96 (1C, C5 Pyr); 115.23 (d, 1-C, ²J( C,F)= 21.43 Hz, 4-FPh); 128.37 (d, 2C, ³J(C,F)=8.05 Hz 4-FPh); 129.82, 130.70, 136.38, 137.13; 146.83, 148.43 (1C, C6, Pyr); 159.43; 1C, ¹J(C,F) not visible

### Example 25

### 2-{4-[5-(4-Fluoro-phenyl)-2-methanesulfinyl-3-(2-methoxy-ethyl)-3H-imidazol-4-yl]-pyridin-2-ylamino}-cyclohexanol

1.05 g (0.0023 mole) 2-{4-[5-(4-fluoro-phenyl)-3-(2-methoxy-ethyl)-2-methylsulfanyl-3H-imidazol-4-yl]-pyridin-2-ylamino}-cyclohexanol dissolved in 30 mL acetic acid (100%) and 0.28 g (0.0025 mole) hydrogen peroxide 30% dissolved in 2 ml glacial acetic acid were reacted for 120 h at RT. The mixture was poured into ice water (10 mL) and adjusted to pH 8-9 with ammonia (32%) to give a precipitate. The precipitate was dissolved in ethyl acetate (30 mL) and separated from the aqueous phase. The aqueous phase was extracted with ethyl acetate (4x 10 mL). The organic phases were combined and washed with water (15 mL). The ethyl acetate phase was dried over Na₂SO₄ solvent was removed. The product crystallized. C₂₄H₂₉FN₄O₃S *(Mr 472,59)*
Yield: 1,1g
Purity: (HPLC 4.1 min): 94,9%
GC-MS
472 (16), 456 (28), 401 (41), 385 (36), 373 (58), 311 (44), 281 (64), 207 (100)
IR (λ[cm⁻¹]): 3337, 2929, 2857, 1734, 160, 1518, 1500,1448, 1220 (4-FPh), 1115, 1042 (SO), 972, 839, 593
¹H-NMR (DMSO-d6)
δ (ppm) 1.25-1.32 (m, 2-H, 2-aminocyclohexanol); 1.43-1.72 (m, 5-H, 2-aminocyclohexanol); 3.11 (s, 3-H, SOCH₃); 3.14 (s, 3-H, NCH₃); 3.34-3.53 (m, 2-H, NH-CH₂-**CH₂**-OCH₃); 3.81 (s, 1-H, C2-H 2-aminocyclohexanol); 3.86 (m, 1-H, C1-H 2-aminocyclohexanol); 4.16-4.35 (m, 2-H, NH-**CH₂**-CH₂-OCH₃); 4.6 (s, 1-H, J= 3.6 Hz, OH); 6.31 (d, 1-H, J= 7.6 Hz, NH); 6.47 (d, 1-H, J=5.2 Hz, C5-H Pyr); 6.59 (s, 1-H, C3-H Pyr); 7.13-7.17 (m, 2-H, C3/5-H 4-FPh); 7.46-7.50 (m, 2-H, C2/6-H 4-FPh); 8.08 (d, 1-H, J=5.2 Hz, C6-H Pyr)
¹³C-NMR (DMSO-d6)
δ (ppm) 20.1 (1-C, 2-aminocyclohexanol;) 24.43 (1-C, 2 aminocyclohexanol); 27.00 (1-C, 2-aminocyclohexanol), 32.38 (1-C, 2-aminocyclohexanol); 39.13 (1-C, SOCH₃); 44.41 (1-C, NH-**CH₂**-CH₂-OCH₃); 52.49 (1-C, C2 2- aminocyclohexanol); 58.59 (1-C, OCH₃); 67.21 (1-C, C1 2-aminocyclohexanol; 70.97 (1-C, NH-CH₂-**CH₂**-OCH₃); 110.31 (1-C, C3 Pyr);112.89 (1-C, C5 Pyr); 115.74 (1-C, ²J (C,F)= 21.43 Hz, C3/5 4-FPh); 128.67 (1-C, ³J (C,F)= 8.65 Hz, C2/6 4-FPh) ; 130.28 (1-C, ⁴J= 3.2 Hz 1-C 4-FPh); 130.44 ; 137.12; 138.01; 148.61; 149.18 (1-C, C6 Pyr); 159.25; 161.18 (1-C, ¹J(C,F)= 233.86 Hz)

### Example 26

### 1-{4-[5-(4-Fluoro-phenyl)-3-(2-methoxy-ethyl)-2-methylsulfanyl-3H-imidazol-4-yl]-pyridin-2-ylamino}-propan-2-one

0.61 g (0.0048 mole) oxalyl chloride were dissolved in dichloromethane (6 mL) and cooled under argon atmosphere to -70° C. 0.81 g (0,0104 mole) DMSO were added dropwise and the mixture was stirred for 30 min. 0.5 g (0.00145 mole) 1-{4-[5-(4-fluoro-phenyl)-3-(2-methoxy-ethyl)-2-methylsulfanyl-3H-imidazol-4-yl]-pyridin-2-ylamino}-propan-2-ol dissolved in dichloromethane (5 mL) were added. After 30 min the mixture was treated with 2.05 g (0.0203 mole) triethylamine and cooling was stopped. 4 mL of water were added at RT and the solution was stirred for ten minutes. After adding of 10 mL water the organic phase was separated and washed with a saturated solution of sodium chloride in water (2 x 15 mL). The aqueous phase was extracted with dichloromethane (15 mL). The organic phases were combined, dried over Na₂SO₄ and filtered. After removing the solvent the product was purified by column chromatography: Al₂O₃ / toluene: THF 5:1
C₂₁H₂₃FN₄O₂S *(Mr 414,51):*
Yield: 358 mg (59%);
Purity (HPLC RT=4.1 min.): 99%;
mp: 112°C
GC-MS: (RT=12,6 min); m/z (%) 414 (48), 371 (100), 313 (13), 279 (12);
IR (λ[cm⁻¹]): 3411(NH), 2929, 1705(CO), 1606, 1518, 1499, 1316, 1214(4-FPh), 1108,843,812.
¹H-NMR (DMSO-d6): δ (ppm) 2.1 (s, 3-H, -CO-**CH₃**); 2.64 (s, 3-H, SCH₃); 3.12 (s, 3-H, OCH₃); 3.39 (t, 2-H, J= 5,2 Hz, N-CH₂-**CH₂**-OCH₃); 3.96 (t, 2-H, J=5,6 Hz, N-CH₂-CH₂-OCH₃); 4.13 (d, 2-H, J= 6.0 Hz, N-**CH₂**-CO-CH₃) 6.52 (d, 1-H, J= 5.6 Hz, C5-H Pyr); 6.63 (s, 1-H, C3-H Pyr); 6.99 (m, 1-H, J= 6.0 Hz, NH); 7.09-7.11 (m, 1-H, C3/5-H 4-FPh); 7.41-7.44 (m, 1-H, C2/6-H 4-FPh); 8.05 (d, 1-H, ³J= 5.2 Hz, C6-H Pyr) ¹³C-NMR (DMSO-d6): δ (ppm) 15.68 (1-C S-CH₃) 26.87 (1-C, -CO-**CH₃**); 43.60 (1-C, N-**CH₂**-CH₂-OCH₃); 50.94 (1-C, **CH₂**-CO-CH₃); 57.99 (1-C, OCH₃); 69.78 (1-C, N-CH₂-**CH₂**-OCH₃); 109.84 (1-C, C3 Pyr),113.45 (1-C, C5 Pyr); 114.96 (d, 2-C, ²J(C,F)=21.33 Hz, 4-FPh); 127.84 (d, 2-C ³J(C,F)=8.05 Hz, 4-FPh); 128.21; 130.41; 136.29; 138.77; 143.27; 148.25 (1-C, C6 Pyr); 158.63; 10.83 (d, 1-C, ¹J(C,F)=243.22 Hz); 206.04 (1-C, CO)

### Example 27

### 4-{4-[5-(4-Fluoro-phenyl)-3-methyl-2-methylsulfanyl-3H-imidazol-4-yl]-pyridin-2-ylamino}-cyclohexanone

0.6 g (0.0048 mole) oxalyl chloride were dissolved in dichloromethane (6.5 mL) and cooled under argon atmosphere to -70° C. 0.81 g (0,0104 mole) DMSO were added dropwise and the mixture was stirred for 30 min. 0.59 g (0.00145 mole) 4-{4-[5-(4-fluoro-phenyl)-3-methyl-2-methylsulfanyl-3H-imidazol-4-yl]-pyridin-2-ylamino}-cyclohexanol dissolved in dichloromethane (4 mL) were added. After 30 min the mixture was treated with 2.03 g (0.0201 mole) triethylamine and cooling was stopped. 4 mL of water were added at RT and the solution was stirred for ten minutes. After adding of 10 mL water the organic phase was separated and washed with a saturated solution of sodium chloride in water (2 x 15 mL). The aqueous phase was extracted with dichloromethane (15 mL). The organic phases were combined, dried over Na₂SO₄ and filtered. After removing the solvent the product was purified by column chromatography two times:
1. SiO₂ (Geduran 60) / toluene: THF 3:1
2. SiO₂ (Geduran 60) / ethyl acetate: n-hexane 4:1

The product crystallized after complete removal of the solvent in vacuum. C₂₂H₂₃FN₄OS *(Mr 410, 52):*
Yield: 190 mg (32%);
Purity (HPLC RT=3.99 min): 96%.
GC-MS: (RT=19,74 min.) m/z (%) 410 (100), 353 (41), 339 (32), 313 (97), 281 (33), 241 (11)
HPLC-MS: m/z (MH⁺) 411
IR (λ[cm⁻¹]): 3370, 2932, 1712 (CO), 1603, 1517, 1500, 1477, 1372, 1218 (4-F-Ph), 1156,1044,839,812,591
¹H-NMR (CDCl₃): δ (ppm) 1.69-1.78 (m, 2-H 4-aminocyclohexanone); 2.26-2.30 (m, 2-H, 4-aminocyclohexanone); 2.43-2.46 (m, 4-H, 4-aminocyclohexanone); 2.71 (s, 3-H, SCH₃); 3.48 (s, 3-H, NCH₃); 4.03-4.15 (m 1-H,C4H-OH); 4.64 (s, 1-H, NH); 6.27 (s,1-H, C3-H Pyr); 6.56 (d, 1 H, J=5.2 Hz , C5-H Pyr); 6.92-6.96 (m, 2-H, C3/5-H 4-FPh); 7.45-7.49 (m, 2-H, C2/6-H 4-FPh); 8.17 (d, 1-H, J=5.2 Hz, C6-H Pyr)
¹³C-NMR (CDCl₃): δ (ppm) 15.96 (1C, S-CH₃); 31.8 (1C, NCH₃); 32.02 (2C, 4-aminocyclohexanone); 38.89 (2C, 4-amino-cyclohexanone); 48.13 (1C, C4 4-aminocyclohexanone); 108.53 (1C, C3-Pyr); 114.16 (1C, C5-Pyr); 115.15 (d, 2C, ²J(C,F)=21.43 Hz 4-FPh); 128.16; 128.82 (d, 2C, ³J(C,F)=8.15 Hz 4-FPh); 130.18; 140.25; 144.63; 148.74 (1C, C6, Pyr); 159.02, 161.99 (1C, ^{1J}(C,F)=246.14 Hz)

### Example 28

### 2-{4-[5-(4-Fluoro-phenyl)-3-methyl-2-methylsulfanyl-3H-imidazol-4-yl]-pyridin-2-ylamino}-cyclohexanone

1.51 g (0.012 mole) oxalyl chloride were dissolved in dichloromethane (12 mL) and cooled under argon atmosphere to -70° C. 2.03 g (0,026 mole) DMSO were added dropwise and the mixture was stirred for 30 min. 1.5 g (0.0036 mole) 4-{4-[5-(4-Fluoro-phenyl)-3-methyl)-2-methylsulfanyl-3H-imidazol-4-yl]-pyridin-2-ylamino}-cyclohexanol dissolved in dichloromethane (12 mL) were added. After 30 min the mixture was treated with 5.1 g (0.051 mole) triethylamine and cooling was removed. 15 mL of water were added at RT and the solution was stirred for ten minutes. After adding of 30 mL water the organic phase was separated and washed with a saturated solution of sodium chloride in water (3 x 30 mL). The aqueous phase was extracted with dichloromethane (30 mL). The organic phases were combined, dried over Na₂S0₄ and filtered. After removing the solvent the product was purified by column chromatography: Sio₂ (Geduran 60) / ethyl acetate: n-hexane 4:1
C₂₂H₂₃FN₄0S *(Mr 410,52):*
Yield: 400 mg (27%);
Purity (HPLC RT=4.3 min.): 94.4%;
Mp: 71 °C.
GC-MS: M/Z (REL. INT [%]) =17,73 min; m/z (%) 410 (100), 393 (31), 380 (16), 352 (37), 339 (18), 313 (80), 281 (27)
IR (λ[cm⁻¹]): 3382, 2931, 2861, 1712 (CO), 1605, 1511, 1496, 1217 (4-FPh), 1125, 972,838,812
¹H-NMR (DMSO-d6): δ (ppm) 1.44-1,60 (m, 2-H, 2-aminocyclohexanone,); 1.78-1.83 (m, 2-H, 2-aminocyclohexanone); 2.03-2.08 (m, 1-H, aminocyclohexanone) 2.29-2.40 (m, 2-H, 2-aminocyclohexanone); 2.52-2.59 (m, 1-H, C6-H 2-aminocyclohexanone); 2.64 (s, 1-H, SCH₃); 3.38 (s, 3-H, NCH₃) 4.62-4.48 (m, 1-H; C2-H 2-aminocyclohexanone) 6.47 (d, 1-H, J= 6.4 Hz, C5-H Pyr); 6.60 (s, 1-H, C3-H Pyr); 6.74 (d, 1-H, J= 7.2 Hz, NH); 7.08-7.12 (m, 2-H, C3/5-H 4-FPh); 7.43-7.46 (m, 2-H, C2/6-H 4-FPh); 8.03 (d, 1-H, J= 5.2 Hz, C6-H Pyr).
¹³C-NMR (DMSO-d6): δ (ppm) 15.79 (1-C, SCH₃); 24.39 (1-C, 2-aminocylohexanone); 28.00 (1-C, 2-aminocylohexanone); 31.91 (1-C, NCH₃); 34.89 (1-C, aminocylohexanone); 41.20 (1-C, aminocylohexanone); 59.20 (1-C, C2 aminocylohexanone); 110.08 (1-C, C3 Pyr); 113.48 (1-C, C5 Pyr); 115.52 (1-C, ²J( C,F)= 21.3 Hz, C3/5 4-FPh); 128.56 (1-C, ³J(C,F)=8.05 Hz, C2/6 4-FPh); 129.06; 131.13 (1-C, ⁴J=2.21 Hz, C1 4-FPh); 136.90; 138.98; 143.62, 148.67 (1-C, C6 Pyr); 158.90; 161.42 (1-C, ⁴J (C,F)=243.22 Hz); 209.06 (1-C, C1 2-aminocyclohexanone).

### Example 29

### 4-[5-(4-Fluoro-phenyl)-2-methanesulfinyl-3-(2-methoxy-ethyl)-3H-imidazol-4-yl]-2-isopropoxy-pyridine and 4-[5-(4-Fluoro-phenyl)-2-methanesulfonyl-3-(2-methoxy-ethyl)-3H-imidazol-4-yl]-2-isopropoxy-pyridine

### 4[5-(4-Fluoro-phenyl)-2-methanesulfanyl-3-(2-methoxy-ethyl)-3H-imidazol-4-yl]-2-isopropoxy-pyridine

2-Fluoro-4-[5-(4-fluoro-phenyl)-3-(2-methoxy-ethyl)-2-methylsulfanyl-3H-imidazol-4-yl]-pyridine (1.2 g) were dissolved in HCI-isopropanol (about 1.25 mole, 12 g) und heated to 80 °C in a closed vessel. The reaction was terminated after about 16 h. Excessive HCl was neutralized with aqueous NaHCO₃ solution and the product was extracted into ethyl acetate. The ethyl acetate phase was extracted with water, dried over Na₂SO₄, filtered and concentrated under vacuum. Purification by column chromatography (SiO2 / EtOAc-hexane 8:2) gave an oil.
Purity HPLC (RT=9.17 min.) >99%,
1 H-NMR: ppm (CDCl3) 1.36 (d, 6H, 6.0 Hz, 2xCH3, isoprop); 2.71 (s, 3H, S-CH3); 3.22 (s, 3H, O-CH3); 3.47 (t, 2H, 5.6 Hz, ethyl); 4.02 (t, 2H, 5.4 Hz, ethyl); 5.30 (quint, 1 H, 6.0 Hz, C-H, isoprop); 6.67 (s, 1 H, C3-H, Py); 6.78 (d, 4.0 Hz, 1 H, C5-H, Py); 6.88-6.93 (m, 2H, C3/5-H, 4F-Ph); 7.40-7.44 (m, 2H, C2/6-H, 4F-Ph); 8.18 (d, 1H, 4.8 Hz, C6-H, Py);

### 4-[5-(4-Fluoro-phenyl)-2-methanesulfinyl-3-(2-methoxy-ethyl)-3H-imidazol-4-yl]-2-isopropoxy-pyridine

The conversion of the sulfanyl to the sulfinyl compound was carried out according to the general method: The sulfanyl compound (0.3 g) was dissolved in 2 g glacial acetic acid and then concentrated H₂O₂ solution (0.13 g, 30 % solution) was added. The reaction was monitored. If required, several additional aliquots of H₂O₂ were added (0.05 g). After a reaction time of 72 h the reaction was terminated at a conversion of 80 %.The reaction mixture was partitioned between water and THF, the phases were separated and the water phase was extracted with several aliquots of THF. The THF phase was extracted with water, dried over Na₂SO₄ filtered and concentrated under vacuum. Purification and isolation of the product was carried out by column chromatography (cc): SiO₂/ EtOAc-hexane 6:4 to EtOAc 100%
Yield: 0.165 g (55%); semi-solid mass:
Purity HPLC: (RT= 8.24 min.) >99%.
1H-NMR: ppm (CDCl3) 1.36 (d, 6H, 6.0 Hz, 2xCH3, isoprop); 3.21 (s, 3H, SO-CH3); 3.22 (s, 3H, O-CH3); 3.42-3.48 (m, 1 H, ethyl); 3.52-3.57 (m, 1 H, ethyl); 4.18-4.24 (m, 1 H, ethyl); 4.43-4.50 (m, 1 H, ethyl); 5.32 (quint, 1 H, 6.0 Hz, C-H, isoprop); 6.61 (s, 1 H, C3-H, Py); 6.77 (d, 4.0 Hz, 1 H, C5-H, Py); 6.89-6.94 (m, 2H, C3/5-H, 4F-Ph); 7.40-7.44 (m, 2H, C2/6-H, 4F-Ph); 8.22 (d, 1 H, 4.8 Hz, C6-H, Py).

### 4-[5-(4-Fluoro-phenyl)-2-methanesulfonyl-3-(2-methoxy-ethyl)-3H-imidazol-4-yl]-2-isopropoxy-pyridine

The conversion of the sulfanyl to the sulfinyl compound was carried out according to the general method with m-chloro-perbenzoic acid (mCPBA) in 2-fold stoichiometric amount: The sulfanyl compound (0.22 g) and mCPBA (0.13 g, 70%) were dissolved in CH₂Cl₂ and stirred under heating (36°C). After 1 h additional mCPBA (0.13 g, 70%) was added and stirring and heating was continued for 1 h. After a reaction time of about 2.5 h the reaction mixture was extracted with half-saturated Na₂HCO₃ solution and then with water. The CH₂Cl₂ phase was dried over Na₂SO₄ , filtered and concentrated in vacuum. Purification and isolation of the product was carried out by column chromatography: SiO₂ / EtOAc-hexane 3:7:
Yield: 0.185 g (80%);
Purity HPLC: (RT= 8.56 min.) >99%.
1H-NMR: ppm (CDCl3) 1.37 (d, 6H, 6.0 Hz, 2xCH3, isoprop); 3.21 (s, 3H, O-CH3); 3.49 (s, 3H, S02-CH3); 3.62 (t, 2H, 5.4 Hz, ethyl); 4.42 (t, 2H, 5.4 Hz, ethyl); 5.33 (quint, 1 H, 6.0 Hz, C-H, isoprop); 6.70 (s, 1 H, C3-H, Py); 6.80 (d, 4.0 Hz, 1 H, C5-H, Py); 6.92-6.96 (m, 2H, C3/5-H, 4F-Ph); 7.38-7.41 (m, 2H, C2/6-H, 4F-Ph); 8.25 (d, 1 H, 4.8 Hz, C6-H, Py).

### Example 30

### 4-[5-(4-Fluoro-phenyl)-3-(2-methoxy-ethyl)-2-methylsulfanyl-3H-imidazol-4-yl]-2-(1-phenyl-ethoxy)-pyridine

In a reaction vessel 1-phenylethanol (0.66 g) in diethylene glycol dimethylether (4 g) as solvent was converted to the alcoholate with NaH (0.260 g). To the alcoholate solution 2-fluoro-4-[5-(4-fluoro-phenyl)-3-(2-methoxy-ethyl)-2-methylsulfanyl-3H-imidazol-4-yl]-pyridine (0.36 g), dissolved in diethylene glycol dimethylether (1.2 g), was added and heated to 90 °C. The reaction temperature was increased to 130°C and stirring was continued until a HPLC sample showed complete conversion of the starting material (RT = 7.62 min.) (2 h). CH₂Cl₂ (40 ml) was added and the organic phase was extracted with water (6 x 25 mL). The organic phase was dried over Na₂SO₄, filtered and concentrated. The liquid residue was charged from EtOAc solution to SiO₂ and purified by cc: SiO₂ / hexane, then EtOAc/hexane=2/8.
GC-MS: (BP220_0: RT = 11.763 min.) 95 %;
70 eV-EI-MS: m/z (rel. Int.[%]) 465 (10), 464 (30), 463 = M⁺ (100), 448 (10), 359 (20), 358 (50), 328 (10); 314 (7); 312 (12), 301 (13), 300 (30), 296 (10), 285 (7), 268 (20), 105 =C8H9⁺; (70),

### Example 31

### 2-Chloro-N-{4-[5-(4-fluoro-phenyl)-3-(2-methoxy-ethyl)-2-methylsulfanyl-3H-imidazol-4-yl]-pyridin-2-yl}-acetamide

In a round-bottom flask, 1.07 g (3.0 mmole) 4-[5-(4-fluoro-phenyl)-3-(2-methoxyethyl)-2-methylsulfanyl-3H-imidazol-4-yl]-pyridin-2-ylamine was dissolved in 30 ml THF, 0.63 g K₂CO₃ was added and the mixture was vigorously stirred. 810 mg chloroacetyl chloride, dissolved in 6 ml THF were added dropwise. Thereafter, the mixture was kept at 50°C for 2 h. The reaction was terminated by addition of 40 ml water and after extraction with 30 ml ethyl acetate the organic layer was separated and washed with water and brine and dried over sodium sulfate. After removal of the solvent, the residue was re-crystallized from isopropanol.
Yield: 61 %
Purity (HPLC/UV): > 99%

### Example 32

### Tetrahydro-pyran-4-carboxylic acid {4-[5-(4-fluoro-phenyl)-3-(2-methoxy-ethyl)-2-methylsulfanyl-3H-imidazol-4-yl]-pyridin-2-yl}-amide

### Method A:

111 mg (0.8 mmole) tetrahydropyran-4-yl-carboxylic acid and 165 (1.0 mmole) 1,1'-carbonyldiimidazole were stirred in 2.5 ml THF at 60°C. After 1 h, the oil bath was removed and 286 mg 4-[5-(4-fluoro-phenyl)-3-(2-methoxy-ethyl)-2-methylsulfanyl-3H-imidazol-4-yl]-pyridin-2-ylamine, dissolved in 2.5 ml THF were added. The mixture was stirred at 60°C overnight, then poured into 30 ml dichloromethane and washed with 15 ml water. The organic phase was dried over sodium sulfate and after removal of the solvent the product was purified by chromatography on silica (THF/toluene 30/70).
Yield: 53 %
Purity (HPLC/UV): > 99 %

### Method B:

0.13 g (10 mmole) tetrahydropyran-4-yl-carboxylic acid and 5.1 ml (70 mmole) thionylchloride were stirred at RT for 1 h. Thereafter, thionylchloride was removed under vacuum and the oily residue was dissolved in 2 ml dichloromethane. 358 mg (10 mmole) 4-[5-(4-fluoro-phenyl)-3-(2-methoxy-ethyl)-2-methylsulfanyl-3H-imidazol-4-yl]-pyridin-2-ylamine were dissolved in 10 ml pyridine and cooled to 0-5°C in an icebath. The dichloromethane solution containing tetrahydropyran-4-yl-carboxylic acid chloride was added dropwise, the mixture was kept at 0-5°C. After 2 h, the solvent was removed, the residue dissolved in ethyl acetate, washed with water, dried over sodium sulfate and concentrated under vacuum. The product was purified by chromatography on silica (Methanol / Ethyl acetate 2.5/97.5).

### Example 33

### Tetrahydro-pyran-4-carboxylic acid {4-[5-(4-fluoro-phenyl)-2-methanesulfinyl-3-(2-methoxy-ethyl)-3H-imidazol-4-yl]-pyridin-2-yl}-amide

0.095 g (2 mmole) Tetrahydro-pyran-4-carboxylic acid {4-[5-(4-fluoro-phenyl)-3-(2-methoxy-ethyl)-2-methylsulfanyl-3H-imidazol-4-yl]-pyridin-2-yl}-amide was dissolved in 1.6 ml THF. Sodium periodate (0.070 g), dissolved in 0.8 ml water was added and the mixture was refluxed for 10 h. THF was removed under vacuum, water was added and the residue extracted with ethyl acetate. The organic phase was dried over sodium sulfate, the solvent removed under vacuum and the product was purified by chromatography on silica (methanol/ethyl acetate 5/95).
Yield: 30 %
Purity (HPLC/UV): > 99 %

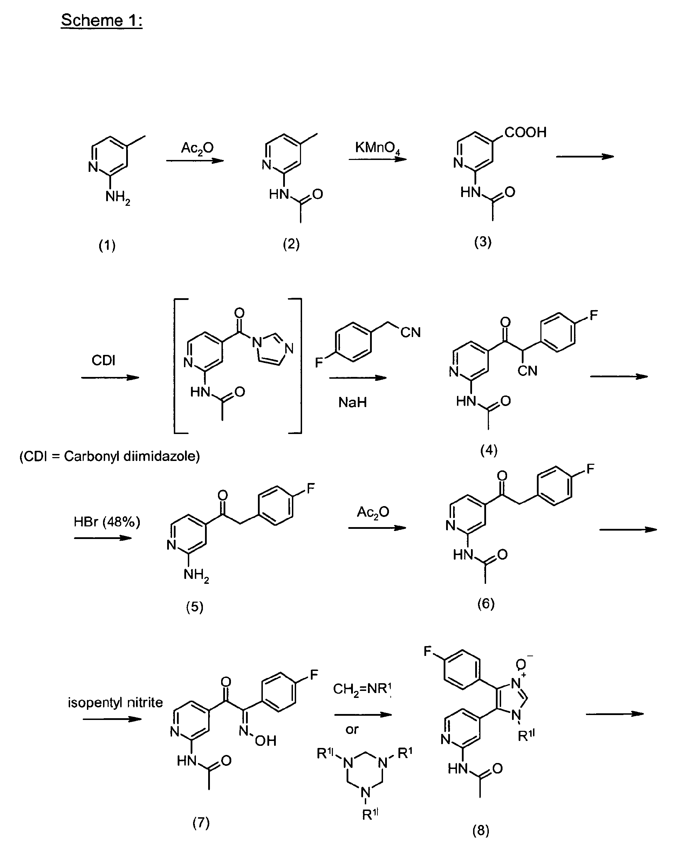

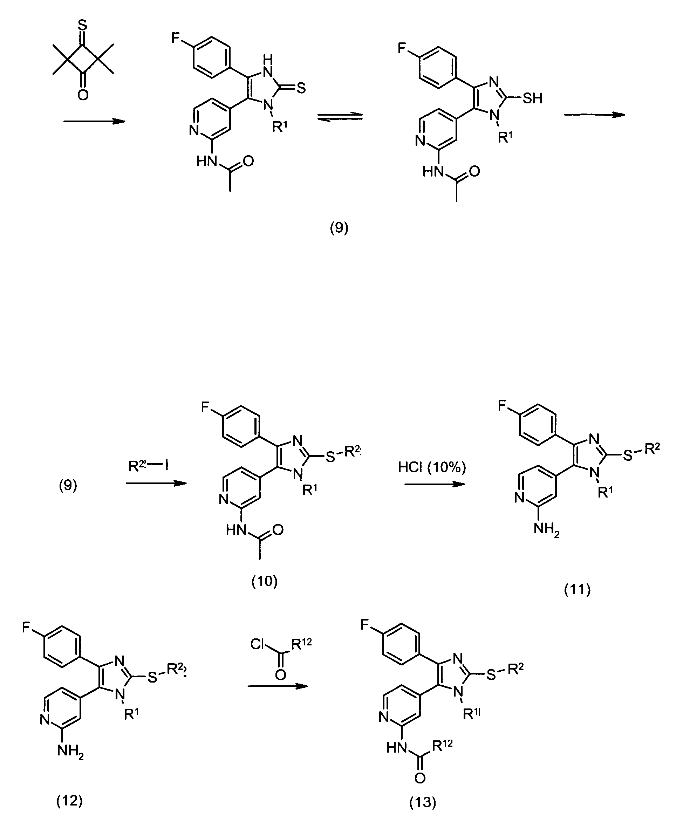

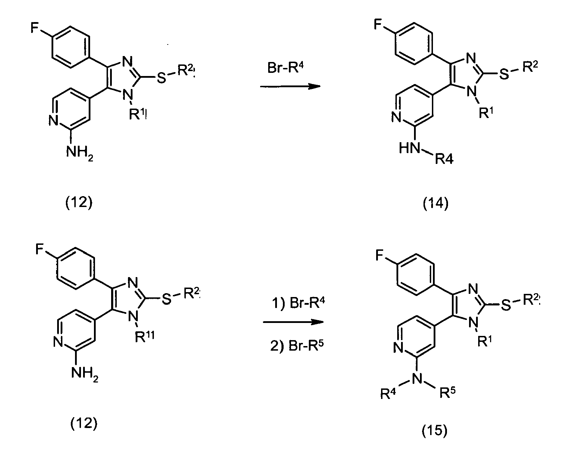

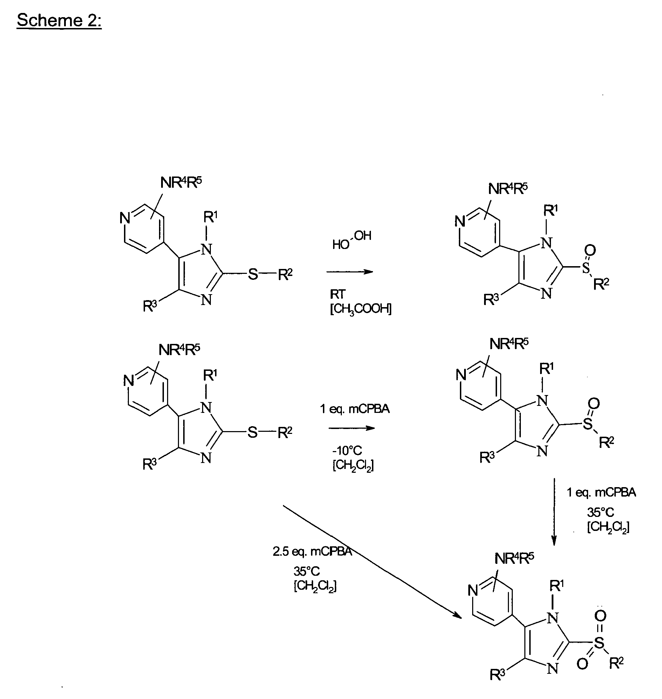

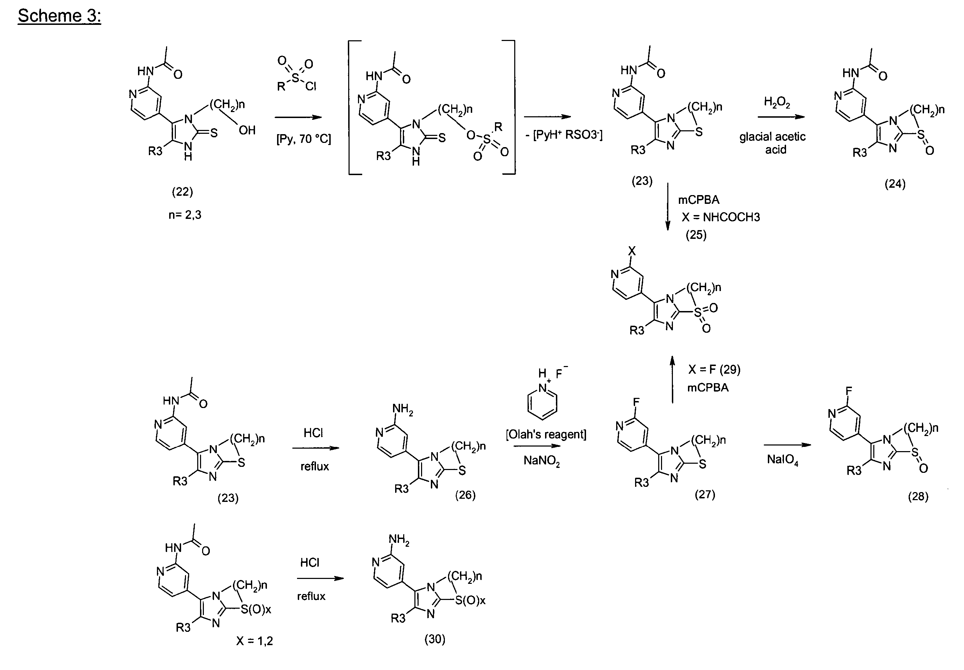

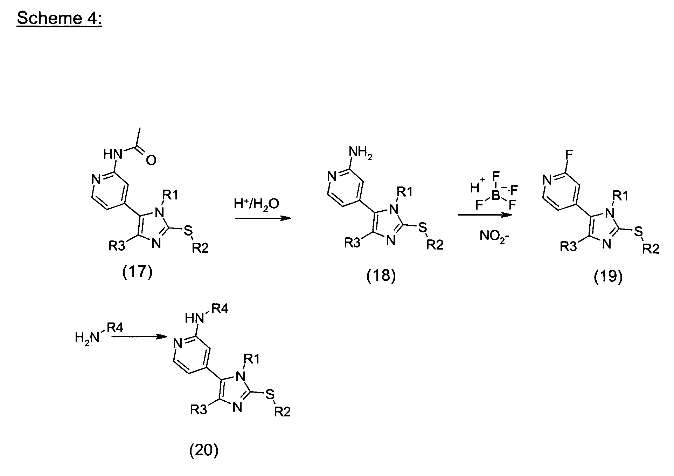

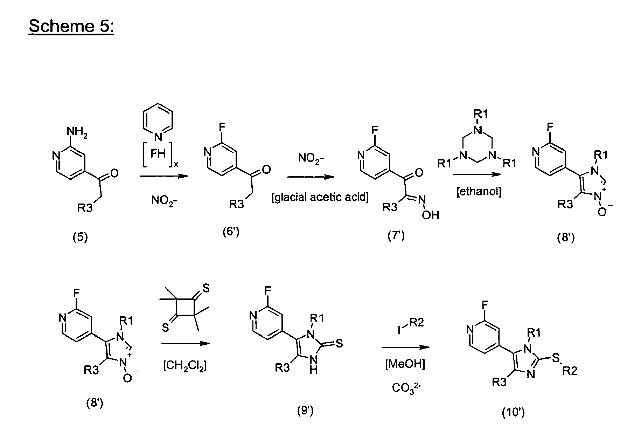

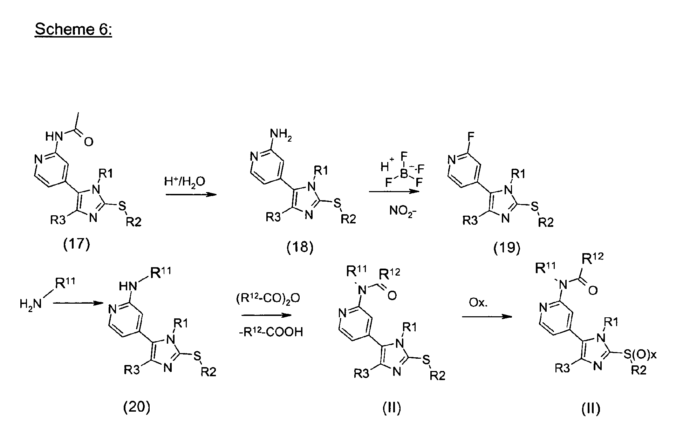

## Claims

1. An imidazole compound of the formula I 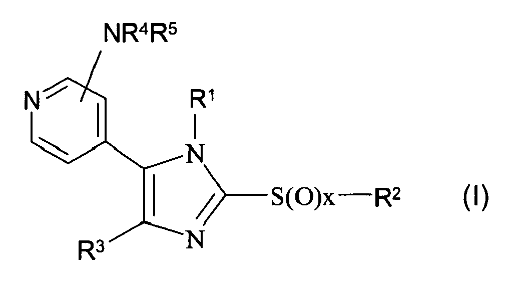 in which
R¹ is selected from:
a) C₁-C₆-alkyl which is optionally substituted by one or two groups independently of one another selected from
hydroxy;
C₁-C₄-alkoxy;
C₂-C₆-alkenyloxy;
C₂-C₆-alkynyloxy;
CO₂H ;
CO₂-C₁-C₆-alkyl;
CN;
halogen;
C₁-C₆-alkyl- SO₃;
C₁-C₆-alkylthio;
NR⁷R⁸, wherein R⁷ and R⁸ are independently of one another H, C₁-C₆-alkyl or hydroxy-C₁-C₆-alkyl;
R⁹CONR¹⁰, R⁹ and R¹⁰ are independently of one another H or C₁-C₆-alkyl;
a nonaromatic heterocyclic radical having 5 or 6 ring atoms and 1 or 2 heteroatoms, selected independently of one another from N, O and S, which heterocyclic radical may be substituted by 1, 2, 3 or 4 C₁-C₆-alkyl groups;
b) A (OA) OB,
in which
A is -CH₂CH₂-, -CH₂CH₂CH₂-, 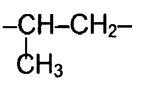 or 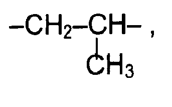 n is 1, 2, 3, 4 or 5, and B is H or C₁-C₄-alkyl;
c) C₁-C₆-oxoalkyl;
d) C₂-C₆-alkenyl
e) C₃-C₇-cycloalkyl;
f) (C₃-C₇cycloalkyl)-C₁-C₆-alkyl;
g) aryl which is optionally substituted by one or more halogen atoms or a C₁-C₄-alkylsulfanyl group;
h) aminoaryl, where the amino group is optionally substituted by one or two C₁-C₄-alkyl groups;
i) aryl-C₁-C₆-alkyl;
j) an aromatic or nonaromatic heterocyclic radical having 5 or 6 ring atoms and 1 or 2 heteroatoms selected independently of one another from N, O and S, which heterocyclic radical is optionally substituted by 1, 2, 3 or 4 C₁-C₄-alkyl groups, an aryl or aryl-C₁-C₄-alkyl group; and
k) H;
R² is selected from:
a) C₁-C₆-alkyl,
b) phenyl-C₁-C₄-alkyl, where the phenyl group may have one or two substituents which are selected independently of one another from C₁-C₄-alkyl, halogen, C₁-C₄-alkylsulfanyl, C₁-C₄-alkylsulfinyl and C₁-C₄-alkylsulfonyl,
c) C₂-C₆-alkenyl,
d) C₂-C₆-alkenyl which is substituted by one or two halogen atoms and/or phenyl groups, where the phenyl group may be substituted independently by one or two C₁-C₄-alkyl or halogen atoms,
e) C₂-C₆-alkynyl,
f) C₂-C₆-alkynyl which is substituted by a phenyl group which may be optionally substituted by one or two C₁-C₄-alkyl or halogen atoms,
g) C₁-C₆-alkyl which is substituted by C₁-C₄-alkylsulfanyl, C₁-C₄-alkylsulfinyl or C₁-C₄-alkylsulfonyl;
h) C₁-C₆-alkyl which is substituted by -CO-Het wherein Het is a nonaromatic heterocyclic radical having 5 or 6 ring atoms and 1 or 2 heteroatoms which are selected independently of one another from N, O, and S, or C₁-C₄-alkylsulfanyl, C₁-C₄-alkylsulfinyl or C₁-C₄-alkylsulfonyl;
i) phenyl; and
j) phenyl which has one or two substituents which are selected independently of one another from C₁-C₄-alkyl, halogen, C₁-C₄-alkylsulfanyl, C₁-C₄-alkylsulfinyl or C₁-C₄-alkylsulfonyl;
R¹ and R² together are -CH₂CH₂- or -CH₂CH₂CH₂-,
x is 0, 1 or 2,
R³ is phenyl which is unsubstituted or substituted by 1 or 2 halogen atoms, C₁-C₄-alkyl groups or trifluoromethyl groups,
R⁴ is selected from
a) (C₁-C₆-alkoxy)-C₁-C₆-alkyl;
b) (C₁-C₆-alkoxy)-C₃-C₇-cycloalkyl;
c) hydroxy-C₁-C₆-alkyl;
d) hydroxy-C₃-C₇-cycloalkyl;
e) C₃-C₇-oxocycloalkyl;
f) an nonaromatic heterocyclic radical having 5 or 6 ring atoms and 1 or 2 heteroatoms selected independently of one another from N, O and S, which heterocyclic radical is attached via a carbon atom to the amino group and is optionally substituted by 1, 2, 3 or 4 C₁-C₄-alkyl groups, an aryl or aryl-C₁-C₄-alkyl group;
g) C₁-C₆ -alkyl which is substituted by an nonaromatic heterocyclic radical having 5 or 6 ring atoms and 1 or 2 heteroatoms selected independently of one another from N, O and S, which heterocyclic radical is optionally substituted by 1, 2, 3 or 4 C₁-C₄-alkyl groups, an aryl or aryl-C₁-C₄-alkyl group; and
h) C₁-C₆-oxoalkyl;
i) phenyl-C₁-C₄-alkyl, wherein the alkyl group is substituted by 1 or 2 substituents independently selected from OH or C₁-C₄-alkoxy;
j) phenyl-C₁-C₄-alkyl, wherein the phenyl group is substituted by 1 or 2 substituents independently selected from OH or C₁-C₄-alkoxy;
k) C₃-C₇-cycloalkyl-C₁-C₄-alkyl, wherein the cycloalkyl group is substituted by 1 or 2 substituents independently selected from OH or C₁-C₄-alkoxy; and
l) phenyl, which is substituted by 1 or 2 substituents independently selected from Hal, OH, C₁-C₄-alkoxy, CF₃, amino, mono-C₁-C₄-alkylamino, di-C₁-C₄-alkylamino, C₁-C₄-alkyl, C₁-C₄-alkylcarbonyl and C₁-C₄-alkyl which is substituted by 1 or 2 substituents independently selected from OH or C₁-C₄-alkoxy;
R⁵ is H, C₁-C₄-alkyl, phenyl or benzyl, and
the optical isomers and physiologically tolerated salts thereof.

2. A compound as claimed in claim 1 of the formula I in which R¹ is selected from:
H;
C₁-C₆-alkyl which is optionally substituted by one or two hydroxy or C₁-C₄-alkoxy groups or one nonaromatic heterocyclic radical having 5 or 6 ring atoms and 1 or 2 heteroatoms which are selected independently of one another from N, O and S,
- A (̵ OA-)̵ₙ OB,
in which
A is -CH₂CH₂-, -CH₂CH₂CH₂-, 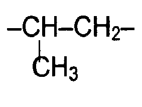 or 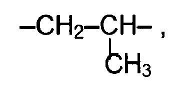 n is 1, 2, 3, 4 or 5, and B is H or C₁-C₄-alkyl,
C₂-C₆-alkenyl,
C₃-C₇-cycloalkyl,
amino-C₁-C₆-alkyl, where the amino group is optionally substituted by one or two C₁-C₄-alkyl groups,
an aromatic or nonaromatic heterocyclic radical having 5 or 6 ring atoms and 1 or 2 heteroatoms selected independently of one another from N, O and S, which heterocyclic radical is optionally substituted by 1, 2, 3 or 4 C₁-C₄-alkyl groups.

3. A compound as claimed in claim 1 of the formula I in which R¹ is selected from:
H;
C₁-C₆-alkyl which is optionally substituted by one or two hydroxy or C₁-C₄-alkoxy groups or one nonaromatic heterocyclic radical having 5 or 6 ring atoms and 1 or 2 heteroatoms which are selected independently of one another from N, O and S, or
-A (̵OA)̵ₙ OB,
in which
A is -CH₂CH₂-, -CH₂CH₂CH₂-, 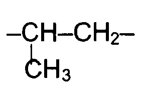 or 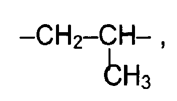 n is 1, 2, 3, 4 or 5 and B is H or C₁-C₄-alkyl.

4. A compound of the formula I as claimed in any of the preceding claims, where R¹ is H, C₁-C₄-alkyl, C₁-C₄-alkoxy-C₂-C₄-alkyl or hydroxy-C₂-C₄-alkyl.

5. A compound of the formula I as claimed in any of the preceding claims, in which R² is C₁-C₆-alkyl, phenyl-C₁-C₄-alkyl, phenyl or phenyl which has one or two substituents which are selected independently of one another from C₁-C₄-alkyl and halogen.

6. A compound of the formula I as claimed in any of the preceding claims, in which R² is C₁-C₆-alkyl or phenyl-C₁-C₄-alkyl.

7. A compound of the formula I as claimed in any of the preceding claims, in which R³ is 4-fluorophenyl.

8. A compound as claimed in any one of the preceding claims claim 1, where R⁴ is selected from (C₁-C₆-alkoxy)-C₁-C₆-alkyl, hydroxy-C₃-C₇-cycloalkyl, C₃-C₇-oxocycloalkyl, a non-aromatic heterocyclic radical having 5 or 6 ring atoms and 1 or 2 heteroatoms selected independently of one another from N, O or S, which heterocyclic radical is attached via a carbon atom to the amino group and is optionally substituted by 4 C₁-C₄-alkyl groups, or C₁-C₆ -alkyl which is substituted by a non-aromatic heterocyclic radical having 5 or 6 ring atoms and 1 or 2 heteroatoms selected independently of one another from N, O or S, which heterocyclic radical is optionally substituted by 1, 2, 3 or 4 C₁-C₄-alkyl groups and R⁵ is H.

9. A compound as claimed in any of the preceding claims, where NR⁴R⁵ is in 2-position.

10. A compound as claimed in claim 1, namely
{4-[5-(4-Fluoro-phenyl)-3-methyl-2-methylsulfanyl-3H-imidazol-4-yl]-pyridin-2-yl}-(2-methoxy-ethyl)-amine,
{4-[5-(4-Fluoro-phenyl)-2-methanesulfinyl-3-methyl-3H-imidazol-4-yl]-pyridin-2-yl}-(2-methoxy-ethyl)-amine,
1-{4-[5-(4-Fluoro-phenyl)-3-methyl-2-methylsulfanyl-3H-imidazol-4-yl]-pyridin-2-ylamino}-propan-2-ol,
1-{4-[5-(4-Fluoro-phenyl)-2-methanesulfinyl-3-methyl-3H-imidazol-4-yl]-pyridin-2-ylamino}-propan-2-ol,
4-{4-[5-(4-Fluoro-phenyl)-3-methyl-2-methylsulfanyl-3H-imidazol-4-yl]-pyridin-2-ylamino}-cyclohexanol,
4-{4-[5-(4-Fluoro-phenyl)-2-methanesulfinyl-3-methyl-3H-imidazol-4-yl]-pyridin-2-ylamino}-cyclohexanol,
4-{4-[5-(4-Fluoro-phenyl)-3-methyl-2-methylsulfanyl-3H-imidazol-4-yl]-pyridin-2-ylamino}-cyclohexanone,
(1 R,2R)-2-{4-[5-(4-Fluoro-phenyl)-3-methyl-2-methylsulfanyl-3H-imidazol-4-yl]-pyridin-2-ylamino}-cyclohexanol,
2-{4-[5-(4-Fluoro-phenyl)-3-methyl-2-methylsulfanyl-3H-imidazol-4-yl]-pyridin-2-ylamino}-cyclohexanol,
(1 R,2R)-2-{4-[5-(4-Fluoro-phenyl)-2-methanesulfinyl-3-methyl-3H-imidazol-4-yl]-pyridin-2-ylamino}-cyclohexanol,
2-{4-[5-(4-Fluoro-phenyl)-3-methyl-2-methylsulfanyl-3H-imidazol-4-yl]-pyridin-2-ylamino}-cyclohexanone,
{4-[5-(4-Fluoro-phenyl)-3-methyl-2-methylsulfanyl-3H-imidazol-4-yl]-pyridin-2-yl}-(2,2,6,6-tetramethyl-piperidin-4-yl)-amine,
{4-[5-(4-Fluoro-phenyl)-2-methanesulfinyl-3-methyl-3H-imidazol-4-yl]-pyridin-2-yl}-(2,2,6,6-tetramethyl-piperidin-4-yl)-amine,
(1-Ethyl-pyrrolidine-2-ylmethyl)-{4-[5-(4-fluoro-phenyl)-3-methyl-2-methylsulfanyl-3H-imidazol-4-yl]-pyridin-2-yl}-amine,
(1-Ethyl-pyrrolidine-2-ylmethyl)-{4-[5-(4-fluoro-phenyl)-2-methanesulfinyl-3-methyl-3H-imidazol-4-yl]-pyridin-2-yl}-amine,
(1-Benzyl-piperidin-4-yl)-{4-[5-(4-fluoro-phenyl)-3-methyl-2-methylsulfanyl-3H-imidazol-4-yl]-pyridin-2-yl}-amine,
(1-Benzyl-piperidin-4-yl)-{4-[5-(4-fluoro-phenyl)-2-methanesulfinyl-3-methyl-3H-imidazol-4-yl]-pyridin-2-yl}-amine,
(1-Benzyl-piperidin-4-yl)-{4-[5-(4-fluoro-phenyl)-2-methanesulfinyl-3-methyl-3H-imidazol-4-yl]-pyridin-2-yl}-amine,
{4-[5-(4-Fluoro-phenyl)-3-methyl-2-methylsulfanyl-3H-imidazol-4-yl]-pyridin-2-yl}-(tetrahydro-pyran-4-yl)-amine,
{4-[5-(4-Fluoro-phenyl)-2-methanesulfinyl-3-methyl-3H-imidazol-4-yl]-pyridin-2-yl}-(tetrahydro-pyran-4-yl)-amine,
1-{4-[5-(4-Fluoro-phenyl)-3-(2-methoxy-ethyl)-2-methylsulfanyl-3H-imidazol-4-yl]-pyridin-2-ylamino}-propan-2-ol,
1-{4-[5-(4-Fluoro-phenyl)-2-methanesulfinyl-3-(2-methoxy-ethyl)-3H-imidazol-4-yl]-pyridin-2-ylamino}-propan-2-ol,
1-{4-[5-(4-Fluoro-phenyl)-3-(2-methoxy-ethyl)-2-methylsulfanyl-3H-imidazol-4-yl]-pyridin-2-ylamino}-propan-2-one,
2-{4-[5-(4-Fluoro-phenyl)-3-(2-methoxy-ethyl)-2-methylsulfanyl-3H-imidazol-4-yl]-pyridin-2-ylamino}-propan-1-ol,
(R)-2-{4-[5-(4-Fluoro-phenyl)-3-(2-methoxy-ethyl)-2-methylsulfanyl-3H-imidazol-4-yl]-pyridin-2-ylamino}-propan-1-ol,
(S)-2-{4-[5-(4-Fluoro-phenyl)-3-(2-methoxy-ethyl)-2-methylsulfanyl-3H-imidazol-4-yl]-pyridin-2-ylamino}-propan-1-ol,
(S)-2-{4-[5-(4-Fluoro-phenyl)-3-(2-methoxy-ethyl)-2-methylsulfanyl-3H-imidazol-4-yl]-pyridin-2-ylamino}-4-methyl-pentan-1-ol,
4-{4-[5-(4-Fluoro-phenyl)-3-(2-methoxy-ethyl)-2-methylsulfanyl-3H-imidazol-4-yl]-pyridin-2-ylamino}-cyclohexanol,
4-{4-[5-(4-Fluoro-phenyl)-2-methanesulfinyl-3-(2-methoxy-ethyl)-3H-imidazol-4-yl]-pyridin-2-ylamino}-cyclohexanol,
4-{4-[5-(4-Fluoro-phenyl)-2-methanesulfonyl-3-(2-methoxy-ethyl)-3H-imidazol-4-yl]-pyridin-2-ylamino}-cyclohexanol,
2-{4-[5-(4-Fluoro-phenyl)-3-(2-methoxy-ethyl)-2-methylsulfanyl-3H-imidazol-4-yl]-pyridin-2-ylamino}-cyclohexanol,
2-{4-[5-(4-Fluoro-phenyl)-2-methanesulfinyl-3-(2-methoxy-ethyl)-3H-imidazol-4-yl]-pyridin-2-ylamino}-cyclohexanol,
3-{4-[5-(4-Fluoro-phenyl)-3-(2-methoxy-ethyl)-2-methylsulfanyl-3H-imidazol-4-yl]-pyridin-2-ylamino}-cyclohexanone,
3-{4-[5-(4-Fluoro-phenyl)-3-(2-methoxy-ethyl)-2-methylsulfanyl-3H-imidazol-4-yl]-pyridin-2-ylamino}-cyclohexanol,
(1-Ethyl-pyrrolidine-2-ylmethyl)-{4-[5-(4-fluoro-phenyl)-3-(2-methoxy-ethyl)-2-methylsulfanyl-3H-imidazol-4-yl]-pyridin-2-yl}-amine,
(1-Ethyl-pyrrolidine-2-ylmethyl)-{4-[5-(4-fluoro-phenyl)-2-methanesulfinyl-3-(2-methoxy-ethyl)-3H-imidazol-4-yl]-pyridin-2-yl}-amine,
{4-[5-(4-Fluoro-phenyl)-3-(2-methoxy-ethyl)-2-methylsulfanyl-3H-imidazol-4-yl]-pyridin-2-yl}-(2,2,6,6-tetramethyl-piperidin-4-yl)-amine,
{4-[5-(4-Fluoro-phenyl)-2-methanesulfinyl-3-(2-methoxy-ethyl)-3H-imidazol-4-yl]-pyridin-2-yl}-(2,2,6,6-tetramethyl-piperidin-4-yl)-amine,
(1-Benzyl-piperidin-4-yl)-{4-[5-(4-fluoro-phenyl)-3-(2-methoxy-ethyl)-2-methylsulfanyl-3H-imidazol-4-yl]-pyridin-2-yl}-amine,
(1-Benzyl-piperidin-4-yl)-{4-[5-(4-fluoro-phenyl)-2-methanesulfinyl-3-(2-methoxy-ethyl)-3H-imidazol-4-yl]-pyridin-2-yl}-amine.

11. A compound as claimed in claim 1, wherein
R¹ is selected from C₁-C₆-alkyl which may be substituted with hydroxy or C₁-C₆-alkoxy groups;
R² is C₁-C₆-alkyl;
R³ is 4-fluorophenyl;
R⁴ is selected from (C₁-C₆-alkoxy)-C₁-C₆-alkyl, hydroxy-C₃-C₇-cycloalkyl, C₃-C₇-oxocycloalkyl, a non-aromatic heterocyclic radical having 5 or 6 ring atoms and 1 or 2 heteroatoms selected independently of one another from N, O or S, which heterocyclic radical is attached via a carbon atom to the amino group and is optionally substituted by 4 C₁-C₄-alkyl groups, or C₁-C₆ -alkyl which is substituted by a non-aromatic heterocyclic radical having 5 or 6 ring atoms and 1 or 2 heteroatoms selected independently of one another from N, O or S, which heterocyclic radical is optionally substituted by 1, 2, 3 or 4 C₁-C₄-alkyl groups and R⁵ is H.

12. A compound as claimed in any one of the preceding claims claim 1, where R⁴ is selected from (C₁-C₆-alkoxy)-C₁-C₆-alkyl, hydroxy-C₃-C₇-cycloalkyl, C₃-C₇-oxocycloalkyl, a non-aromatic heterocyclic radical having 5 or 6 ring atoms and 1 or 2 heteroatoms selected independently of one another from N, O or S, which heterocyclic radical is attached via a carbon atom to the amino group and is optionally substituted by 4 C₁-C₄-alkyl groups, or C₁-C₆ -alkyl which is substituted by a non-aromatic heterocyclic radical having 5 or 6 ring atoms and 1 or 2 heteroatoms selected independently of one another from N, O or S, which heterocyclic radical is optionally substituted by 1, 2, 3 or 4 C₁-C₄-alkyl groups and R⁵ is H.

13. A compound of the formula II 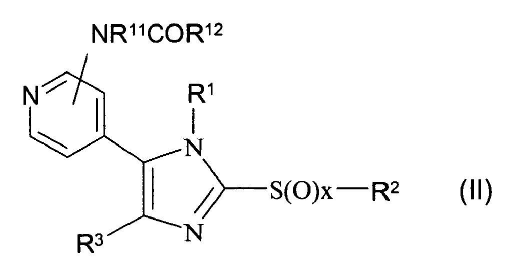 wherein R¹, R², R³ and x are as defined above, R¹¹ is H or C₁-C₄-alkyl and R¹² is C₁-C₄-alkyl which is substituted by 1 or 2 substituents independently selected from halogen, OH, C₁-C₄-alkoxy, C₁-C₄-alkylcarbonyloxy, phenyl and substituted phenyl which carries 1 or 2 substituents independently selected from halogen, OH, C₁-C₄-alkoxy and C₁-C₄-alkyl, an aromatic or nonaromatic heterocyclic radical having 5 or 6 ring atoms and 1 or 2 heteroatoms selected independently of one another from N, O and S, which heterocyclic radical is optionally substituted by 1, 2, 3 or 4 C₁-C₄-alkyl groups, an aryl or aryl-C₁-C₄-alkyl group or C₁-C₆ -alkyl which is substituted by a aromatic heterocyclic radical having 5 or 6 ring atoms and 1 or 2 heteroatoms selected independently of one another from N, O and S, which heterocyclic radical is optionally substituted by 1 or 2 C₁-C₄-alkyl groups. R¹¹ is preferably H and R¹² is preferably furanyl, thienyl, tetrahydropyranyl, tetrahydrofuranyl, thiophen-C₁-C₄-alkyl or furanyl- C₁-C₄-alkyl. In a furher preferred embodiment R¹² is chloromethyl, hydroxymethyl, 1-hydroxyethyl, 2-hydroxyethyl, methoxymethyl, 1-methoxyethyl, 2-methoxyethyl, acetyloxymethyl, 1-acethyloxyethyl or 2-acethyloxyethyl. The substituent of the 4-pyridyl group is preferably in 2-position.

14. A pharmaceutical composition comprising at least one compound as claimed in any of claims 1 to 13, where appropriate together with one or more pharmaceutically acceptable carriers and/or additives.

15. The use of at least one of the compounds as claimed in any of claims 1 to 13 for producing a pharmaceutical composition for the treatment of disorders associated with an impairment of the immune system.

16. A method for treating disorders associated with an impairment of the immune system, where an amount, which has an immunomodulating effect and or inhibits cytokine release, of a compound of the formula I as claimed in any of claims 1 to 13 is administered to a person requiring such a treatment.
